(19) Europäisches Patentamt
European Patent Office
Office européen des brevets

(11) EP 3 455 250 B1

(12) **FASCICULE DE BREVET EUROPEEN**

(45) Date de publication et mention de la délivrance du brevet:
**07.10.2020 Bulletin 2020/41**

(21) Numéro de dépôt: **17725911.6**

(22) Date de dépôt: **12.05.2017**

(51) Int Cl.:
*A61P 17/02* (2006.01) *C07K 14/78* (2006.01)
*C12M 3/00* (2006.01)

(86) Numéro de dépôt international:
**PCT/EP2017/061517**

(87) Numéro de publication internationale:
**WO 2017/194761 (16.11.2017 Gazette 2017/46)**

(54) **POLYPEPTIDE DERIVE DE LA TROPOELASTINE ET MATERIAU BIOCOMPATIBLE LE COMPRENANT**

AUS TROPOELASTIN ABGELEITETES POLYPEPTID UND BIOKOMPATIBLES MATERIAL DAMIT

POLYPEPTIDE DERIVED FROM TROPOELASTIN AND BIOCOMPATIBLE MATERIAL COMPRISING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.05.2016 FR 1654306**

(43) Date de publication de la demande:
**20.03.2019 Bulletin 2019/12**

(73) Titulaires:
- **Centre National de la Recherche Scientifique 75016 Paris (FR)**
- **Université Claude Bernard Lyon 1 69622 Villeurbanne (FR)**

(72) Inventeurs:
- **DEBRET, Romain 69008 Lyon (FR)**
- **FAYE, Clément 34160 Campagne (FR)**
- **SOHIER, Jérôme 69007 Lyon (FR)**
- **SOMMER, Pascal 13006 Marseille (FR)**

(74) Mandataire: **Lavoix 2, place d'Estienne d'Orves 75441 Paris Cedex 09 (FR)**

(56) Documents cités:
**WO-A1-99/03886**

- **BANDIERA ANTONELLA ET AL: "Expression and characterization of human-elastin-repeat-based temperature-responsive protein polymers for biotechnological purposes", BIOTECHNOLOGY AND APPLIED BIOCHEMISTRY, ACADEMIC PRESS, US, vol. 42, no. 3, 1 décembre 2005 (2005-12-01), pages 247-256, XP009128167, ISSN: 0885-4513 & GIANNI CIOFANI ET AL: "Human recombinant elastin-like protein coatings for muscle cell proliferation and differentiation", ACTA BIOMATERIALIA, vol. 9, no. 2, 1 février 2013 (2013-02-01) , pages 5111-5121, XP055334338, AMSTERDAM, NL ISSN: 1742-7061, DOI: 10.1016/j.actbio.2012.10.016 & BANDIERA A ET AL: "Phase transition and particle formation of a Human Elastin-Like polypeptide", BIOENGINEERING CONFERENCE, 2009 IEEE 35TH ANNUAL NORTHEAST, IEEE, PISCATAWAY, NJ, USA, 3 avril 2009 (2009-04-03), pages 1-2, XP031460701, ISBN: 978-1-4244-4362-8**
- **DATABASE Geneseq [Online] 9 décembre 2010 (2010-12-09), "Human elastin-like polypeptide (HELP)12 sequence, SEQ ID NO: 1 # 3.", XP002766101, extrait de EBI accession no. GSP:AYL00909 Database accession no. AYL00909 & WO 2010/119420 A1 (UNIV DEGLI STUDI TRIESTE [IT]; BANDIERA ANTONELLA [IT]) 21 octobre 2010 (2010-10-21)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 455 250 B1

- GISELLE C. YEO ET AL: "Fabricated Elastin", ADVANCED HEALTHCARE MATERIALS, vol. 4, no. 16, 1 novembre 2015 (2015-11-01), pages 2530-2556, XP055330918, DE ISSN: 2192-2640, DOI: 10.1002/adhm.201400781 cité dans la demande
- STEVEN G. WISE ET AL: "Tropoelastin: A versatile, bioactive assembly module", ACTA BIOMATERIALIA, vol. 10, no. 4, 1 avril 2014 (2014-04-01), pages 1532-1541, XP055330913, AMSTERDAM, NL ISSN: 1742-7061, DOI: 10.1016/j.actbio.2013.08.003 cité dans la demande
- D. V. BAX ET AL: "Cell Adhesion to Tropoelastin Is Mediated via the C-terminal GRKRK Motif and Integrin V 3", JOURNAL OF BIOLOGICAL CHEMISTRY, vol. 284, no. 42, 16 octobre 2009 (2009-10-16), pages 28616-28623, XP055336641, US ISSN: 0021-9258, DOI: 10.1074/jbc.M109.017525

SEQUENCE LISTING

<110> Centre National de la Recherche Scientifique Université Claude Bernard Lyon

<120> Polypeptide dérivé de la tropoélastine et matériau biocompatible le comprenant

<130> 63590

<150> FR1654306
<151> 2016-05-13

<160> 18

<170> PatentIn version 3.5

<210> 1
<211> 6
<212> PRT
<213> Artificial Sequence

<220>

<223> Séquence hydrophobe consensus

<220>
<221> MISC_FEATURE
<222> (1)..(1)
<223> X est Ala, Val, Leu ou Ile.

<220>
<221> MISC_FEATURE
<222> (3)..(3)
<223> X est Ala, Val, Leu ou Ile

<220>
<221> MISC_FEATURE
<222> (4)..(4)
<223> X est Ala, Val, Leu ou Ile.

<400> 1

```
Xaa Gly Xaa Xaa Pro Gly
1               5
```

<210> 2
<211> 7
<212> PRT
<213> Artificial Sequence

<220>
<223> Séquence minimale de réticulation

<400> 2

```
Ala Ala Ala Lys Ala Ala Lys
1               5
```

<210> 3
<211> 11
<212> PRT
<213> Artificial Sequence

<220>
<223> Séquence de réticulation

<400> 3

```
Ala Ala Ala Lys Ala Ala Ala Lys Ala Ala Lys
1               5                   10
```

<210> 4
<211> 6
<212> PRT
<213> Artificial Sequence

<220>
<223> Séquence consensus du domaine C

<220>
<221> MISC_FEATURE

<222> (1)..(1)
<223> X est Ala, Arg, Asn, Asp, Gln, Glu, Gly, His, Ile, Leu, Lys, Met, Phe, Trp, Ser, Thr, Tyr ou Val.

<220>
<221> MISC_FEATURE
<222> (3)..(3)
<223> X est Ala, Arg, Asn, Asp, Gln, Glu, Gly, His, Ile, Leu, Lys, Met, Phe, Trp, Ser, Thr, Tyr ou Val.

<220>
<221> MISC_FEATURE
<222> (4)..(4)
<223> X est Ala, Arg, Asn, Asp, Gln, Glu, Gly, His, Ile, Leu, Lys, Met, Phe, Trp, Ser, Thr, Tyr ou Val.

<400> 4

```
Xaa Gly Xaa Xaa Pro Gly
1               5
```

<210> 5
<211> 4
<212> PRT
<213> Artificial Sequence

<220>
<223> Séquence C-terminale minimale

<400> 5

```
Arg Lys Arg Lys
1
```

<210> 6
<211> 14
<212> PRT
<213> Artificial Sequence

<220>
<223> Séquence C-terminale

<400> 6

```
Gly Gly Ala Cys Leu Gly Lys Ala Cys Gly Arg Lys Arg Lys
1               5                   10
```

<210> 7
<211> 6
<212> PRT
<213> Artificial Sequence

<220>
<223> Séquence hydrophobe consensus

<220>
<221> MISC_FEATURE
<222> (4)..(4)
<223> X est Ala ou Leu.

<400> 7

```
Val Gly Val Xaa Pro Gly
1               5
```

<210> 8
<211> 728
<212> PRT
<213> Artificial Sequence

<220>
<223> Polypeptide

<400> 8

```
Val Gly Val Ala Pro Gly Val Gly Val Leu Pro Gly Val Gly Val Ala
1               5                   10                  15

Pro Gly Val Gly Val Leu Pro Gly Val Gly Val Ala Pro Gly Val Gly
            20                  25                  30

Val Leu Pro Gly Val Gly Val Ala Pro Gly Val Gly Val Leu Pro Gly
        35                  40                  45

Val Gly Val Ala Pro Gly Val Gly Val Leu Pro Gly Val Gly Val Ala
    50                  55                  60

Pro Gly Val Gly Val Leu Pro Gly Ala Ala Ala Lys Ala Ala Ala Lys
65                  70                  75                  80

Ala Ala Lys Val Gly Val Ala Pro Gly Val Gly Val Leu Pro Gly Val
                85                  90                  95
```

```
Gly Val Ala Pro Gly Val Gly Val Leu Pro Gly Val Gly Val Ala Pro
            100                 105                 110

Gly Val Gly Val Leu Pro Gly Val Gly Val Ala Pro Gly Val Gly Val
        115                 120                 125

Leu Pro Gly Val Gly Val Ala Pro Gly Val Gly Val Leu Pro Gly Val
    130                 135                 140

Gly Val Ala Pro Gly Val Gly Val Leu Pro Gly Ala Ala Ala Lys Ala
145                 150                 155                 160

Ala Ala Lys Ala Ala Lys Val Gly Val Ala Pro Gly Val Gly Val Leu
                165                 170                 175

Pro Gly Val Gly Val Ala Pro Gly Val Gly Val Leu Pro Gly Val Gly
            180                 185                 190

Val Ala Pro Gly Val Gly Val Leu Pro Gly Val Gly Val Ala Pro Gly
        195                 200                 205

Val Gly Val Leu Pro Gly Val Gly Val Ala Pro Gly Val Gly Val Leu
    210                 215                 220

Pro Gly Val Gly Val Ala Pro Gly Val Gly Val Leu Pro Gly Ala Ala
225                 230                 235                 240

Ala Lys Ala Ala Ala Lys Ala Ala Lys Val Gly Val Ala Pro Gly Val
                245                 250                 255

Gly Val Leu Pro Gly Val Gly Val Ala Pro Gly Val Gly Val Leu Pro
            260                 265                 270

Gly Val Gly Val Ala Pro Gly Val Gly Val Leu Pro Gly Val Gly Val
        275                 280                 285

Ala Pro Gly Val Gly Val Leu Pro Gly Val Gly Val Ala Pro Gly Val
    290                 295                 300

Gly Val Leu Pro Gly Val Gly Val Ala Pro Gly Val Gly Val Leu Pro
305                 310                 315                 320

Gly Ala Ala Ala Lys Ala Ala Ala Lys Ala Ala Lys Val Gly Val Ala
                325                 330                 335

Pro Gly Val Gly Val Leu Pro Gly Val Gly Val Ala Pro Gly Val Gly
            340                 345                 350
```

```
Val Leu Pro Gly Val Gly Val Ala Pro Gly Val Gly Val Leu Pro Gly
        355                 360                 365

Val Gly Val Ala Pro Gly Val Gly Val Leu Pro Gly Val Gly Val Ala
    370                 375                 380

Pro Gly Val Gly Val Leu Pro Gly Val Gly Val Ala Pro Gly Val Gly
385                 390                 395                 400

Val Leu Pro Gly Ala Ala Ala Lys Ala Ala Ala Lys Ala Ala Lys Val
                405                 410                 415

Gly Val Ala Pro Gly Val Gly Val Leu Pro Gly Val Gly Val Ala Pro
            420                 425                 430

Gly Val Gly Val Leu Pro Gly Val Gly Val Ala Pro Gly Val Gly Val
        435                 440                 445

Leu Pro Gly Val Gly Val Ala Pro Gly Val Gly Val Leu Pro Gly Val
    450                 455                 460

Gly Val Ala Pro Gly Val Gly Val Leu Pro Gly Val Gly Val Ala Pro
465                 470                 475                 480

Gly Val Gly Val Leu Pro Gly Ala Ala Ala Lys Ala Ala Ala Lys Ala
                485                 490                 495

Ala Lys Val Gly Val Ala Pro Gly Val Gly Val Leu Pro Gly Val Gly
            500                 505                 510

Val Ala Pro Gly Val Gly Val Leu Pro Gly Val Gly Val Ala Pro Gly
        515                 520                 525

Val Gly Val Leu Pro Gly Val Gly Val Ala Pro Gly Val Gly Val Leu
    530                 535                 540

Pro Gly Val Gly Val Ala Pro Gly Val Gly Val Leu Pro Gly Val Gly
545                 550                 555                 560

Val Ala Pro Gly Val Gly Val Leu Pro Gly Val Gly Val Ala Pro Gly
                565                 570                 575

Val Gly Val Leu Pro Gly Val Gly Val Ala Pro Gly Val Gly Val Leu
            580                 585                 590

Pro Gly Val Gly Val Ala Pro Gly Val Gly Val Leu Pro Gly Val Gly
```

595 600 605

Val Ala Pro Gly Val Gly Val Leu Pro Gly Val Gly Val Ala Pro Gly
610 615 620

Val Gly Val Leu Pro Gly Val Gly Val Ala Pro Gly Val Gly Val Leu
625 630 635 640

Pro Gly Val Gly Val Ala Pro Gly Val Gly Val Leu Pro Gly Val Gly
645 650 655

Val Ala Pro Gly Val Gly Val Leu Pro Gly Val Gly Val Ala Pro Gly
660 665 670

Val Gly Val Leu Pro Gly Val Gly Val Ala Pro Gly Val Gly Val Leu
675 680 685

Pro Gly Val Gly Val Ala Pro Gly Val Gly Val Leu Pro Gly Val Gly
690 695 700

Val Ala Pro Gly Val Gly Val Leu Pro Gly Gly Gly Ala Cys Leu Gly
705 710 715 720

Lys Ala Cys Gly Arg Lys Arg Lys
725

<210> 9
<211> 737
<212> PRT
<213> Artificial Sequence

<220>
<223> Polypeptide précédé de Flag

<400> 9

```
Cys Asp Tyr Lys Asp Asp Asp Asp Lys Val Gly Val Ala Pro Gly Val
1               5                   10                  15

Gly Val Leu Pro Gly Val Gly Val Ala Pro Gly Val Gly Val Leu Pro
            20                  25                  30

Gly Val Gly Val Ala Pro Gly Val Gly Val Leu Pro Gly Val Gly Val
        35                  40                  45

Ala Pro Gly Val Gly Val Leu Pro Gly Val Gly Val Ala Pro Gly Val
    50                  55                  60

Gly Val Leu Pro Gly Val Gly Val Ala Pro Gly Val Gly Val Leu Pro
65                  70                  75                  80
```

```
Gly Ala Ala Ala Lys Ala Ala Ala Lys Ala Ala Lys Val Gly Val Ala
                85                  90                  95

Pro Gly Val Gly Val Leu Pro Gly Val Gly Val Ala Pro Gly Val Gly
            100                 105                 110

Val Leu Pro Gly Val Gly Val Ala Pro Gly Val Gly Val Leu Pro Gly
        115                 120                 125

Val Gly Val Ala Pro Gly Val Gly Val Leu Pro Gly Val Gly Val Ala
    130                 135                 140

Pro Gly Val Gly Val Leu Pro Gly Val Gly Val Ala Pro Gly Val Gly
145                 150                 155                 160

Val Leu Pro Gly Ala Ala Ala Lys Ala Ala Ala Lys Ala Ala Lys Val
                165                 170                 175

Gly Val Ala Pro Gly Val Gly Val Leu Pro Gly Val Gly Val Ala Pro
            180                 185                 190

Gly Val Gly Val Leu Pro Gly Val Gly Val Ala Pro Gly Val Gly Val
        195                 200                 205

Leu Pro Gly Val Gly Val Ala Pro Gly Val Gly Val Leu Pro Gly Val
    210                 215                 220

Gly Val Ala Pro Gly Val Gly Val Leu Pro Gly Val Gly Val Ala Pro
225                 230                 235                 240

Gly Val Gly Val Leu Pro Gly Ala Ala Ala Lys Ala Ala Ala Lys Ala
                245                 250                 255

Ala Lys Val Gly Val Ala Pro Gly Val Gly Val Leu Pro Gly Val Gly
            260                 265                 270

Val Ala Pro Gly Val Gly Val Leu Pro Gly Val Gly Val Ala Pro Gly
        275                 280                 285

Val Gly Val Leu Pro Gly Val Gly Val Ala Pro Gly Val Gly Val Leu
    290                 295                 300

Pro Gly Val Gly Val Ala Pro Gly Val Gly Val Leu Pro Gly Val Gly
305                 310                 315                 320

Val Ala Pro Gly Val Gly Val Leu Pro Gly Ala Ala Ala Lys Ala Ala
```

325 330 335

Ala Lys Ala Ala Lys Val Gly Val Ala Pro Gly Val Gly Val Leu Pro
340 345 350

Gly Val Gly Val Ala Pro Gly Val Gly Val Leu Pro Gly Val Gly Val
355 360 365

Ala Pro Gly Val Gly Val Leu Pro Gly Val Gly Val Ala Pro Gly Val
370 375 380

Gly Val Leu Pro Gly Val Gly Val Ala Pro Gly Val Gly Val Leu Pro
385 390 395 400

Gly Val Gly Val Ala Pro Gly Val Gly Val Leu Pro Gly Ala Ala Ala
405 410 415

Lys Ala Ala Ala Lys Ala Ala Lys Val Gly Val Ala Pro Gly Val Gly
420 425 430

Val Leu Pro Gly Val Gly Val Ala Pro Gly Val Gly Val Leu Pro Gly
435 440 445

Val Gly Val Ala Pro Gly Val Gly Val Leu Pro Gly Val Gly Val Ala
450 455 460

Pro Gly Val Gly Val Leu Pro Gly Val Gly Val Ala Pro Gly Val Gly
465 470 475 480

Val Leu Pro Gly Val Gly Val Ala Pro Gly Val Gly Val Leu Pro Gly
485 490 495

Ala Ala Ala Lys Ala Ala Ala Lys Ala Ala Lys Val Gly Val Ala Pro
500 505 510

Gly Val Gly Val Leu Pro Gly Val Gly Val Ala Pro Gly Val Gly Val
515 520 525

Leu Pro Gly Val Gly Val Ala Pro Gly Val Gly Val Leu Pro Gly Val
530 535 540

Gly Val Ala Pro Gly Val Gly Val Leu Pro Gly Val Gly Val Ala Pro
545 550 555 560

Gly Val Gly Val Leu Pro Gly Val Gly Val Ala Pro Gly Val Gly Val
565 570 575

```
Leu Pro Gly Val Gly Val Ala Pro Gly Val Gly Val Leu Pro Gly Val
            580                 585                 590

Gly Val Ala Pro Gly Val Gly Val Leu Pro Gly Val Gly Val Ala Pro
        595                 600                 605

Gly Val Gly Val Leu Pro Gly Val Gly Val Ala Pro Gly Val Gly Val
    610                 615                 620

Leu Pro Gly Val Gly Val Ala Pro Gly Val Gly Val Leu Pro Gly Val
625                 630                 635                 640

Gly Val Ala Pro Gly Val Gly Val Leu Pro Gly Val Gly Val Ala Pro
                645                 650                 655

Gly Val Gly Val Leu Pro Gly Val Gly Val Ala Pro Gly Val Gly Val
            660                 665                 670

Leu Pro Gly Val Gly Val Ala Pro Gly Val Gly Val Leu Pro Gly Val
        675                 680                 685

Gly Val Ala Pro Gly Val Gly Val Leu Pro Gly Val Gly Val Ala Pro
    690                 695                 700

Gly Val Gly Val Leu Pro Gly Val Gly Val Ala Pro Gly Val Gly Val
705                 710                 715                 720

Leu Pro Gly Gly Gly Ala Cys Leu Gly Lys Ala Cys Gly Arg Lys Arg
                725                 730                 735

Lys
```

<210> 10
<211> 12
<212> PRT
<213> Artificial Sequence

<220>
<223> Motif dodécapeptide

<400> 10

```
Val Gly Val Ala Pro Gly Val Gly Val Leu Pro Gly
1               5                   10
```

<210> 11
<211> 1767
<212> DNA
<213> Artificial Sequence

<220>
<223> Séquence codant pour polypeptide

<400> 11

catatgtgtg actacaaaga tgatgatgat aaagttggcg ttgctccggg cgttggcgtg 60
ctgccgggcg ttggcgttgc tccgggcgtt ggcgtgctgc cgggtgtggg tgttgcaccg 120
ggtgtcggtg tgctgccggg tgttggcgtc gctccgggtg tgggtgttct gccgggtgtc 180
ggtgtggcac cgggtgttgg cgtcctgccg ggtgtgggcg tggccccggg cgttggtgtg 240
ctgccgggtg cagccgcaaa agctgcggcc aaagcagcta aggttggtgt cgcaccgggc 300
gttggcgttc tgccgggcgt cggtgtggct ccgggtgttg gtgtcctgcc gggcgttggt 360
gtggccccgg gtgtcggcgt gctgccgggt gttggtgtcg caccgggtgt tggcgtgctg 420
ccgggtgtcg gcgttgcacc gggcgtgggt gtcctgccgg gcgtcggtgt tgctccgggc 480
gtgggcgttc tgccgggtgc agcagcaaag gccgccgcga aggccgccaa agtgggtgtc 540
gcaccgggcg tcggcgtcct gccgggtgtc ggcgtggccc cgggtgtggg cgtgctgccg 600
ggcgtgggcg tggccccggg tgttggtgtt ctgccgggcg tgggtgttgc cccgggcgtt 660
ggtgtcctgc cgggtgttgg cgttgccccg ggtgtgggtg tgctgccggg tgtgggcgtc 720
gccccgggcg tgggcgtcct gccgggcgcc gcggccaagg ccgccgcgaa ggcggccaaa 780
gtcggcgtcg caccgggcgt tggtgttctg ccgggtgttg gcgtggcgcc gggcgtcggc 840
gttctgccgg gtgttggtgt ggcgccgggc gtgggcgtat taccgggtgt gggcgtcgca 900
ccgggcgtcg gtgtcctgcc gggcgtgggt gtcgccccgg gtgtgggcgt cctgccgggt 960
gttggtgttg ccccgggcgt gggcgtgctg ccgggtgccg ccgccaaggc cgccgcgaaa 1020
gccgccaaag tcggtgttgc accgggtgtt ggcgttctgc cgggtgtggg tgtcgcgcct 1080
ggcgttggag ttttacctgg cgtgggtgtc gcgccgggcg tgggtggtgt tggagtttta 1140
cctggtgttg gtgtcgcccc gggtgtcggc gttttacctg gtgtcggtgt cgccccgggc 1200
gtgggtgtgc tgccgggcgc agcagccaag gccgccgcta aggccgccaa agtcggtgtc 1260
gcacctggtg ttggtgtgct gccgggcgtc ggcgttgcgc ctggtgttgg agttcttcct 1320
ggtgtgggcg ttgcaccggg agtgggcgtg ttacctggtg ttggcgtcgc ccctggcgtc 1380
ggagtgttac cgggcgttgg tgtggcgccg ggtgtcggcg tattaccggg cgttggcgtc 1440
gcccctggag tcggcgtgct gccgggcgcc gcggcgaagg ccgccgccaa agcagctaaa 1500
gttggcgtcg cccctggggt cggtgtgtta ccgggcgtcg gcgtggcgcc gggtgtcgga 1560
gtcttaccgg gcgtgggcgt ggcgcctggc gtcggtgttc tgccgggtgt gggcgttgcg 1620
ccgggcgtag gcgttttacc cggtgtgggt gtcgcgccgg gtgtgggtgt cctgccgggt 1680
gtcggtgttg cccctggcgt cggtgtatta ccgggcggtg gcgcgtgcct gggtaaagcc 1740
tgtggccgta aacgcaagta agtcgac 1767

<210> 12

<211> 1794
<212> DNA
<213> Artificial Sequence

<220>
<223> Séquence codant pour polypeptide (avec C et Flag)

<400> 12

```
catatgtgtg actacaaaga tgatgatgat aaagttggcg ttgctccggg cgttggcgtg      60
ctgccgggcg ttggcgttgc tccgggcgtt ggcgtgctgc cgggtgtggg tgttgcaccg     120
ggtgtcggtg tgctgccggg tgttggcgtc gctccgggtg tgggtgttct gccgggtgtc     180
ggtgtggcac cgggtgttgg cgtcctgccg ggtgtgggcg tggccccggg cgttggtgtg     240
ctgccgggtg cagccgcaaa agctgcggcc aaagcagcta aggttggtgt cgcaccgggc     300
gttggcgttc tgccgggcgt cggtgtggct ccgggtgttg gtgtcctgcc gggcgttggt     360
gtggccccgg gtgtcggcgt gctgccgggt gttggtgtcg caccgggtgt tggcgtgctg     420
ccgggtgtcg gcgttgcacc gggcgtgggt gtcctgccgg gcgtcggtgt tgctccgggc     480
gtgggcgttc tgccgggtgc agcagcaaag gccgccgcga aggccgccaa agtgggtgtc     540
gcaccgggcg tcggcgtcct gccgggtgtc ggcgtggccc cgggtgtggg cgtgctgccg     600
ggcgtgggcg tggccccggg tgttggtgtt ctgccgggcg tgggtgttgc cccgggcgtt     660
ggtgtcctgc cgggtgttgg cgttgccccg ggtgtgggtg tgctgccggg tgtgggcgtc     720
gccccgggcg tgggcgtcct gccgggcgcc gcggccaagg ccgccgcgaa ggcggccaaa     780
gtcggcgtcg caccgggcgt tggtgttctg ccgggtgttg gcgtggcgcc gggcgtcggc     840
gttctgccgg gtgttggtgt ggcgccgggc gtgggcgtat taccgggtgt gggcgtcgca     900
ccgggcgtcg gtgtcctgcc gggcgtgggt gtcgccccgg gtgtgggcgt cctgccgggt     960
gttggtgttg ccccgggcgt gggcgtgctg ccgggtgccg ccgccaaggc cgccgcgaaa    1020
gccgccaaag tcggtgttgc accgggtgtt ggcgttctgc cgggtgtggg tgtcgcgcct    1080
ggcgttggag ttttacctgg cgtgggtgtc gcgccgggcg tgggtgttct gccgggcgtt    1140
ggcgtggcac ctggtgttgg agttttacct ggtgttggtg tcgccccggg tgtcggcgtt    1200
ttacctggtg tcggtgtcgc cccgggcgtg ggtgtgctgc cgggcgcagc agccaaggcc    1260
gccgctaagg ccgccaaagt cggtgtcgca cctggtgttg gtgtgctgcc gggcgtcggc    1320
gttgcgcctg gtgttggagt tcttcctggt gtgggcgttg caccgggagt gggcgtgtta    1380
cctggtgttg gcgtcgcccc tggcgtcgga gtgttaccgg gcgttggtgt ggcgccgggt    1440
gtcggcgtat taccgggcgt tggcgtcgcc cctggagtcg gcgtgctgcc gggcgccgcg    1500
gcgaaggccg ccgccaaagc agctaaagtt ggcgtcgccc ctggggtcgg tgtgttaccg    1560
```

```
ggcgtcggcg tggcgccggg tgtcggagtc ttaccgggcg tgggcgtggc gcctggcgtc     1620

ggtgttctgc cgggtgtggg cgttgcgccg ggcgtaggcg ttttacccgg tgtgggtgtc     1680

gcgccgggtg tgggtgtcct gccgggtgtc ggtgttgccc ctggcgtcgg tgtattaccg     1740

ggcggtggcg cgtgcctggg taaagcctgt ggccgtaaac gcaagtaagt cgac           1794
```

<210> 13
<211> 728
<212> PRT
<213> Artificial Sequence

<220>
<223> Polypeptide muté N°1

<400> 13

```
Val Gly Val Ala Pro Gly Val Gly Val Leu Pro Gly Val Gly Val Ala
1               5                   10                  15

Pro Gly Val Gly Val Leu Pro Gly Val Gly Val Ala Pro Gly Val Gly
            20                  25                  30

Val Leu Pro Gly Val Gly Val Ala Pro Gly Val Gly Val Leu Pro Gly
        35                  40                  45

Val Gly Val Ala Pro Gly Val Gly Val Leu Pro Gly Val Gly Val Ala
    50                  55                  60

Pro Gly Val Gly Val Leu Pro Gly Ala Ala Ala Lys Ala Ala Ala Lys
65                  70                  75                  80

Ala Ala Lys Val Gly Val Ala Pro Gly Val Gly Val Leu Pro Gly Val
                85                  90                  95

Gly Val Ala Pro Gly Val Gly Val Leu Pro Gly Val Gly Val Ala Pro
            100                 105                 110

Gly Val Gly Val Leu Pro Gly Val Gly Val Ala Pro Gly Val Gly Val
        115                 120                 125

Leu Pro Gly Val Gly Val Ala Pro Gly Val Gly Val Leu Pro Gly Val
    130                 135                 140

Gly Val Ala Pro Gly Val Gly Val Leu Pro Gly Ala Ala Ala Lys Ala
145                 150                 155                 160

Ala Ala Lys Ala Ala Lys Val Gly Val Ala Pro Gly Val Gly Val Leu
                165                 170                 175
```

```
Pro Gly Val Gly Val Ala Pro Gly Val Gly Val Leu Pro Gly Val Gly
            180                 185                 190

Val Ala Pro Gly Val Gly Val Leu Pro Gly Val Gly Val Ala Pro Gly
        195                 200                 205

Val Gly Val Leu Pro Gly Val Gly Val Ala Pro Gly Val Gly Val Leu
    210                 215                 220

Pro Gly Val Gly Val Ala Pro Gly Val Gly Val Leu Pro Gly Ala Ala
225                 230                 235                 240

Ala Lys Ala Ala Ala Lys Ala Ala Lys Val Gly Val Ala Pro Gly Val
                245                 250                 255

Gly Val Leu Pro Gly Val Gly Val Ala Pro Gly Val Gly Val Leu Pro
            260                 265                 270

Gly Val Gly Val Ala Pro Gly Val Gly Val Leu Pro Gly Val Gly Val
        275                 280                 285

Ala Pro Gly Val Gly Val Leu Pro Gly Val Gly Val Ala Pro Gly Val
    290                 295                 300

Gly Val Leu Pro Gly Val Gly Val Ala Pro Gly Val Gly Val Leu Pro
305                 310                 315                 320

Gly Ala Ala Ala Lys Ala Ala Ala Lys Ala Ala Lys Val Gly Val Ala
                325                 330                 335

Pro Gly Val Gly Val Leu Pro Gly Val Gly Val Ala Pro Gly Val Gly
            340                 345                 350

Val Leu Pro Gly Val Gly Val Ala Pro Gly Val Gly Val Leu Pro Gly
        355                 360                 365

Val Gly Val Ala Pro Gly Val Gly Val Leu Pro Gly Val Gly Val Ala
    370                 375                 380

Pro Gly Val Gly Val Leu Pro Gly Val Gly Val Ala Pro Gly Val Gly
385                 390                 395                 400

Val Leu Pro Gly Ala Ala Ala Lys Ala Ala Ala Lys Ala Ala Lys Val
                405                 410                 415

Gly Val Ala Pro Gly Val Gly Val Leu Pro Gly Val Gly Val Ala Pro
            420                 425                 430
```

```
Gly Val Gly Val Leu Pro Gly Val Gly Val Ala Pro Gly Val Gly Val
            435                 440                 445

Leu Pro Gly Val Gly Val Ala Pro Gly Val Gly Val Leu Pro Gly Val
    450                 455                 460

Gly Val Ala Pro Gly Val Gly Val Leu Pro Gly Val Gly Val Ala Pro
465                 470                 475                 480

Gly Val Gly Val Leu Pro Gly Ala Ala Ala Lys Ala Ala Ala Lys Ala
                485                 490                 495

Ala Lys Val Gly Val Ala Pro Gly Val Gly Val Leu Pro Gly Val Gly
            500                 505                 510

Val Ala Pro Gly Val Gly Val Leu Pro Gly Val Gly Val Ala Pro Gly
        515                 520                 525

Val Gly Val Leu Pro Gly Val Gly Val Ala Pro Gly Val Gly Val Leu
    530                 535                 540

Pro Gly Val Gly Val Ala Pro Gly Val Gly Val Leu Pro Gly Val Gly
545                 550                 555                 560

Val Ala Pro Gly Val Gly Val Leu Pro Gly Val Gly Val Ala Pro Gly
                565                 570                 575

Val Gly Val Leu Pro Gly Val Gly Val Ala Pro Gly Val Gly Val Leu
            580                 585                 590

Pro Gly Val Gly Val Ala Pro Gly Val Gly Val Leu Pro Gly Val Gly
        595                 600                 605

Val Ala Pro Gly Val Gly Val Leu Pro Gly Val Gly Val Ala Pro Gly
    610                 615                 620

Val Gly Val Leu Pro Gly Val Gly Val Ala Pro Gly Val Gly Val Leu
625                 630                 635                 640

Pro Gly Val Gly Val Ala Pro Gly Val Gly Val Leu Pro Gly Val Gly
                645                 650                 655

Val Ala Pro Gly Val Gly Val Leu Pro Gly Val Gly Val Ala Pro Gly
            660                 665                 670

Val Gly Val Leu Pro Gly Val Gly Val Ala Pro Gly Val Gly Val Leu
        675                 680                 685
```

Pro Gly Val Gly Val Ala Pro Gly Val Gly Val Leu Pro Gly Val Gly
690 695 700

Val Ala Pro Gly Val Gly Val Leu Pro Gly Gly Gly Ala Cys Leu Gly
705 710 715 720

Lys Ala Cys Gly Arg Lys Pro Arg
725

<210> 14
<211> 728
<212> PRT
<213> Artificial Sequence

<220>
<223> Polypeptide muté N°2

<400> 14

Val Gly Val Ala Pro Gly Val Gly Val Leu Pro Gly Val Gly Val Ala
1 5 10 15

Pro Gly Val Gly Val Leu Pro Gly Val Gly Val Ala Pro Gly Val Gly
20 25 30

Val Leu Pro Gly Val Gly Val Ala Pro Gly Val Gly Val Leu Pro Gly
35 40 45

Val Gly Val Ala Pro Gly Val Gly Val Leu Pro Gly Val Gly Val Ala
50 55 60

Pro Gly Val Gly Val Leu Pro Gly Ala Ala Ala Lys Ala Ala Ala Lys
65 70 75 80

Ala Ala Lys Val Gly Val Ala Pro Gly Val Gly Val Leu Pro Gly Val
85 90 95

Gly Val Ala Pro Gly Val Gly Val Leu Pro Gly Val Gly Val Ala Pro
100 105 110

Gly Val Gly Val Leu Pro Gly Val Gly Val Ala Pro Gly Val Gly Val
115 120 125

Leu Pro Gly Val Gly Val Ala Pro Gly Val Gly Val Leu Pro Gly Val
130 135 140

Gly Val Ala Pro Gly Val Gly Val Leu Pro Gly Ala Ala Ala Lys Ala
145 150 155 160

```
Ala Ala Lys Ala Ala Lys Val Gly Val Ala Pro Gly Val Gly Val Leu
                165                 170                 175

Pro Gly Val Gly Val Ala Pro Gly Val Gly Val Leu Pro Gly Val Gly
            180                 185                 190

Val Ala Pro Gly Val Gly Val Leu Pro Gly Val Gly Val Ala Pro Gly
        195                 200                 205

Val Gly Val Leu Pro Gly Val Gly Val Ala Pro Gly Val Gly Val Leu
    210                 215                 220

Pro Gly Val Gly Val Ala Pro Gly Val Gly Val Leu Pro Gly Ala Ala
225                 230                 235                 240

Ala Lys Ala Ala Ala Lys Ala Ala Lys Val Gly Val Ala Pro Gly Val
                245                 250                 255

Gly Val Leu Pro Gly Val Gly Val Ala Pro Gly Val Gly Val Leu Pro
            260                 265                 270

Gly Val Gly Val Ala Pro Gly Val Gly Val Leu Pro Gly Val Gly Val
        275                 280                 285

Ala Pro Gly Val Gly Val Leu Pro Gly Val Gly Val Ala Pro Gly Val
    290                 295                 300

Gly Val Leu Pro Gly Val Gly Val Ala Pro Gly Val Gly Val Leu Pro
305                 310                 315                 320

Gly Ala Ala Ala Lys Ala Ala Ala Lys Ala Ala Lys Val Gly Val Ala
                325                 330                 335

Pro Gly Val Gly Val Leu Pro Gly Val Gly Val Ala Pro Gly Val Gly
            340                 345                 350

Val Leu Pro Gly Val Gly Val Ala Pro Gly Val Gly Val Leu Pro Gly
        355                 360                 365

Val Gly Val Ala Pro Gly Val Gly Val Leu Pro Gly Val Gly Val Ala
    370                 375                 380

Pro Gly Val Gly Val Leu Pro Gly Val Gly Val Ala Pro Gly Val Gly
385                 390                 395                 400

Val Leu Pro Gly Ala Ala Ala Lys Ala Ala Ala Lys Ala Ala Lys Val
                405                 410                 415
```

Gly Val Ala Pro Gly Val Gly Val Leu Pro Gly Val Gly Val Ala Pro
420 425 430

Gly Val Gly Val Leu Pro Gly Val Gly Val Ala Pro Gly Val Gly Val
435 440 445

Leu Pro Gly Val Gly Val Ala Pro Gly Val Gly Val Leu Pro Gly Val
450 455 460

Gly Val Ala Pro Gly Val Gly Val Leu Pro Gly Val Gly Val Ala Pro
465 470 475 480

Gly Val Gly Val Leu Pro Gly Ala Ala Ala Lys Ala Ala Ala Lys Ala
485 490 495

Ala Lys Val Gly Val Ala Pro Gly Val Gly Val Leu Pro Gly Val Gly
500 505 510

Val Ala Pro Gly Val Gly Val Leu Pro Gly Val Gly Val Ala Pro Gly
515 520 525

Val Gly Val Leu Pro Gly Val Gly Val Ala Pro Gly Val Gly Val Leu
530 535 540

Pro Gly Val Gly Val Ala Pro Gly Val Gly Val Leu Pro Gly Val Gly
545 550 555 560

Val Ala Pro Gly Val Gly Val Leu Pro Gly Val Gly Val Ala Pro Gly
565 570 575

Val Gly Val Leu Pro Gly Val Gly Val Ala Pro Gly Val Gly Val Leu
580 585 590

Pro Gly Val Gly Val Ala Pro Gly Val Gly Val Leu Pro Gly Val Gly
595 600 605

Val Ala Pro Gly Val Gly Val Leu Pro Gly Val Gly Val Ala Pro Gly
610 615 620

Val Gly Val Leu Pro Gly Val Gly Val Ala Pro Gly Val Gly Val Leu
625 630 635 640

Pro Gly Val Gly Val Ala Pro Gly Val Gly Val Leu Pro Gly Val Gly
645 650 655

Val Ala Pro Gly Val Gly Val Leu Pro Gly Val Gly Val Ala Pro Gly

```
                660                 665                 670

Val Gly Val Leu Pro Gly Val Gly Val Ala Pro Gly Val Gly Val Leu
        675                 680                 685

Pro Gly Val Gly Val Ala Pro Gly Val Gly Val Leu Pro Gly Val Gly
    690                 695                 700

Val Ala Pro Gly Val Gly Val Leu Pro Gly Gly Gly Ala Gly Leu Gly
705                 710                 715                 720

Lys Ala Cys Gly Arg Lys Arg Lys
                725
```

<210> 15
<211> 40
<212> DNA
<213> Artificial

<220>
<223> Sonde sens mutant N°1

<400> 15
aagcctgtgg ccgtaaacct aggtaagtcg acaagcttgc 40

<210> 16
<211> 40
<212> DNA
<213> Artificial

<220>
<223> Sonde antisens mutant N°1

<400> 16
gcaagcttgt cgacttacct aggtttacgg ccacaggctt 40

<210> 17
<211> 29
<212> DNA
<213> Artificial

<220>
<223> Sonde sens mutant N°2

<400> 17
gcgtttacgg ccaccggctt tacccaggc 29

<210> 18
<211> 29
<212> DNA
<213> Artificial

<220>
<223> Sonde antisens mutant N°2

<400> 18
gcctgggtaa agccggtggc cgtaaacgc 29

## Description

La présente invention a pour objet un polypeptide dérivé de la tropoélastine et un matériau biocompatible composite comprenant ledit polypeptide. Elle a également pour objet un procédé de synthèse d'un tel matériau biocompatible ainsi que les applications de ce matériau, notamment dans le domaine biologique et médical, et en particulier pour la culture cellulaire et l'ingénierie tissulaire. L'invention se situe donc dans le domaine de la biochimie des polypeptides et des biomatériaux composites à base de protéines.

Les supports connus d'ingénierie tissulaire, qui peuvent être élaborés à partir de composants naturels tels que notamment le chitosan, le collagène, l'acide hyaluronique ou l'alginate, ou bien de composants synthétiques, tels que les polymères organiques, connaissent tous la même limite due à l'impossibilité actuelle de reproduire un tissu avec des propriétés élastiques analogues aux tissus recherchés. Cette limite résulte notamment de l'absence de supports matriciels adaptés pour créer le microenvironnement présentant un rapport adéquat entre les composants élastiques et viscoélastiques des tissus néosynthétisés et de leurs matrices support. Les supports commerciaux connus à ce jour n'incorporent pas de composante élastique en leur sein, ce qui a pour conséquence immédiate une contraction du tissu implanté, qui résulte en une modification du phénotype cellulaire et une variabilité des réponses biologiques induites.

Dans les applications d'ingénierie tissulaire, les biomatériaux à base de protéines offrent une alternative intéressante aux polymères synthétiques pour leur utilisation potentielle comme structure tridimensionnelle. Le microenvironnement cellulaire influence le comportement des cellules par l'intermédiaire de facteurs biophysiques, mécaniques et biochimiques, spécifiques au tissu. Les biomatériaux doivent donc répondre à de nombreux critères et doivent notamment être capables de reproduire les propriétés des tissus natifs, tout en étant biocompatibles, non immunogènes et biodégradables. Ils doivent également imiter étroitement la fonction de la matrice extracellulaire afin de favoriser l'attachement, la migration et la prolifération cellulaire. L'ingénierie des tissus mous est particulièrement complexe car elle requiert en outre des propriétés d'élasticité fonctionnelle.

On connaît des biomatériaux comprenant des polypeptides dérivés de l'élastine qui incorporent des polypeptides composés de séquences penta-peptidiques répétées, préparés par synthèse chimique, ou bien des protéines synthétisées par voie recombinante (Annabi *et al,* 2013). Ces matériaux présentent des propriétés mécaniques et biochimiques adaptées à des applications d'ingénierie tissulaire en général, cependant leurs propriétés in vivo n'ont pas été étudiées en détail ni de manière spécifique à un ou plusieurs tissus.

On connaît aussi un polypeptide dérivé de la tropoélastine humaine, associé à un groupement RGD (Arginine-Glycine-Acide Aspartique) (Wise *et al,* 2014). Toutefois, afin d'obtenir une tenue structurale suffisante en 3D, ces molécules doivent être mélangées et façonnées avec d'autres polymères plus rigides, naturels ou non, et les biomatériaux résultants sont donc confrontés aux limites citées précédemment, c'est-à-dire la biocompatibilité, la structure, les propriétés mécaniques, l'« informativité biologique » de la charpente, la production en « chimie verte » et la standardisation de la production.

Le brevet EP 1 007 555 décrit un dérivé de la tropoélastine humaine dont la séquence en acides aminés dérive de la séquence de la tropoélastine native. La séquence en acides aminés de ce dérivé est homologue, c'est-à-dire présente au moins 65% d'identité, avec la séquence de la tropoélastine native prise dans son ensemble. Le polypeptide est capable de se lier aux glycosaminoglycanes (GAGs) et son utilisation dans le domaine médical, sous forme liée à un support, notamment un implant, est mentionnée.

Bandiera et al (Biotechnol Appl Biochem 2005, 42, 247-256) décrit la synthèse de polymères protéiques à base d'unité d'élastine humaine.
La demande WO2010/119420 décrit une séquence qui correspond à un polypeptide elastin-like.
Enfin, la demande WO99/03886 décrit des dérivés de tropoélastine.

On connaît aussi des protéines dérivées de l'élastine comprenant des répétitions de séquences pentapeptidiques VPGXG où X représente n'importe quel acide aminé naturel ou modifié à l'exception de la proline (Yeo *et al,* 2015). Ces protéines et leurs produits de dégradation sont biocompatibles, présentent des capacités d'auto-assemblage et des propriétés mécaniques similaires à la protéine native, ainsi qu'une capacité de coacervation. La possibilité d'insérer des séquences bioactives à ces protéines afin de leur conférer des fonctions biologiques est mentionnée. In vitro, les gels formés à partir de ces protéines se sont révélés être une matrice extracellulaire pour des fibroblastes et des neuroblastes (Jeon *et al,* 2011).

Les inventeurs ont maintenant conçu, synthétisé et caractérisé in vitro et in vivo une nouvelle protéine dérivée de la tropoélastine, inspirée par la structure de la tropoélastine humaine et qui présente l'avantage de ne pas comporter de motifs exogènes. Un polypeptide dérivé de l'élastine (ou PDE) selon l'invention comporte des domaines hydrophobes mais également des domaines de réticulation, inspirés eux aussi de la tropoélastine humaine. Un polypeptide selon l'invention est à ce jour le seul polypeptide dérivé de l'élastine (ou PDE) comprenant la région C-terminale de la tropoélastine humaine (ou domaine 36) qui contient à la fois le motif RKRK (SEQ ID N°4), connu pour se lier à des cellules sur l'intégrine $\alpha_V\beta_3$, et un pont disulfure destiné à faciliter l'assemblage des fibres élastiques. Contrairement aux PDE de l'état de l'art, qui sont formés par la répétition de pentapeptides, les domaines hydrophobes d'une PDE selon l'invention consistent en la répétition d'hexapeptides. Un tel motif hexapeptidique est très conservé dans la tropoélastine humaine et possède une activité biologique par l'intermédiaire du récepteur membranaire liant l'élastine (Elastin Binding Protein ou EBP).

Les inventeurs ont démontré qu'une protéine dérivée de l'élastine selon l'invention possède des propriétés d'auto-assemblage et de biodégradabilité. Les structures réticulées comprenant un polypeptide selon l'invention et un agent réticulant, d'une part, ainsi que les structures réticulées comprenant un polypeptide selon l'invention, un agent réticulant bifonctionnel et un polymère hydrophile, d'autre part, possèdent les caractéristiques d'un matériau élastique, bénéficiant ainsi de la contribution biomimétique de la PDE, et sont biocompatibles et biodégradables. De plus, la grande vascularisation et la faible réaction inflammatoire induites lors de l'implantation in vivo de structures réticulées comprenant un polypeptide selon l'invention et un polymère synthétique démontrent le fort potentiel de ces matériaux biocompatibles pour l'ingénierie des tissus élastiques.

Un polypeptide et un matériau biocompatible selon l'invention permettent donc de disposer de biomatériaux modulables et façonnables industriellement. La modulation de la séquence en acides aminés et du poids moléculaire du polypeptide, ainsi que des paramètres mécaniques du matériau permet de reproduire les propriétés mécaniques de tissus élastiques humains ciblés. Un matériau selon l'invention peut être préparé en n'utilisant que des produits et des procédés dits « de chimie verte » et selon un procédé aisément standardisé, qui ne génère pas de composés toxiques ou dangereux, même après dégradation, et ne nécessite pas d'équipement particulier. Enfin, la porosité modulable et la biodégradabilité inattendue d'un matériau biocompatible selon l'invention sont des atouts permettant de faciliter sa colonisation cellulaire et son remplacement par un néo-tissu, qui sont deux critères favorables en médecine régénérative.

Selon un premier aspect, l'invention a pour objet un polypeptide isolé dérivé de la tropoélastine humaine. Dans un mode de réalisation préféré, un polypeptide selon l'invention comprend la séquence en acides aminés SEQ ID N°8 ou consiste en la séquence en acides aminés SEQ ID N°9. L'invention a également pour objet la séquence nucléotidique codant pour un polypeptide selon l'invention.

Selon un deuxième aspect, l'invention a pour objet une structure réticulée biocompatible comprenant un polypeptide dérivé de la tropoélastine selon l'invention. Dans un premier mode de réalisation préféré, une telle structure réticulée comprend un polypeptide selon l'invention et un agent réticulant. Dans un second mode de réalisation préféré, une telle structure réticulée comprend un polypeptide selon l'invention, un agent réticulant et un polymère hydrophile.

Selon un troisième aspect, l'invention a pour objet un procédé de synthèse d'une structure réticulée selon l'invention. Dans un mode de réalisation préféré, un tel procédé comprend le mélange d'un polypeptide dérivé de l'élastine selon l'invention, d'un agent réticulant et d'un polymère hydrophile, suivi du coulage dudit mélange dans un moule approprié et dans des conditions conduisant à la génération de pores au sein du biomatériau, notamment en présence d'un matériau porogène adapté.

Selon un quatrième aspect, l'invention a pour objet un support de culture cellulaire constitué par un matériau biocompatible selon l'invention, et son utilisation in vitro pour la culture de différents types de cellules.

Enfin, selon un cinquième aspect, l'invention a pour objet un matériau biocompatible selon l'invention pour son utilisation pour l'ingénierie tissulaire, et de façon préférée pour la régénération des tissus mous ou pour son utilisation pour la synthèse d'un biomatériau composite, de préférence un biomatériau composite comprenant, ou consistant en, un matériau biocompatible selon l'invention et un autre composé, ledit composé étant constitué par un matériau synthétique ou d'origine naturelle.

## DESCRIPTION DETAILLEE

La présente invention a pour premier objet un polypeptide isolé dérivé de la tropoélastine, selon la revendication 1.

Est également divulgué un polypeptide isolé dérivé de la tropoélastine, dont la séquence en acides aminés comprend, ou consiste en, successivement, de l'extrémité N-terminale à l'extrémité C-terminale dudit polypeptide :

- Un domaine comprenant au moins 2 et au plus 40 répétitions d'une unité comprenant
  - Une région hydrophobe (A) comprenant successivement au moins 6 et au plus 80 séquences $X_1GX_2X_3PG$ (SEQ ID N°1)

    dans lesquelles $X_1$, $X_2$ et $X_3$ représentent un acide aminé hydrophobe indépendamment choisi dans le groupe consistant en : Alanine, Valine, Leucine et Isoleucine ; lesdites SEQ ID N°1 étant identiques ou différentes,

    ou une région hydrophobe (A') comprenant une séquence présentant au moins 65% d'identité avec la séquence de ladite région hydrophobe (A), et

- Une région (B) comprenant au moins la séquence
  AAAKAAK (SEQ ID N°2)
  et de préférence comprenant la séquence
  AAAKAAAKAAK (SEQ ID N°3) ou une région (B') d'au moins 8 acides aminés, comprenant au moins la séquence AAAKAAK (SEQ ID N°2) et présentant au moins 65% d'identité avec la séquence SEQ ID N°3, et
- Un domaine (D) comprenant au moins deux acides aminés cystéine, non consécutifs, et la séquence
  RKRK (SEQ ID N°5),
  et de préférence comprenant la séquence
  GGACLGKACGRKRK (SEQ ID N°6),
  ou une séquence comprenant obligatoirement la séquence SEQ ID N°5 et présentant au moins 65% d'identité avec ladite séquence SEQ ID N°6.

Un polypeptide isolé dérivé de la tropoélastine, selon la présente invention comprend en outre un domaine hydrophobe (C) supplémentaire, ledit domaine (C) étant situé entre les domaines (B) et (D) dudit polypeptide. Ledit domaine hydrophobe supplémentaire (C) comprenant successivement au moins 6 et au plus 20 séquences
$X_4GX_5X_6PG$ (SEQ ID N°4)
dans laquelle $X_4$, $X_5$ et $X_6$ représentent un acide aminé hydrophobe indépendamment choisi dans le groupe consistant en : Alanine, Valine, Leucine et Isoleucine ; lesdites SEQ ID N°4 étant identiques ou différentes.

Selon cet aspect particulier, la présente invention a donc pour objet un polypeptide isolé dérivé de la tropoélastine, dont la séquence en acides aminés comprend successivement, de l'extrémité N-terminale à l'extrémité C-terminale dudit polypeptide :

- Un domaine consistant en au moins 2 et au plus 40 répétitions d'une unité comprenant

  - Une région hydrophobe (A) consistant en successivement au moins 6 et au plus 20 séquences
    $X_1GX_2X_3PG$ (SEQ ID N°1)
    dans lesquelles $X_1$, $X_2$ et $X_3$ représentent un acide aminé hydrophobe indépendamment choisi dans le groupe consistant en : Alanine, Valine, Leucine et Isoleucine ; lesdites SEQ ID N°1 étant identiques ou différentes, et
  - Une région (B) comprenant au moins la séquence
    AAAKAAK (SEQ ID N°2)
    et de préférence comprenant la séquence
    AAAKAAAKAAK (SEQ ID N°3)
  - Un domaine hydrophobe (C) comprenant successivement au moins 6 et au plus 20 séquences
    $X_4GX_5X_6PG$ (SEQ ID N°4)
    dans laquelle $X_4$, $X_5$ et $X_6$ représentent un acide aminé hydrophobe indépendamment choisi dans le groupe consistant en : Alanine, Valine, Leucine et Isoleucine ; lesdites SEQ ID N°4 étant identiques ou différentes, et
  - Un domaine (D) comprenant au moins deux acides aminés cystéine, non consécutifs, et la séquence
    RKRK (SEQ ID N°5),
    et de préférence comprenant la séquence
    GGACLGKACGRKRK (SEQ ID N°6).

Le terme « tropoélastine » désigne un polypeptide d'un poids moléculaire d'environ 72 kDa et dont la séquence en acides aminés est publiquement accessible sous la référence AAC98394 (version AAC98394.1 GI: 182020, datée du 8 mars 2002. Banque NCBI « Protein »). La tropoélastine est codée par un gène unique *ELN* (GID : 2006, MIM : 130160. Banque NCBI « Gene ») dont l'épissage alternatif lors de l'expression de la protéine conduit à l'existence de nombreux variants (ou polymorphes) de la tropoélastine. Le terme de « coacervation » désigne l'agrégation non-covalente des monomères de tropoélastine sous la forme de sphérules, notamment par l'intermédiaire de ses domaines hydrophobes. La liaison covalente de multiples molécules de tropoélastine conduit à la formation de polymères insolubles d'élastine. Les termes « dérivé de la tropoélastine » et « dérivé de l'élastine » sont utilisés dans les documents de l'état de la technique et sont considérés comme équivalents lorsqu'ils désignent un polypeptide non auto-agrégé de façon covalente.

L'expression « dérivé de la tropoélastine » désigne un polypeptide dont la séquence en acides aminés est organisée en domaines successifs comprenant des motifs répétés, cette organisation et ces motifs répétés étant inspirés de la tropoélastine humaine native. Les dérivés de la tropoélastine se distinguent de la tropoélastine native en ce que leur séquence en acides aminés est altérée par rapport à la tropoélastine native par substitution, addition et/ou délétion d'acides aminés.

Le terme « polypeptide isolé » désigne un polypeptide obtenu par une méthode connue par un homme du métier, et notamment par voie recombinante ou par synthèse chimique, ledit polypeptide étant ensuite séparé, par purification partielle ou totale, de son environnement initial. De même, le terme « acide nucléique isolé » désigne un acide nucléique obtenu par une méthode connue par un homme du métier, et notamment selon toute méthode connue du génie génétique ou par synthèse chimique, suivie d'une purification dudit acide nucléique. Un polypeptide isolé selon l'invention diffère donc d'un polypeptide naturel, mais n'est pas limité à une méthode particulière de préparation ou de synthèse. De même, un acide nucléique isolé selon l'invention diffère d'un acide aminé naturel tout en n'étant pas limité à une méthode particulière de synthèse.

De préférence, un polypeptide selon l'invention est produit par voie recombinante, selon des techniques bien connues de l'homme du métier spécialisé dans ce domaine. La production d'une protéine recombinante inclut notamment le choix de l'hôte destiné à la production, par exemple une bactérie, une cellule eucaryote de levure ou de mammifère ou un animal transgénique, et la conception d'une séquence nucléotidique synthétique adaptée à ladite production, notamment par le choix d'un vecteur de production et le choix des codons utilisés afin d'optimiser la production. Une protéine recombinante peut être produite sous la forme d'une protéine de fusion, qui associé la protéine d'intérêt à un domaine « étiquette » (ou « Tag ») destiné à permettre l'identification et/ou la purification de ladite protéine d'intérêt. La purification de la protéine d'intérêt est ensuite réalisée selon une méthode parmi les nombreux protocoles accessibles à l'homme du métier.

L'invention a pour premier objet un polypeptide dérivé de la tropoélastine présentant au moins une des caractéristiques mécanique ou biologique de la tropoélastine prise à l'état naturel, notamment en ce qu'il est capable d'exercer une activité physiologique, même partielle, de la tropoélastine, telle que notamment :

- propriété d'élasticité, incluant notamment le retour à une forme repliée après une distension de la molécule, appliquée dans des conditions appropriées, et/ou
- capacité de coacervation et/ou
- capacité d'auto-agrégation non-covalente, et/ou
- capacité d'agrégation covalente à des molécules d'élastine, et/ou
- capacité d'adhésion cellulaire in vitro et/ou
- capacité d'adhésion cellulaire in vivo et/ou
- capacité à stimuler la prolifération cellulaire et/ou
- capacité d'adhésion à l'intégrine $\alpha V\beta 3$ et/ou
- capacité d'adhésion aux glycosaminoglycanes, et/ou
- sensibilité à la dégradation par une métalloprotéine connue pour dégrader l'élastine, en particulier par la métalloprotéine matricielle-12 (MMP-12).

Ces propriétés peuvent être observées ou mesurées par toute technique de mesure adaptée connue de l'homme du métier.

Selon un aspect particulier, la structure réticulée d'un polypeptide selon l'invention présente des propriétés élastiques, mesurables et quantifiables par un homme du métier mettant en œuvre un ou plusieurs moyens connus pour déterminer les propriétés mécaniques d'un composé. Les tests des propriétés d'élasticité comprennent, sans s'y limiter, un test d'indentation tel que décrit dans la présente demande et tout test appliqué à une telle structure réticulée permettant de déterminer le module d'Young.

De son extrémité N-terminale à son extrémité C-terminale, un polypeptide selon l'invention comprend trois domaines qui sont maintenant décrits de façon détaillée. De son extrémité N-terminale à son extrémité C-terminale, un polypeptide selon l'invention comprend trois domaines, le premier domaine consiste en les régions désignées par (A) et (B), le second et le troisième domaine correspondent respectivement aux régions (C) et (D) telles qu'elles ont été définies précédemment. Les termes « polypeptide » et « protéine » sont équivalents et désignent un polymère d'acides aminés. Les termes « région » et « domaine » désignent une séquence primaire d'acides aminés. Chaque acide aminé est désigné par son nom ou par son abréviation à trois lettres ou à une lettre selon l'IUPAC. L'expression « acide aminé » inclut les acides aminés naturels, les analogues d'acides aminés, les isomères (D) et (L) d'acides aminés et les dérivés d'acides aminés, comme par exemple les acides aminés protégés.

Un polypeptide selon l'invention comprend une séquence reprenant les caractéristiques uniques de la tropoélastine humaine, à savoir des domaines hydrophobes et des domaines de réticulation plus hydrophiles, dont l'alternance est responsable des propriétés de coacervation et d'élasticité. Un polypeptide selon l'invention comprend également, dans sa partie C-terminale, un domaine codé par l'exon 36, contribuant à l'assemblage des fibres élastiques chez l'homme, et comprenant la séquence RKRK (SEQ ID N°5) qui est impliquée dans la liaison à l'intégrine $\alpha V\beta 3$.

L'expression « région hydrophobe » relative à la séquence en acides aminés d'un polypeptide désigne une séquence riche en acides aminés dont la chaîne latérale présente un caractère hydrophobe ou apolaire. Les acides aminés hydrophobes présentent une chaîne aliphatique composée uniquement d'atomes de carbone et d'hydrogène et peuvent être choisis dans le groupe constitué par : alanine, valine, leucine, isoleucine, méthionine, phénylalanine, tryptophane, proline, et leurs analogues

Le caractère hydrophile ou hydrophobe de la chaîne latérale d'un acide aminé est défini par son index d'hydropathie, qui permet de déterminer la nature hydrophobe d'une région d'une protéine grâce à la séquence d'acides aminés. Plus un groupement est hydrophobe, plus l'index d'hydropathie est fort. Le profil d'hydropathie représente la valeur moyenne de l'index d'hydropathie d'un segment de vingt acides aminés centré autour d'une position. Une valeur positive de l'index correspond à un comportement hydrophobe du segment et une valeur négative correspond à un comportement hydrophile.

Selon des aspects particuliers, dans un polypeptide selon l'invention le premier domaine consiste en au moins 2 et au plus 40, de préférence au moins 2 et au plus 35, au moins 2 et au plus 30, au moins 2 et au plus 20, au moins 2 et au plus 15, au moins 2 et au plus 10, et plus préférentiellement 2, 3, 4, 5, 6, 7, 8, 9 ou 10 répétitions d'une unité comprenant :

- une région hydrophobe (A), et
- une région (B).

Selon un aspect particulier d'un polypeptide selon l'invention, la région hydrophobe (A) consiste en au moins 6 et au plus 20 séquences hexapeptidiques $X_1GX_2X_3PG$ (SEQ ID N°1) répétées, et plus préférentiellement 6, 7, 8, 9, 10, 11, 12, 13, 14, 15, 16, 17, 18 ou 19 répétitions de séquences hexapeptidiques $X_1GX_2X_3PG$ (SEQ ID N°1), lesdites SEQ ID N°1 étant identiques ou différentes. Dans la séquence SEQ ID N°1, $X_1$, $X_2$ et $X_3$ représentent un acide aminé hydrophobe indépendamment choisi dans le groupe consistant en : Alanine, Valine, Leucine et Isoleucine.

Il est également décrit un polypeptide pour lequel la séquence en acides aminés de la région hydrophobe (A') présente au moins 65 % d'identité avec la séquence de la région (A) et présente un caractère globalement hydrophobe.

Dans l'expression « présente au moins 65% d'identité avec ladite région », le pourcentage est purement statistique et les différences entre les deux séquences nucléotidiques ou en acides aminés peuvent être réparties au hasard et sur toute leur longueur. Le pourcentage d'identité de séquence entre deux séquences est défini après l'alignement des séquences à comparer. Quand une position dans l'une des séquences est occupée par la même base ou le même acide aminé, les molécules sont identiques à cette position. Un degré d'identité entre deux séquences est une fonction du nombre de positions identiques entre les deux séquences. Les comparaisons de séquence peuvent être effectuées en utilisant tout algorithme à cet effet connu de l'homme du métier.

Il est décrit un polypeptide dont la séquence en acides aminés d'une région hydrophobe (A') présente au moins 65%, de préférence au moins 70%, au moins75 %, au moins 80%, au moins 85%, au moins 90%, 91%, 92%, 93%, 94%, 95%, 96%, 97%, 98%, au moins 99% ou 100% d'identité avec la séquence en acides aminés de la région hydrophobe (A).

Dans un polypeptide selon l'invention, la région (B) est impliquée dans la réticulation du polypeptide et comprend au moins, ou consiste en, la séquence AAAKAAK (SEQ ID N°2). Selon un aspect particulier d'un polypeptide selon l'invention, la région (B) de réticulation comprend au moins, ou consiste en, la séquence AAAKAAAKAAK (SEQ ID N°3). La région (B) est capable de former une structure secondaire de type hélice alpha.

Il est également décrit un polypeptide dont la séquence en acides aminés de la région (B') présente au moins 65% d'identité avec la séquence (B), de préférence au moins 70%, au moins 75%, au moins 80%, au moins 85%, au moins 90%, 91%, 92%, 93%, 94%, 95%, 96%, 97%, 98% au moins 99% ou 100% d'identité avec la séquence en acides aminés de la région (B).

Selon un aspect particulier, un polypeptide selon l'invention comprend un second domaine (C), défini par son caractère hydrophobe et consistant en successivement, au moins 6 et au plus 20 séquences répétées $X_4GX_5X_6PG$ (SEQ ID N°4) dans lesquelles $X_4$, $X_5$ et $X_6$ représentent un acide aminé indépendamment choisi dans le groupe consistant en : Alanine, Valine, Leucine et Isoleucine; lesdites SEQ ID N°4 étant identiques ou différentes. $X_4$, $X_5$ et $X_6$ sont donc choisis indépendamment parmi les acides aminés suivants : alanine, isoleucine, leucine, et valine.

Ledit second domaine hydrophobe (C) consiste en, successivement au moins 6 et au plus 20 séquences répétées $X_4GX_5X_6PG$ (SEQ ID N°4), et plus préférentiellement 6, 7, 8, 9, 10, 11, 12, 13, 14, 15, 16, 17, 18 ou 19 répétitions de séquences hexapeptidiques $X_4GX_5X_6PG$ (SEQ ID N°4), dans lesquelles $X_4$, $X_5$ et $X_6$ représentent un acide aminé hydrophobe indépendamment choisi dans le groupe consistant en : alanine, valine, leucine, isoleucine ; lesdites SEQ ID N°4 étant identiques ou différentes.

Est également décrit un polypeptide dont la séquence en acides aminés de la région (C') présente au moins 65% d'identité avec la séquence (C), de préférence au moins 70%, au moins 75%, au moins 80%, au moins 85%, au moins 90%, 91%, 92%, 93%, 94%, 95%, 96%, 97%, 98% au moins 99% ou 100% d'identité avec la séquence en acides aminés de la région (C).

Un polypeptide selon l'invention comprend un troisième domaine (D) qui comprend au moins deux acides aminés cystéine non consécutifs et la séquence RKRK (SEQ ID N°5). De préférence ledit domaine (D) :

- comprend, ou consiste en, la séquence GGACLGKACGRKRK (SEQ ID N°6), ou
- comprend, ou consiste en, une séquence comprenant obligatoirement la séquence SEQ ID N°5 et présentant au moins 65% d'identité avec ladite séquence SEQ ID N°6.

Selon un aspect particulier, dans un polypeptide selon l'invention les deux acides aminés cystéine du domaine (D) sont séparées par 1, 2, 3, 4, 5, 6, 7 ou 8 acides aminés et de préférence sont séparées par 4 acides aminés ; lesdites cystéines sont capables de former un pont-disulfure intra-chaîne.

La séquence SEQ ID N°6 est codée par l'exon 36 du gène codant pour la tropoélastine humaine, ce domaine est reconnu pour faciliter l'assemblage des fibres élastiques, et pour jouer un rôle dans la signalisation cellulaire et la propriété de liaison aux GAGs de la protéine.

Selon un aspect plus particulier, un polypeptide selon l'invention comprend un domaine (D) comprenant obligatoirement la séquence SEQ ID N°5 et dont la séquence en acides aminés présente au moins 65 % d'identité, de préférence au moins 70%, au moins 75%, au moins 80%, au moins 85%, au moins 90%, 91%, 92%, 93%, 94%, 95%, 96%, 97%, 98% au moins 99 % ou 100% d'identité avec la séquence SEQ ID N°6.

Selon un aspect encore plus particulier, l'invention a pour objet un polypeptide synthétique dérivé de la tropoélastine, dont la séquence en acides aminés comprend successivement, de l'extrémité N-terminale à l'extrémité C-terminale dudit polypeptide, ou consiste en :

- Un domaine consistant en au moins 2 et au plus 40 répétitions d'une unité comprenant :
  - Une région hydrophobe (A) consistant en successivement au moins 6 et au plus 20 séquences $VGVX_7PG$ (SEQ ID N°7) dans lesquelles $X_7$ représente un acide aminé hydrophobe choisi dans le groupe consistant en : alanine et leucine; lesdites SEQ ID N°7 étant identiques ou différentes, et
  - Une région (B) comprenant au moins, ou consistant en, la séquence AAAKAAK (SEQ ID N°2), et de préférence comprenant la séquence AAAKAAAKAAK (SEQ ID N°3), et
- Un domaine hydrophobe (C) consistant en successivement au moins 6 et au plus 20 séquences $VGX_7PG$ (SEQ ID N°7) dans laquelle $X_7$ représente un acide aminé hydrophobe choisi dans le groupe consistant en : alanine et leucine ; lesdites SEQ ID N°7 étant identiques ou différentes, et
- Un domaine (D) comprenant au moins deux acides aminés cystéine non consécutifs et la séquence RKRK (SEQ ID N°5), et de préférence comprenant, ou consistant en, la séquence GGACLGKACGRKRK (SEQ ID N°6).

Selon un aspect encore plus particulier, l'invention a pour objet un polypeptide synthétique dérivé de la tropoélastine, dont la séquence en acides aminés comprend, ou consiste en, successivement, de l'extrémité N-terminale à l'extrémité C-terminale dudit polypeptide:

- Un domaine comprenant, ou consistant en, 6 répétitions d'une unité comprenant :
  - Une région hydrophobe (A) consistant successivement au moins 3 et au plus 10 séquences VGVAPGVGVLPG (SEQ ID N°10), de préférence au moins 5 et au plus 10, et plus préférentiellement 6, 7, 8 ou 9 répétitions d'une séquence en acides aminés VGVAPGVGVLPG (SEQ ID N°10),
  - Une région (B) comprenant, ou consistant en, la séquence AAAKAAAKAAK (SEQ ID N°3),
- Un domaine hydrophobe (C) consistant en successivement au moins 3 et au plus 10 séquences VGVAPGVGVLPG (SEQ ID N°10), de préférence au moins 5 et au plus 10, et plus préférentiellement 6, 7, 8 ou 9 répétitions d'une séquence en acides aminés VGVAPGVGVLPG (SEQ ID N°10), et
- Un domaine (D) comprenant la séquence GGACLGKACGRKRK (SEQ ID N°6).

Selon un aspect encore plus particulier, l'invention a pour objet un polypeptide synthétique dérivé de la tropoélastine, dont la séquence en acides aminés comprend, ou consiste en, successivement, de l'extrémité N-terminale à l'extrémité C-terminale dudit polypeptide:

- Un domaine comprenant, ou consistant en, 6 répétitions d'une unité comprenant :
  - Une région hydrophobe (A) comprenant, ou consistant en, 6 séquences VGVAPGVGVLPG (SEQ ID N°10),
  - Une région (B) comprenant, ou consistant en, la séquence AAAKAAAKAAK (SEQ ID N°3),

- Un domaine hydrophobe (C) comprenant, ou consistant en, 6 séquences VGVAPGVGVLPG (SEQ ID N°10), et
- Un domaine (D) comprenant, ou consistant en, la séquence GGACLGKACGRKRK (SEQ ID N°6).

Selon cet aspect particulier, un polypeptide selon l'invention est caractérisé par une taille, exprimée en poids moléculaire (kDa) qui est proche, ou du même ordre de grandeur que celle de la trophoélastine native, soit 72 KDa, et un point isoélectrique basique, de préférence très basique, et proche du point isoélectrique de la tropoélastine native, soit 10,4.

Selon un aspect encore plus particulier, l'invention a pour objet un polypeptide synthétique dérivé de la tropoélastine, dont la séquence en acides aminés comprend, ou consiste en, la séquence SEQ ID N°8. Ledit polypeptide comporte une alternance de domaines hydrophobes et de réticulation de la tropoélastine, ainsi que sa région C-terminale (domaine 36) selon la séquence primaire en acides aminés suivante :
$H_2N$-[(VGVAPG-VGVLPG)$_6$-(AAAKAAAKAAK)]$_6$-(VGVAPG-VGVLPG)$_6$-GGACLGKACGRKRK-COOH (SEQ ID N°8).

Selon un autre aspect particulier, l'invention a pour objet un polypeptide synthétique dérivé de la tropoélastine tel que défini précédemment associé, sous la forme d'une protéine de fusion, à un polypeptide-étiquette pouvant être aisément identifié et/ou lié par un ligand connu, notamment lors de la production et de la purification du polypeptide dérivé de la tropoélastine, dans la mesure où l'adjonction dudit polypeptide étiquette n'altère pas les propriétés dudit polypeptide dérivé de la tropoélastine. De préférence, un tel polypeptide-étiquette possède les propriétés suivantes : faible charge électrique, pas d'influence sur la structure du polypeptide dérivé de l'élastine selon l'invention, et est notamment choisi parmi les polypeptides suivants : Flag, poly-Histidine, HA, GST, c-Myc, Glu-Glu, V5, biotine, S15. L'homme du métier peut aisément choisir un polypeptide-étiquette parmi ses connaissances générales et les publications accessibles dans le domaine. Selon un aspect particulier, un polypeptide selon l'invention comprend, de son extrémité N-terminale à son extrémité C-terminale, un polypeptide étiquette et un polypeptide dérivé de la tropoélastine. Plus particulièrement, un polypeptide selon l'invention comprend, de son extrémité N-terminale à son extrémité C-terminale, un acide aminé, notamment une cystéine ou tout autre acide aminé, un polypeptide étiquette et un polypeptide dérivé de la tropoélastine.

Selon un aspect encore plus particulier, l'invention a pour objet un polypeptide synthétique dérivé de la tropoélastine, dont la séquence en acides aminés comprend ou consiste en la séquence SEQ ID N°9. Ce polypeptide comprend, de son extrémité N-terminale à son extrémité C-terminale, une cystéine, un polypeptide étiquette et un polypeptide dérivé de la tropoélastine.

Selon un autre aspect, l'invention a pour objet une séquence nucléotidique isolée codant pour un polypeptide selon l'invention.

Selon un aspect particulier, l'invention a pour objet une séquence nucléotidique isolée comprenant ou consistant en la séquence SEQ ID N°11 codant pour le polypeptide selon l'invention de séquence SEQ ID N°8.

Selon un autre aspect particulier, l'invention a pour objet une séquence nucléotidique isolée comprenant ou consistant en la séquence SEQ ID N°12 codant pour le polypeptide selon l'invention de séquence SEQ ID N°9.

Selon un autre aspect, l'invention a pour objet un vecteur nucléotidique comprenant une séquence nucléotidique codant pour un polypeptide, selon l'invention. Par « vecteur » on désigne une molécule d'acide nucléique capable de transporter une séquence nucléotidique à laquelle elle est liée, ou bien de permettre l'expression de la protéine codée par une séquence nucléotidique à laquelle elle est liée de façon opérationnelle (vecteur d'expression).

Selon un aspect particulier, l'invention a pour objet un vecteur nucléotidique comprenant la séquence SEQ ID N°11 codant pour le polypeptide selon l'invention de séquence SEQ ID N°8.

Selon un autre aspect particulier, l'invention a pour objet un vecteur nucléotidique comprenant la séquence SEQ ID N°12 codant pour le polypeptide selon l'invention de séquence SEQ ID N°9.

La présente invention a pour second objet un matériau biocompatible comprenant un polypeptide selon l'invention.

La présente invention a pour second objet un matériau biocompatible comprenant un polypeptide selon l'invention, caractérisée en ce que ledit polypeptide est réticulé. En d'autres termes, ledit polypeptide est auto-assemblé par des liaisons covalentes.

La présente invention a pour second objet un matériau biocompatible comprenant un polypeptide selon l'invention et un agent réticulant.

Le terme « matériau biocompatible » selon l'invention désigne une structure tridimensionnelle, ou réticulée pouvant être acceptée par un être vivant, autrement dit pouvant remplir sa fonction sans effet adverse sur l'environnement biologique dans lequel il est implanté. Un matériau biocompatible selon l'invention est de préférence un hydrogel, c'est-à-dire un matériau comportant une composante solide, constituée par une matrice de chaînes polypeptidiques comprenant éventuellement des chaînes polymère, et une composante liquide constituée par une forte proportion d'eau, d'un liquide aqueux ou d'un fluide biologique.

De préférence, un matériau biocompatible selon l'invention est non-toxique et non-cancérigène. De préférence, un matériau biocompatible selon l'invention est biodégradable, ou résorbable. Le terme « biodégradable » désigne la capacité d'un produit à être dégradé sous l'action d'au moins un organisme vivant, la biodégradabilité s'appréciant en tenant compte du degré de décomposition et du temps nécessaire pour obtenir cette décomposition.

Selon un premier aspect, un matériau biocompatible selon l'invention comprend au moins un polypeptide selon l'invention. Selon un autre aspect, un matériau biocompatible selon l'invention comprend au moins un polypeptide selon l'invention et un agent réticulant.

Selon un deuxième aspect, un matériau biocompatible selon l'invention comprend au moins un polypeptide selon l'invention et un polymère, de préférence hydrophile. Selon un autre aspect, un matériau biocompatible selon l'invention comprend au moins un polypeptide selon l'invention, un polymère, de préférence hydrophile, et un agent réticulant.

Le terme « agent réticulant » désigne un agent capable de créer des liaisons entre les constituants pour former une structure ou un arrangement en réseau. Dans un matériau biocompatible selon l'invention, ledit agent réticulant est choisi parmi les agents non-toxiques et biocompatibles.

Selon le premier aspect d'un matériau biocompatible selon l'invention, dans lequel celui-ci comprend un polypeptide selon l'invention et un agent réticulant, l'agent réticulant peut être une enzyme, un composé chimique réactif ou un agent réticulant traditionnel réagissant sur les amines primaires. Un tel agent réticulant est de préférence choisi dans le groupe constitué par : aldéhydes (tel que glutaraldéhyde), enzymes (transglutaminases, lysyls oxydases), agents réticulant naturels (génipine), ou tout agent homofonctionnel ou hétérofonctionnel dont les groupements réactifs peuvent être les esters activés, les aldéhydes, les carbodiimides, les isothiocyanates, les époxydes, les groupements photoactivables. L'espaceur entre les groupements réactifs est traditionnellement, mais non exclusivement, une chaine polyéthylène glycol ou une chaine aliphatique. Selon un aspect plus particulier, un biomatériau selon l'invention comprend un agent réticulant de préférence choisi dans le groupe constitué par : un agent réticulant naturel (génipine) ou enzymes (lysyls oxydases, transglutaminases), ou les agents bifonctionnels à base de polyéthylène glycol (NHS-PEG-NHS), présentant généralement une faible toxicité.

Selon un second aspect particulier, la présente invention a pour objet un matériau biocompatible comprenant un polypeptide selon l'invention et un polymère hydrophile. Selon cet aspect particulier, ledit polymère hydrophile comprend des groupements réactionnels destinés à permettre la formation de liaisons covalentes avec le polypeptide. Par exemple, ledit polymère peut comprendre des groupements photosensibles (acryliques ou métacryliques), la réticulation du gel ayant lieu, en présence du polypeptide, sous l'effet d'une source lumineuse. Selon un autre aspect particulier, ledit polypeptide et ledit polymère hydrophile sont réticulés par l'intermédiaires d'agents de type « chimie-click ». La chimie click, de type acétylènique-azido ou de type thiol maléimide, nécessite une modification en amont du polymère, cette modification est bien connue de l'homme de l'art.

Le terme « hydrophile » désigne la capacité de créer des liaisons hydrogène avec les molécules d'eau. L'expression « polymère hydrophile » désigne un polymère capable de créer de nombreuses liaisons avec les molécules d'eau, et par conséquent de comprendre une forte proportion de phase aqueuse.

Selon un second aspect particulier, la présente invention a pour objet un matériau biocompatible comprenant un polypeptide selon l'invention, un agent réticulant et un polymère hydrophile.

Dans un matériau biocompatible selon l'invention, ledit polymère hydrophile et l'agent réticulant permettent une réticulation contrôlée autour du polypeptide selon l'invention, il ne s'agit pas de l'adsorption non spécifique du polypeptide à la surface d'un matériau constitué par le polymère hydrophile réticulé.

Selon un autre aspect particulier, la présente invention a pour objet un matériau biocompatible comprenant un polypeptide selon l'invention, un agent réticulant et un polymère hydrophile et ramifié. Le terme « ramifié » est ici équivalent du terme « arborescent » et désigne un polymère dont la structure n'est pas linéaire mais est tridimensionnelle.

Un polymère adapté à un matériau biocompatible selon l'invention présente idéalement les propriétés suivantes : procédé de synthèse standardisé, bon rendement de synthèse, homogénéité, comportement mécanique activité biologique adaptés, possibilités importantes de réactivité chimique, biocompatibilité et permettant une bonne adhérence des cellules.

Selon un aspect encore plus particulier, l'invention a pour objet un matériau biocompatible comprenant un polypeptide selon l'invention, un agent réticulant et un polymère hydrophile, ledit polymère hydrophile étant de préférence ramifié et choisi parmi les polymères de lysine, et notamment parmi les dendrimères de polylysine ou d'autres polymères aminés les polyéthylènes imines(PEI), les polyamidoamines (PAMAM), les kératines, le chitosan.

Au sens de la présente invention, on entend par « dendrimères de polylysine », des polymères arborescents de L-lysine tels ceux décrits par Denkewalter *et al (1981,* 1983) et par Rodriguez-Hernandez *et al* (2003) et Klok *et al* (2002) ainsi que ceux à base de D- ou de L-lysine décrits dans la demande internationale WO2006/114528 et appelés dendrimères greffés de polylysine (DGL). Tous ces dendrimères sont soit constitués uniquement de lysine, la lysine étant soit sous la forme (D) soit sous la forme (L), soit constitués de plus de 30% en mole de lysine et contenant également des unités d'autres aminoacides. Dans le cas où les dendrimères sont constitués uniquement de (D)-Lysine ou de (L)-Lysine on parle d'homopolylysine, dans les autres cas on parle d'hétéropolylysines.

Dans un mode de réalisation avantageux de l'invention, on utilise des dendrimères greffés de polylysine (DGL) de génération 1 à 7, natifs ou modifiés, de préférence des DGL de génération 2, 3, 4 ou 5, et plus préférentiellement des DGL de génération 3. Compte tenu de la très faible taille des DGL de génération 1 (DGL-1), les concentrations de DGL-1 à utiliser pour l'obtention d'un hydrogel conduiraient à un matériau de rigidité plus importante que celle de matériau préparés à partir de DGL de génération 2 ou 3, et présentent donc peu d'intérêt pour l'invention, alors que des dendrimères de générations supérieures à 7 ont tendance à former des agrégats qui rendent difficile leur utilisation.

On entend par dendrimères greffés de polylysine natifs des dendrimères dans lesquels les fonctions amines sont libres. On entend par dendrimères greffés de polylysine modifiés, des dendrimères dont tout ou partie des fonctions amines sont liées de manière covalente ou non à un autre acide aminé, à des fonctions chimiques électropositives (aminés quaternaires, guanidine), à des fonctions hydrophobes telles que les chaines ($C_2$-$C_{18}$)alkyles, des fonctions cholestérols, ou des poly-éthylène-glycol. L'homme du métier saura réaliser les modifications de ces groupes amines en faisant appel à ses connaissances générales ou aux techniques décrites dans la littérature, comme par exemple celles décrites par Rossi J.C. *et al* (2012). Il saura également choisir le type de modifications à apporter en fonction du matériau biocompatible à synthétiser.

Selon un aspect encore plus particulier, l'invention a pour objet un matériau biocompatible comprenant un polypeptide selon l'invention, un agent réticulant et un polymère hydrophile, ledit polymère hydrophile étant un dendrimère greffé de polylysine, constitué uniquement de L-lysine, c'est-à-dire un homopolymère de L-lysine et qui constitue une charpente polycationique, de préférence arborescente.

Selon un aspect encore plus particulier, un polymère d'un matériau biocompatible selon l'invention est un dendrimère greffé de polylysine préparé selon le procédé décrit dans la demande internationale publiée sous le numéro WO 2006/114528. Ce polymère est modulable en taille, selon son niveau de génération, un comportement mécanique et une activité biologique spécifiques pouvant être attribués aux différents polymères selon leur taille.

De préférence, dans un matériau selon l'invention, le dendrimère greffé de polylysine est un dendrimère de génération 3, préparé selon le procédé décrit dans la demande internationale publiée sous le numéro WO 2006/114528, avec un poids moléculaire moyen d'environ 15000 à environ 30000 Da, de préférence environ 21500 Da, plus préférentiellement d'environ 22000 Da, une polydispersité d'environ 1,4 et d'environ 80 à environ 170, notamment environ 123 groupements -$NH_2$ externes libres. Ce dendrimère greffé de polylysine présente l'avantage d'offrir un bon rendement de synthèse, une homogénéité, un bon comportement mécanique et une bonne activité biologique lorsqu'il il est réticulé avec un polypeptide selon l'invention. Il permet une réactivité chimique importante par la présence de multiples fonctions amines primaires. Il est biocompatible et totalement non immunogène, contrairement à la polylysine non linéaire. Il permet une bonne adhérence et un bon étalement des cellules, entraînant très peu de différentiation et permettant une activation de la transcription d'un gène codant pour une protéine de surface, la sous-unité $\alpha$5 des intégrines. De plus, il est furtif vis-à-vis du système immunitaire, il n'induit donc pas la production d'anticorps dirigés contre les dendrimères de lysine.

Selon un autre aspect particulier, l'invention a pour objet un matériau biocompatible selon l'invention comprenant un polypeptide selon l'invention, un agent réticulant et un polymère hydrophile, dans lequel ledit agent réticulant est un agent homo-bifonctionnel ou hétéro-bifonctionnel.

Selon un aspect plus particulier, un matériau selon l'invention comprend un agent réticulant bifonctionnel comprenant une chaîne de polyéthylène glycol, de longueur variable, de préférence comprise entre 200 Da et 20000 Da, de préférence 2000 Da, fonctionnalisée à chacune de ses extrémités par un groupement capable d'interagir avec les fonctions de type amine primaire, et notamment avec la chaîne latérale d'une lysine.

Selon un aspect plus particulier, un matériau selon l'invention comprend un agent réticulant bifonctionnel, fonctionnalisé à chacune de ses extrémités par une fonction ester activée, par 2-(N-Succinimidyl-succinylamino)ethyl (NHS) ou par toute autre fonction chimique capable d'interagir avec les fonctions de type amine primaire.

Selon un aspect encore plus particulier, un matériau selon l'invention comprend un agent réticulant comprenant une chaîne de PEG fonctionnalisée à chacune de ses extrémités par une fonction ester activée, par 2-(N-Succinimidyl-succinylamino)ethyl (PEG-NHS).

Selon un aspect encore plus particulier de l'invention, ledit matériau biocompatible est caractérisé en ce que :

- ledit polypeptide comprend la séquence en acides aminés SEQ ID N°8, et de préférence comprend la séquence SEQ ID N°9
- ledit polymère est un dendrimère greffé de polylysine de génération 3, et
- ledit agent réticulant est bifonctionnel et comprend une chaîne de polyéthylène glycol (PEG) fonctionnalisée à chacune de ses extrémités, de préférence par une fonction ester activée, par 2-(N-Succinimidyl-succinylamino)ethyl (PEG-NHS).

La présente invention a pour troisième objet un procédé de préparation d'un matériau biocompatible selon l'invention, ledit procédé comprenant une étape de mélange d'un polypeptide selon l'invention et d'un agent réticulant.

L'homme du métier déterminera aisément, selon le choix dudit agent réticulant, qu'il soit de nature chimique ou enzymatique, les conditions réactionnelles nécessaires pour que la réticulation ait lieu.

Selon un aspect particulier d'un procédé selon l'invention, le mélange d'un polypeptide selon l'invention et d'un agent réticulant est réalisé dans les conditions suivantes :

- Concentration en agent réticulant d'au moins 10 mM, et de préférence de 50 mM, l'agent réticulant étant notamment la génipine ou un agent comprenant une chaîne de PEG fonctionnalisée à chacune de ses extrémités par 2-(N-Succinimidyl-succinylamino)ethyl (PEG-NHS ou NHS-PEG-NHS).
- Concentration en polypeptide selon l'invention, d'au moins 1 mg/ml, de préférence comprise entre 1 et 100 mg/ml, et de préférence de 50 mg/ml.
- Milieu réactionnel constitué par un tampon, tel que du PBS (NaCl 150 mM, pH 7,4),
- Température : pour un agent de type génipine : comprise entre 35 et 45°C, de préférence comprise entre 36 et 44°C, de préférence d'environ 42°C, et préférentiellement de 42°C ; pour un agent de type PEG-NHS : comprise entre 4 et 45°C, de préférence comprise entre 4 et 25°C, préférentiellement de 4°C,
- Durée de réaction de durée comprise entre plusieurs minutes et plusieurs heures, la réaction comprenant typiquement une phase de mise en contact du polypeptide et de l'agent réticulant, d'une durée comprise entre 5 et 20 minutes, et de préférence d'environ 15 minutes pour un agent de type PEG-NHS, ou d'une durée comprise entre 1 et 24 heures, et de préférence 16 heures pour un agent de type génipine, suivie d'un lavage, le matériau obtenu est ensuite déposé dans tout solvant ou solution adaptée, notamment du PBS à pH 7,4.
- Optionnellement, le matériau peut être séché ou lyophilisé, puis héhydraté dans tout solvant ou solution adaptée, notamment du PBS à pH 7,4.

La concentration des différents composants permet de moduler les différentes propriétés du matériau biocompatible. Le taux d'agent réticulant permet de varier la rigidité du biomatériau, le taux de polypeptide élastique permet de moduler la composante élastique du matériau.

Le mélange d'un polypeptide selon l'invention et d'un agent réticulant conduit à la gélification de l'ensemble, pour générer un matériau de type élastomère, un élastomère étant défini comme un polymère présentant des propriétés élastiques, obtenues après réticulation. Le contrôle de la gélification permet de manipuler les éléments à l'état soluble pour imposer une forme au matériau. De plus, la concentration des différents composants permet de moduler les différentes propriétés des structures réticulées obtenues par la mise en œuvre du procédé. Le taux de polypeptide élastique permet de moduler la composante élastique de la structure. La préparation des élastomères s'effectue généralement dans des conditions très douces, la gélification s'effectuant à température ambiante et pression atmosphérique et le recours aux solvants organiques est rarement requis.

Selon un aspect encore plus particulier d'un procédé selon l'invention, un hydrogel est préparé par le mélange d'un polypeptide selon l'invention et d'un agent réticulant, en présence d'un polymère hydrophile. Selon un autre aspect d'un procédé selon l'invention, le mélange d'un polypeptide selon l'invention et d'un agent réticulant est réalisé en présence d'un polymère hydrophile et ramifié. Ledit polymère hydrophile est choisi notamment parmi : les polymères de lysine, les polyéthylènes imines(PEI), les polyamidoamines (PAMAM), les kératines et le chitosan. La préparation des hydrogels s'effectue généralement dans des conditions très douces, la gélification s'effectuant à température ambiante et pression atmosphérique et le recours aux solvants organiques est rarement requis.

Selon un aspect encore plus particulier d'un procédé selon l'invention, ledit polymère est un dendrimère greffé de polylysine, de préférence de génération 3, préparé selon le procédé décrit dans la demande internationale publiée sous le numéro WO 2006/114528.

Selon cet aspect particulier d'un procédé selon l'invention de préparation d'un matériau biocompatible, un polypeptide selon l'invention et un polymère hydrophile sont mélangés en présence d'un agent réticulant, ledit agent réticulant étant un agent bifonctionnel choisi parmi les agents comprenant une chaîne de taille variable fonctionnalisée à chacune de ses extrémités par un groupement capable d'interagir avec les fonctions de type amine primaire, et notamment avec la chaîne latérale d'un acide aminé lysine. Selon cet aspect particulier d'un procédé selon l'invention, ledit agent réticulant comprend une chaîne de PEG fonctionnalisée à chacune de ses extrémités par une fonction ester activée par 2-(N-Succinimidyl-succinylamino)ethyl (PEG-NHS).

Le mélange d'un polypeptide selon l'invention, d'un polymère hydrophile et d'un agent réticulant conduit à la gélification de l'ensemble. Selon un aspect particulier, dans un procédé selon l'invention de préparation d'un matériau biocompatible, le mélange d'un polypeptide selon l'invention, d'un agent réticulant et d'un polymère est réalisé dans les conditions suivantes :

- Durée de réaction comprise entre 15 secondes et 10 minutes
- Concentration en agent réticulant d'au moins 20 mg/ml, et de préférence comprise entre 50 et 100 mg/ml
- Concentration en polypeptide selon l'invention, d'au moins 0,1 mg/ml, de préférence comprise entre 1 et 20 mg/ml, et de préférence de 3,75 mg/ml,

- Concentration en polymère d'au moins 25 mg/ml et de préférence comprise entre 25 et 100 mg/ml.

Le taux (ou concentration) d'agent réticulant et de polymère permet de varier la rigidité du biomatériau, le taux (ou concentration) de polypeptide élastique permet de moduler la composante élastique du matériau.

Selon un autre aspect plus particulier d'un procédé selon l'invention, la préparation d'un matériau biocompatible comprend :

- une étape de mélange d'un polypeptide selon l'invention et d'un agent réticulant, en présence ou en absence d'un polymère hydrophile, pour obtenir un matériau biocompatible,
- une étape de coulage dudit matériau biocompatible dans un moule d'une forme définie.

L'étape de coulage permet d'imposer la forme souhaitée au biomatériau.

Selon un autre aspect plus particulier d'un procédé selon l'invention, la préparation d'un matériau biocompatible comprend en outre une étape de génération de pores au sein dudit matériau, les étapes de génération de pores et de coulage pouvant être successives, dans n'importe quel ordre, ou simultanées.

L'organisation macro-environnementale générée par la taille des pores permet d'utiliser le matériau biocompatible en tant que matrice de guidage pour les cellules ou comme matrice provisoire de comblement. Le terme « porosité » désigne le rapport entre le volume des régions vides et le volume total du matériau. Une porosité modulable peut ainsi être réalisée autour de porogènes développés à façon et selon des procédés adaptés. Les pores du gel permettent la diffusion de nutriments vers les cellules ainsi que celle des produits de dégradation du métabolisme cellulaire vers l'extérieur du gel.

Les pores au sein du matériau peuvent être générés par tout procédé connu de l'homme du métier. Selon un premier aspect de l'invention, les pores peuvent être générés par tout procédé chimique ou physique connu de l'homme du métier. Selon un aspect particulier, le matériau biocompatible peut être mélangé à un matériau porogène qui sera ensuite retiré du mélange, selon un procédé connu de l'homme du métier, générant ainsi un matériau biocompatible poreux.

Selon un aspect encore plus particulier d'un procédé selon l'invention, la préparation du matériau biocompatible comprend une étape de coulage dudit matériau biocompatible dans un moule d'une forme définie, en présence d'un matériau porogène, et une étape d'extraction, ou de retrait, dudit matériau porogène.

Selon cet aspect, le procédé permet de réticuler l'hydrogel autour d'échafaudages temporaires, ou porogènes, qui seront retirés ensuite pour laisser place à des lacunes interconnectées à travers lesquelles des cellules pourront migrer librement. Cette formulation permet donc la colonisation de l'hydrogel par des cellules résidentes (in vivo) ou par des cellules isolées dans le cadre de la reconstruction tissulaire in vitro.

Selon cet aspect particulier de l'invention, l'étape de coulage du matériau biocompatible en présence d'un matériau porogène est réalisée dans des conditions permettant la réticulation dudit matériau biocompatible autour dudit matériau porogène.

Dans un procédé selon l'invention, ledit matériau porogène est constitué par tout matériau adapté, choisi notamment parmi les alcanes linéaires ou ramifiés, et notamment la paraffine, et parmi les polymères aliphatiques ou aromatiques.

Selon un aspect particulier d'un procédé selon l'invention, ledit matériau porogène est constitué par de la paraffine, de préférence celle-ci se présente sous la forme de billes de taille calibrée. Le porogène établi consiste donc en un assemblage compact de billes de paraffine aux dimensions calibrées, de 1 micron à 1 millimètre, de préférence entre 10 microns et 500 microns, de préférence de 50 à 250 microns, notamment de 50 à 100 microns, de 100 à 180 microns, et de 180 à 250 microns. Ces assemblages compacts sont réalisés par fusion contrôlée des billes, par une incubation à des températures et des temps contrôlés, ou par centrifugation. L'extraction de la paraffine est réalisée par des bains consécutifs dans de l'alcool éthylique bouillant. Il en résulte des structures poreuses et interconnectées. Ces différentes conditions réactionnelles permettent de moduler finement les propriétés structurelles d'un matériau selon l'invention (porosité, taille des pores, taux et taille d'interconnexion entre les pores).

L'invention a également pour objet un procédé comprenant le coulage d'un matériau composite en présence d'un matériau porogène tel que défini précédemment.

L'invention a donc pour objet un matériau biocompatible tel qu'obtenu par la mise en œuvre d'un procédé selon l'invention, ledit matériau résultant de la réaction entre un polypeptide selon l'invention et un agent réticulant, selon un premier aspect, ou bien ledit matériau résultant de la réaction entre un polypeptide selon l'invention, un polymère hydrophile et un agent réticulant, selon un second aspect.

La présente invention a pour quatrième objet un support de culture cellulaire in vitro comprenant un matériau biocompatible obtenu par un procédé selon l'invention.

Selon un aspect plus particulier, un support de culture cellulaire selon l'invention comprend, ou consiste en, un biomatériau comprenant un polypeptide selon l'invention et un agent réticulant.

Selon un aspect plus particulier, un support de culture cellulaire selon l'invention comprend, ou consiste en, un biomatériau comprenant un polypeptide selon l'invention, un polymère hydrophile et un agent réticulant. Selon cet aspect, ledit polypeptide est comprend la séquence SEQ ID N°8 ou SEQ ID N°9 et/ou ledit polymère est un dendrimère greffé de polylysine de génération 3, obtenu par un procédé décrit dans WO 2006/114528 et/ou ledit agent réticulant est PEG-NSS. De préférence, selon cet aspect ledit support de culture est poreux.

L'organisation macro-environnementale générée par la taille des pores permet d'utiliser la structure réticulée, notamment l'hydrogel, en tant que matrice de guidage pour les cellules ou comme matrice provisoire de comblement. Les inventeurs ont montré les capacités de contrôle de pénétration cellulaire ainsi que de biocompatibilité et de biorésorption d'un matériau selon l'invention. En effet, la comparaison du contrôle de colonisation par des fibroblastes humains de matériaux de différente porosité montre qu'une taille de pore unique (100-180 ou 50-100 μm) offre une colonisation cellulaire moins prononcée qu'un mélange de taille de pores de ratio 50/50 ou 75/25 (100-180/50-100, après 14 jours.

Selon un aspect plus particulier, l'invention a pour objet l'utilisation d'un matériau biocompatible selon l'invention, ou obtenu par un procédé selon l'invention, en tant que support de culture cellulaire in vitro, lesdites cellules pouvant être des cellules isolées ou en suspension dans tout type de milieu, naturel ou synthétique, ou des cellules comprises dans un tissu ou un fragment de tissu, ou des cellules de lignée cellulaire ou dans un environnement particulier. En effet, ce support de culture n'induit pas de mort cellulaire, et permet l'adhésion de tout type de cellule adhérente.

Un support de culture cellulaire selon l'invention peut être utilisé pour cultiver tous types de cellules eucaryotes, d'origine humaine ou animale, et de préférence pour cultiver des cellules choisies dans le groupe suivant : cellules de peau, notamment les cellules du derme, de l'épiderme et/ou de l'hypoderme, kératinocytes, fibroblastes, notamment fibroblastes cutanés, cellules osseuses, chondrocytes, cellules de la paroi vasculaire, notamment cellules endothéliales et cellules musculaires lisses, cellules nerveuses, et toutes cellules présentes dans les tissus mous. Un support de culture cellulaire selon l'invention peut également être utilisé pour cultiver des cellules souches, notamment des cellules souches pluripotentes, des cellules souches multipotentes, des cellules souches unipotentes, ainsi que des cellules progénitrices.

Un support de culture cellulaire selon l'invention peut être ensemencé par des cellules par tout moyen connu.

Un support de culture cellulaire selon l'invention peut être utilisé pour toute application dans le domaine de l'étude de l'organisation matricielle des cellules et/ou des tissus, et notamment dans les domaines de la pharmacologie, de la toxicologie et de la cosmétologie.

La présente invention a pour cinquième objet un matériau biocompatible selon l'invention, ou tel qu'obtenu par un procédé selon l'invention, pour son utilisation chez l'homme ou chez l'animal pour l'ingénierie tissulaire ou pour la stimulation de la régénération tissulaire.

La présente invention a également pour objet un matériau biocompatible selon l'invention, ou tel qu'obtenu par un procédé selon l'invention, pour son utilisation chez l'homme ou chez l'animal pour l'ingénierie tissulaire, et de préférence pour l'ingénierie des tissus mous.

Par « ingénierie tissulaire » on entend la substitution, le renforcement, l'induction et/ou la stimulation d'un tissu, ainsi que le conditionnement de cellules multipotentes préalablement à une implantation. Il est entendu que le terme « conditionnement » a pour objet la mise en contact desdites cellules multipotentes avec le matériau biocompatible, ce dernier servant de support ou de véhicule auxdites cellules multipotentes. Le but de l'ingénierie tissulaire consiste à synthétiser, à partir de matrices contenant les ingrédients nécessaires, un tissu remplaçant un tissu endommagé ou manquant.

Par « tissus mous », on entend les tissus présentant des propriétés élastiques, et notamment, sans que cette liste soit limitative, les tissus de la peau, les tissus cardiovasculaires, notamment les tissus des valves cardiaques et/ou des vaisseaux sanguins, les fibres nerveuses, les tissus ostéo-articulaires, notamment les tissus des ligaments, des disques intervertébraux et du cartilage élastique, les plis vocaux.

Une fois implanté à l'endroit souhaité de l'organisme hôte, le matériau doit résister aux diverses contraintes mécaniques et conserver son volume pendant une durée suffisante, tout en transmettant ces contraintes aux cellules encapsulées. De façon idéale, dans le cas de la régénération tissulaire, il est souhaitable que le matériau se dégrade spontanément pour laisser la place au tissu formé.

Selon un aspect particulier, l'invention a pour objet un matériau biocompatible selon l'invention pour l'ingénierie d'un tissu mou choisi dans le groupe des tissus de la peau, des tissus cardiovasculaires et nerveux, des tissus ostéo-articulaires, des tissus musculaires, des tissus des plis vocaux.

De préférence, un matériau biocompatible selon l'invention est caractérisé par des propriétés mécaniques et biologiques en adéquation avec le tissu pour lequel il sera utilisé. En particulier, l'élasticité du matériau utilisé, caractérisée notamment par son module d'Young, sera compatible, et de préférence proche, de l'élasticité du tissu ciblé.

Selon un aspect particulier, l'invention a pour objet un matériau biocompatible pour l'ingénierie cellulaire cutanée, et notamment pour la stimulation de la cicatrisation cutanée.

Selon un aspect encore plus particulier, l'invention a pour objet un matériau biocompatible pour la stimulation de la cicatrisation cutanée de sujets atteints de brûlures importantes et/ou de sujets âgés atteints de plaies chroniques.

Selon un autre aspect particulier, l'invention a pour objet un matériau biocompatible pour le traitement d'une pathologie relative à l'élastogenèse imparfaite ou d'une pathologie liée à un défaut du tissu conjonctif.

Selon un autre aspect particulier, l'invention a pour objet un matériau biocompatible pour le comblement tissulaire, à visée cosmétologique ou en chirurgie réparatrice, en particulier suite à un curetage profond suivant l'ablation d'une tumeur ou pour la réparation des plis vocaux.

Selon un autre aspect particulier, l'invention a pour objet un matériau biocompatible selon l'invention pour la stimulation de la reconstitution osseuse, pour la stimulation de la régénération et le guidage des fibres musculaires, pour la stimulation de la synthèse de fibres vasculaires ou pour le remplacement de vaisseaux sanguins.

Selon un autre aspect particulier, l'invention a pour objet un matériau biocompatible selon l'invention pour le traitement de pathologies induisant une hyper calcification ou pour la stimulation de la calcification de tissus élastiques, notamment les tissus cardio-vasculaires et ostéoarticulaires.

Enfin, l'invention a pour objet un biomatériau composite comprenant, ou consistant en, un matériau biocompatible selon l'invention et un autre composé, ledit composé étant constitué par un matériau synthétique, notamment un polymère, par un matériau d'origine naturelle, ou par un mélange d'un matériau synthétique et d'un matériau d'origine naturelle. Dans le cas d'un polymère, celui-ci est de préférence un polymère biodégradable d'origine synthétique ou naturelle, choisi notamment parmi : les polyesters, le polyéther, les esters, le polycoprolactone, le collagène et la cellulose. Ledit composé peut se présenter sous toute forme appropriée, et notamment sous la forme de fibres ou de nanofibres. Selon un aspect particulier, ledit biomatériau composite peut comprendre un matériau biocompatible selon l'invention en association avec un autre composé, et il peut notamment comprendre une zone dans laquelle le matériau biocompatible selon l'invention et ledit composé sont associés, et, dans le cas de polymères, sont co-polymérisés.

La structure dudit biomatériau composite est appropriée selon l'application envisagée dudit matériau composite, selon un aspect particulier cette structure mime celle d'un organe du corps humain ou animal, plus particulièrement un vaisseau sanguin ou un micro-vaisseau sanguin.

Selon un autre aspect, l'invention a pour objet un procédé de préparation d'un biomatériau composite selon l'invention, ledit biomatériau composite comprenant, ou consistant en, un matériau biocompatible selon l'invention et un autre composé, ledit procédé comprenant une étape de mélange d'un polypeptide selon l'invention et d'un agent réticulant, et une étape de mise en contact dudit mélange avec ledit composé. L'homme du métier déterminera aisément, selon la nature dudit composé, les conditions réactionnelles nécessaires pour que la réticulation ait lieu et pour que le biomatériau composite souhaité soit obtenu.

Selon un autre aspect, l'invention a pour objet l'utilisation d'un matériau biocompatible selon l'invention pour la préparation d'un biomatériau composite comprenant ledit matériau biocompatible et un autre composé, ledit composé étant constitué par un matériau synthétique, tel que notamment un polymère, par un matériau d'origine naturelle, ou par un mélange de matériaux synthétiques et de matériaux d'origine naturelle.

Les exemples 1 à 7 et les figures 1 à 15 qui suivent illustrent l'invention.

Dans l'exemple 1, la figure 1 montre une représentation schématique d'un mode de réalisation particulier d'un polypeptide selon l'invention, selon lequel ledit polypeptide comprend, de son extrémité N-terminale à son extrémité C-terminale : une cystéine ; un polypeptide- étiquette, représenté par un rectangle ; un premier domaine comprenant la répétition d'une unité, ladite unité comprenant une région hydrophobe (A) répétée, représentée par des chevrons, et une région de réticulation (B), représentée par une spirale ; un second domaine hydrophobe (C), représenté par des chevrons ; et un domaine (D) correspondant au domaine codé par l'exon 36 du gène codant pour la tropoélastine, représenté par une ligne courbe. Le polypeptide Electiv est l'un des modes de réalisation encore plus particulier de l'invention, sa séquence en acides aminés est également représentée dans la figure 1. Le polypeptide Elactiv' comprend, de son extrémité N-terminale à son extrémité C-terminale : une cystéine ; un polypeptide-étiquette Flag ; un premier domaine comprenant six répétition d'une unité, ladite unité comprenant une région hydrophobe répétée 6 fois, et une région de réticulation ; un second domaine hydrophobe répété 6 fois ; et un domaine correspondant au domaine codé par l'exon 36 du gène codant pour la tropoélastine.

La figure 2 montre la caractérisation thermique du polypeptide et le profil de coacervation d'une solution d'Elactiv' 1 mg/ml dans du PBS (pH 7,4) dans la plage de température 20-60°C.

Les figures 3A et 3B montrent l'auto-assemblage de coacervats d'Elactiv'. La figure 3A correspond aux images de microcopie électronique à transmission illustrant les coacervats formés à partir de solutions d'Elactiv' à 120 μg/ml (a), 240 μg/ml (b) et 2 mg/ml (c) dans du PBS (Figure 3A). La figure 3B correspond aux images de microscopie électronique à balayage de coacervats d'Elactiv' formés à partir d'une solution d'Elactiv' à 2 mg/ml dans du PBS et illustrant un ensemble de structures (a), de monomères (b et c) et de sphères coalescentes (d-f).

Les figures 4A et 4B correspondent aux images de microscopie électronique à balayage illustrant des structures d'Elactiv' formées à partir de solutions d'Elactiv' à 2 mg/ml contenant de la génipine 50 mM (figure 4A) et formées à partir de solutions d'Elactiv' à 50 mg/ml contenant de la génipine 50 mM (figure 4B).

Les figures 5A, 5B et 5C montrent le résultat de tests d'indentation sur des hydrogels d'Elactiv' réticulé par la génipine avec une charge normale appliquée de 2 mN (figure 5Ai) ou 4 mN (figure 5Aii), l'énergie restituée par l'hydrogel Elactiv' après le test d'indentation avec une charge normale de 2 mN et de 4 mN sur un hydrogel (figure 5B) et le module d'Young (MPa) de l'hydrogel d'Elactiv' défini selon la charge normale appliquée à l'échantillon pendant le test (figure 5C).

Dans l'exemple 2, la figure 6 montre le nombre de cellules adhérentes sur une surface recouverte par Elactiv' en présence ou en absence d'anticorps anti-intégrine $\alpha V\beta 3$ et de L-Lactose.

Dans l'exemple 3, la figure 7A représente le module complexe (E*) d'hydrogels de différentes compositions de DGL/PEG-NHS, exprimées en rapport de concentrations en mg/ml, en présence (●) ou absence (▲) de PDE à 3,75 mg/ml. La figure 7B représente le module de stockage (E') en présence (■) ou absence (X) de PDE à 3,75 mg/ml, et de perte (E") en présence (♦) ou absence (∗) de PDE à 3,75 mg/ml, et tan delta d'hydrogels de différentes compositions de DGL/PEG-NHS exprimées en rapport de concentrations en mg/ml, en présence (▼) ou absence (▲) de PDE à 3,75 mg/ml.

La figure 8 représente des billes de paraffine de différentes granulométrie (rangée du haut : 250-180 microns, 180-100 microns et 100-50 microns respectivement) et les hydrogels poreux résultant colorés au bleu de coomassie (rangée du bas).

Dans l'exemple 5, la figure 9 représente des peaux-reconstruites obtenues avec des hydrogels (DGL/PEG-NHS 50/50 mg/ml) de porosités différentes : 75 % 100-50 μm/25% 180-100 μm (haut de la figure), et 100 % 180-100 μm (bas de la figure). Les fibroblastes ont colonisé les pores de l'hydrogel et les kératinocytes ont formé une couche cornée en surface.

La figure 10 présente la cicatrisation de plaies cutanées après 2 semaines, plaies laissées vides (haut de la figure) ou comblées par des hydrogels (DGL/PEG-NHS 50/50 mg/ml, contenant 0,875 mg/ml de PDE) poreux (180-100 μm) de 4 mm d'épaisseur (bas de la figure). Les hydrogels (astérisque noire) sont intégralement envahis par des cellules et apparaissent fragmentés dans les limites de la plaie originelle (pointillés).

Dans l'exemple 6, les figures 11A et 11B représentent un biomatériau composite tubulaire hydrogel/nanofibres ; la figure 11A représente de façon schématique la structure du biomatériau composite qui comprend, lors de sa synthèse et du centre vers la périphérie : une tige centrale en verre, une couche de nanofibres, un mélange d'hydrogel et de nanofibres puis le tube de verre utilisé pour le moulage du biomatériau composite. Les trois photographies (de gauche à droite) de la figure 11B indiquent : un tube composite de diamètre interne 1 mm et de diamètre externe 2 mm, un grossissement de ce tube, et une micrographie électronique des nanofibres.

Les figures 12A à 12D représentent, des tubes composites nanofibres/hydrogel caractérisés par les valeurs suivantes :

| | Diamètre total du tube de nanofibres | Diamètre interne du tube d'hydrogel : | Diamètre externe du tube d'hydrogel |
|---|---|---|---|
| 12A | 1 mm | 1 mm | 2 mm |
| 12B | 1 mm | 1 mm | 3 mm |
| 12C | 2 mm | 2 mm | 3 mm |
| 12D | 2 mm | 2 mm | 3 mm |

Dans l'exemple 7, les figures 13A et 13B représentent des histogrammes montrant la mesure des diamètres externes (fig. 13A) et internes (fig. 13B) avec, en ordonnées, la valeur donnée par la mesure expérimentale par analyse d'image sous deux angles de rotation : 0° (barres de gauche) et 90° (barres de droite) et en abscisses, la valeur théorique de ces diamètres.

Les figures 14A, 14B et 14C montrent l'incorporation du polypeptide selon l'invention dans les dépôts d'élastine fibrillaire dans des cultures de fibroblastes dermiques. Les figures 14A et 14B montrent respectivement l'imagerie par fluorescence à 8 jours après la confluence (J8) des fibroblastes dermiques non traités (fig. 14A) ou traités à 5 jours après la confluence (J5) avec 1 μg / ml de polypeptide-rhodamine (fig. 14B). La figure 14C montre l'effet de l'ajout de β-aminopropionitrile (β-APN) (inhibiteur des lysyl oxydases) à 350 μM. La tropoélastine est détectée en utilisant un anticorps anti-tropoélastine. Les noyaux sont contre-colorés avec du 4,6-diamidino-2-phénylindole ou DAPI. Barres d'échelle = 60 μm.

Les figures 15A, 15B et 15C montrent l'incorporation du polypeptide sauvage ou muté dans les dépôts d'élastine fibrillaire dans les cultures de fibroblastes dermiques au moyen de l'imagerie par fluorescence à 8 jours après la confluence (J8) des fibroblastes dermiques traités à 5 jours après la confluence (J5) avec 1 μg / ml de polypeptide-rhodamine. Trois versions du polypeptide ont été testées : forme sauvage (fig. 15A), forme mutée sur le motif RKRK > RKPR (fig. 15B), et forme mutée au niveau du pont disulfure C > G (fig. 15C). La tropoélastine est détectée en utilisant un anticorps anti-tropoélastine. Les noyaux sont contre-colorés avec du DAPI. Barres d'échelle = 100 μm.

**EXEMPLE 1: PREPARATION ET CARACTERISATION MECANIQUE DU POLYPEPTIDE**

**Matériel et méthodes**

Le polypeptide Elactiv 'a été conçu pour se conformer à la structure unique de la tropoélastine humaine. Il comporte une alternance de domaines hydrophobes et de réticulation de la tropoélastine, ainsi que sa région C-terminale (domaine 36) selon la séquence primaire en acides aminés suivante : $H_2N$-C-DYKDDDDK-[(VGVAPG-VGVLPG)$_6$-(AAAKAAAKAAK)]$_6$-(VGVAPG-VGVLPG)$_6$-GGACLGKACGRKRK-COOH (SEQ ID N°9).

Le gène codant pour Elactiv ' a été conçu pour se conformer à la structure unique de la tropoélastine humaine. Il code pour une protéine identifiée par une alternance de domaines hydrophobes et de réticulation de la tropoélastine, ainsi que sa région C-terminale (domaine 36) selon la séquence primaire en acides aminés suivante : H2N-C-DYKDDDDK-[(VGVAPG-VGVLPG)$_6$-(AAAKAAAKAAK)]$_6$-(VGVAPG-VGVLPG)$_6$-GGACLGKACGRKRK-COOH (SEQ ID N°9). La synthèse du gène codant pour Elactiv' a été confiée à la société GenScript (Piscataway, États-Unis). La séquence d'ADN (1794 pb) a été optimisée pour une expression en système procaryote *E. coli* (SEQ ID N°12).

Pour la construction du vecteur d'expression pET30a-Elactiv', le gène codant pour Elactiv' a été cloné dans le vecteur d'expression procaryote pET30a, entre les sites NdeI et SalI. Pour ce faire, le vecteur pET30a a été digéré avec les endonucléases de restriction NdeI, SalI (Promega, Charbonnières-les-Bains, France) et le vecteur linéarisé résultant a été conservé et purifié. Le fragment NdeI/SalI d'Elactiv' et le vecteur linéarisé ont été ligaturés en utilisant l'ADN ligase T4 (Promega) par incubation pendant une nuit à 4 ° C. Le vecteur recombinant pET30a-Elactiv' a été amplifié dans des bactéries compétentes JM109, comme décrit précédemment, puis isolé en utilisant le kit Endofree Plasmid Maxi (Qiagen, Courtaboeuf,France). La digestion des vecteurs recombinants avec l'enzyme de restriction EcoRV a permis de valider la purification du plasmide avant le séquençage. Le vecteurs pET30a- Elactiv' a été stocké à -20°C.

L'expression d'Elactiv' a été réalisée comme suit : le vecteur recombinant pET30a-Elactiv a été utilisé pour transformer des bactéries compétentes BL21 (DE3) (Life Technologies, Saint-Aubin, France). Les bactéries transformées ont été ensemencées sur des plaques Luria-Broth (LB)/agar contenant 50 μg/ml de kanamycine (Sigma-Aldrich, Saint-Quentin-Fallavier, France). Une colonie unique a été utilisée pour inoculer 5 ml de LB contenant 50 μg/ml de kanamycine durant la nuit à 37°C sous agitation constante. La culture a été diluée à 1:100 et l'expression d'Elactiv' a été induite lors de la phase exponentielle de croissance par l'addition de 1 mM d'isopropyl-β-D-thiogalactopyranoside (IPTG) (Sigma-Aldrich) pendant 4 h à 30°C sous agitation constante. Les bactéries ont ensuite été récoltées par centrifugation (10 000 g pendant 10 min à 4°C) et le culot cellulaire résultant congelé pendant une nuit à -80°C. Les bactéries ont été remises en suspension dans du tampon Tris-HCl 50 mM pH 8 et incubées 45 min à 80°C. Après incubation 1 h dans de la glace, les fractions solubles et insolubles ont été récoltées par centrifugation (15 000 g, 20 min à 4 ° C) et analysées sur un gel de polyacrylamide - sodium dodécylsulfate (SDS-PAGE) coloré au bleu de coomassie G-250. Ce protocole a donné lieu à environ 20 mg de Elactiv' par litre de culture, dosée à l'aide du kit de dosage de protéines Quant -iT (Life Technologies).

La protéine Elactiv' a été purifiée partir du lysat soluble de E. coli par une méthode température de transition inverse (Tt), tel que rapporté précédemment [Yeo et al, 2011]. Le procédé de purification implique au moins un cycle répété de solubilisation d'Elactiv' dans du tampon phosphate salin (PBS) à 4°C, suivi d'une précipitation et une centrifugation à 42°C. La pureté du Elactiv' a été évaluée par visualisation sur gel de polyacrylamide dodécylsulfate de sodium (SDS-PAGE) coloré au nitrate d'argent. Elactiv' purifié est passé à travers un filtre de 0,2 μm pour assurer une stérilité, et stocké à - 80°C jusqu'à une utilisation ultérieure.

La caractérisation biochimique d'Elactiv' a été réalisée comme suit : la masse moléculaire théorique (Mw) et le point isoélectrique (pI) d'Elactiv' ont été calculés à partir de sa séquence primaire en acides aminés (Vector NTI Advance 11, Life Technologies). La spectrométrie de masse par *Electron Transfert Dissociation* (ETD) a été réalisée par la plate-forme de Protein Science Facility (PSF) (Institut de Biologie et Chimie des Protéines, Lyon, France) pour confirmer la présence de la liaison disulfure dans la région C-terminale (domaine 36) d'Elactiv'. Pour cela, une fragmentation du peptide [579-594] a été générée en mode ETD, en utilisant la spectrométrie de masse en tandem avec la chromatographie liquide (LC-MS / MS).

Les spectres de Dichroïsme circulaire (DC) en UV lointains d'Elactiv' et de la tropoélastine humaine recombinante (hTE) (Sigma-Aldrich) ont été enregistrés avec un spectromètre Chirascan (Applied photophysique, Leatherhead, Royaume-Uni) calibré avec l'acide 1S-(+)-10-camphosulfonique. Les mesures ont été effectuées de 20°C à 60°C dans une cuve en quartz de 0,1 cm de long (Hellma, Müllheim, Allemagne), avec des concentrations d'Elactiv' et de hTE à environ 0,2 mg.ml$^{-1}$ dans du PBS à pH 7,4. Les spectres ont été mesurés dans une fenêtre de longueurs d'ondes de 180 nm à 260 nm. Les spectres ont été traités avec le logiciel Chirascan pour corriger et lisser la ligne de base. Les unités spectrales ont été exprimées en ellipticité molaire moyenne par résidu.

La coacervation d'Elactiv' et la taille des agrégats en solution ont été mesurées par diffusion dynamique de la lumière (DLS) en utilisant un dispositif Malvern-DLS Zatasizer Nano S ZEN1600 (Malvern Instrument), pour une gamme de températures comprises entre 20°C et 60°C, avec un temps de stabilisation de 5 min. Les échantillons d'Elactiv' ont été préparés à 1 mg / ml dans du PBS à pH 7,4. Onze essais ont été effectués par échantillon pour déterminer la taille des agrégats de particules, afin d'obtenir une valeur moyenne finale à température constante.

Pour l'observation par microscopie électronique à transmission, Elactiv' a été dissout à différentes concentrations (de 120 μg / ml à 2 mg / ml) dans du PBS froid (4°C). Toutes les solutions ont été équilibrées à 42° C pendant 15 min pour induire la coacervation. Une grille formvar-carbon (Electron Microscopy Sciences, Hatfield, Royaume-Uni) a été déposée sur une goutte de chaque échantillon à 42°C. Au bout de 2 minutes, les solutions en excès sur la grille ont été transférées et les échantillons adsorbés ont été fixés avec une solution de glutaraldéhyde à 2%, pendant 2 min à 42°C. Les échantillons ont été lavés trois fois avec de l'eau distillée, et colorés pendant 10 secondes avec de l'acide tungstique phosphoré 2% à pH 7 (KOH 1 M) et séchés à l'air. Les observations ont été réalisées avec un microscope électronique à transmission Philips CM120, équipé d'une caméra numérique CDD (Gatan), au Centre technique des microstructures (Université Lyon 1).

Pour la microscopie électronique à balayage, une solution froide de génipine (Sigma-Aldrich) a été ajoutée à des solutions similaires d'Elactiv' à 4°C (2 mg / ml dans du PBS) pour atteindre une concentration finale de 2 et 50 mM. Toutes les solutions ont été équilibrées à 42°C pendant 15 minutes pour faciliter la coacervation et la réticulation, puis étalées sur des lamelles de verre (Braunschweig, Allemagne), et séchées à l'air à 42°C pendant 15 min. Les lamelles résultantes ont été ensuite lavées plusieurs fois dans du PBS avant la déshydratation dans des solutions d'éthanol graduelles (de 50% à 100%). Les échantillons ont ensuite été trempés dans de l'hexaméthyldisilazane 100% pendant 3 minutes et transférés dans un dessicateur pendant au moins 2 heures pour éviter une contamination de l'eau et évaporer le solvant résiduel. Tous les échantillons ont été montés sur des talons d'aluminium et pulvérisés d'une couche mince (~ 3 nm) de cuivre (Bal-tec MED 020, Leica Microsystèmes SAS, Nanterre, France). L'imagerie a été réalisée à l'aide d'un microscope électronique à balayage Hitachi S800 FEG au Centre Technique des microstructures (Université Lyon 1).

Les hydrogels d'Elactiv' réticulés à la génipine ont été préparés comme suit : Elactiv' purifié a été dissout dans du PBS (NaCl 150 mM, pH 7,4) à une concentration finale de 50 $mg.ml^{-1}$, à 4°C. L'échantillon a été coacervé à 42°C et culotté par centrifugation à 300g dans des tubes de microcentrifugation de 1,5 ml à fond plat jusqu'à une hauteur de 1 mm. Le culot a été recouvert avec 200 μl de génipine 50 mM (Sigma-Aldrich) et réticulé pendant une nuit à 42°C. Le gel a été récupéré et réhydraté dans du PBS à pH 7,4.

Les propriétés mécaniques d'Elactiv' ont été déterminées notamment à l'aide d'un dispositif original d'indentation de faible charge, basée sur la technique développée précédemment pour les tissus biologiques *in vivo* et *in vitro* (Pailler-Mattei *et al,* 2013). Le test d'indentation consiste à enregistrer la profondeur de pénétration d'un indenteur rigide en fonction de la charge normale appliqué, au cours d'une expérience de chargement / déchargement. Dans la présente étude, les tests d'indentation sont effectués en mode de déplacement contrôlé. Le déplacement en Z est obtenu à partir de la table de déplacement National Instrument, et commandé par un capteur de déplacement. Le déplacement maximum au cours du cycle de chargement / déchargement peut atteindre environ 500 μm avec une résolution de 1 μm. Le dispositif expérimental offre une large gamme de vitesses indentation de 1 à 100 $\mu m.s^{-1}$.

Dans la présente étude, deux charges normales différentes ont été appliquées, $F_z$=2 mN et $F_z$=4mN, pour une vitesse d'indentation constante V = 20 $\mu m.s^{-1}$. L'indenteur utilisé était un indenteur sphérique en acier, avec un rayon de courbure R = 1,6 mm. Il n'y avait pas de période d'attente entre le chargement et le déchargement et un seul cycle de chargement a été effectué pour chaque essai. Les tests d'indentation ont été répétés cinq fois sur l'échantillon Elactiv'. L'échantillon a été déposé dans un cristallisoir en verre pour les essais. Les tests d'indentation sont effectués en immersion totale dans du sérum physiologique afin d'éviter ou de minimiser l'effet d'adhérence dû à des phénomènes capillaires entre l'échantillon et l'indenteur.

**Résultats**

**Synthèse** : Elactiv' a été conçu en se conformant à la structure unique de la tropoélastine humaine. Il comporte une alternance de domaines hydrophobes (VGVAPG-VGVLPG) et de réticulation (AAAKAAAKAAK) correspondant à des motifs hautement conservés parmi les espèces, ainsi qu'une région C-terminale (domaine 36) comprenant un pont disulfure et une séquence chargée positivement RKRK. Ce domaine est reconnu pour faciliter l'auto-assemblage de la tropoélastine et son incorporation dans les fibres élastiques. Par ailleurs, un octapeptide FLAG a été ajouté à la région N-terminale pour faciliter la détection d'Elactiv' sans influer sur la structure globale de la protéine ni sur son activité biologique (figure 1). Le gène codant pour Elactiv' a été cloné dans le vecteur d'expression procaryote pET30a. Le vecteur recombinant pET30a-Elactiv' a été utilisé pour transformer des bactéries E. coli BL21 (DE3) compétentes. Les bactéries ont été stimulées avec 1 mM d'IPTG pour induire l'expression d'Elactiv'. Après analyse par SDS-PAGE, Elactiv' a été détectée dans les fractions cellulaires solubles et insolubles du lysat bactérien avec un poids moléculaire apparent d'environ 55 kDa. Les résultats sont comparés pour des cultures induites et non-induites. La purification d'Elactiv' a été faite à partir du lysat *E.Coli* soluble par des cycles successifs de transition inverse (ITC), comme indiqué précédemment (Yeo *et al,* 2011). Les échantillons ont été analyses par SDS-PAGE et le gel a été coloré au nitrate d'argent. Elactiv' a pu être formellement identifiée par western-blot en utilisant un anticorps anti-FLAG.

**Caractérisation physico-chimique d'Elactiv**

Elactiv' a un poids moléculaire théorique de 50,6 kDa et un point isoélectrique très basique de 10.31, du même ordre de grandeur que la tropoélastine humaine (10.4). La LC-MS / MS analysée par ETD a permis de confirmer la présence du pont disulfure dans la région C-terminale.

Le dichroïsme circulaire (CD) a été utilisé pour déterminer les structures secondaires d'Elactiv' en comparaison avec la tropoélastine humaine recombinante (hTE). Les spectres ont été enregistrés dans une solution de PBS à 20, 40 et 60°C, pour une concentration de 0,2 mg / ml. A 20°C, les profils d'Elactiv' et de hTE sont caractérisés par un pic négatif à 198 nm et un épaulement négatif inférieur à 220 nm, ce qui suggère une conformation en hélice gauche polyproline II (PPII), bien que l'épaulement soit moins prononcé dans Elactiv'. En augmentant la température de 20 à 60°C, le pic est légèrement décalé vers le rouge, en particulier pour Elactiv', résultant d'une diminution de la stabilité de l'hélice PPII.

**Caractérisation thermique**

Le profil thermique a révélé que la coacervation d'une solution Elactiv' dans du PBS commence à apparaître lorsque la température atteint 30°C, correspondant à la température de transition (Tt), et une coacervation maximale a été obtenue à 60°C (figure 2). La figure 2 montre le profil de coacervation d'une solution d'Elactiv' 1 mg/ml dans du PBS (pH 7.4) dans la plage de température 20-60°C. La variation de la turbidité de la solution à chaque temperature est exprimée en pourcentage d'un maximum. Par ailleurs, l'analyse en DLS a confirmé une variation de la taille des agrégats d'Elactiv' en suspension qui augmente au-dessus de la Tt dépendant de la température, provoquant une brusque augmentation de la turbidité (Tableau 1).

Tableau 1

| Température (°C) | 20 | 60 | 20 |
|---|---|---|---|
| Taille (nm) | 62,8 | $1,3.10^4$ | 81,9 |

La taille de ces agrégats varie de 60 nm (20°C) à 1104 nm à 60°C. Lorsque la solution d'Elactiv' est refroidie de nouveau à 20°C, les agrégats retrouvent une taille de 80 nm, du même ordre que la condition initiale démontrant la réversibilité du phénomène. Les résultats sont exprimés par la moyenne ± ESM de 3 replicats.

**Auto-assemblage des molécules d'Elactiv'**

Pour étudier davantage les propriétés d'agrégation d'Elactiv', des solutions de 120 µg à 2 mg / ml ont été préparées dans du PBS et chauffées à une température (42°C) supérieure à la température de transition pour induire coacervation. Elactiv' a ensuite été visualisée par coloration négative en microscopie électronique à transmission (TEM) A 120 µg/ ml, Elactiv' forme de petites sphères individuelles de faible densité électronique interne (flèches blanches dans la figure) indiquant de très probables sphères creuses (figure 3Aa). À partir de 240 µg / ml, les sphères ne restent plus individualisées, mais commencent à fusionner formant des agrégats allant jusqu'à 200 nm de diamètre (figure 3Ab). A 2 mg / ml, les agrégats montrent une densité électronique externe plus élevée en raison de la fusion des sphères individuelles d'Elactiv' (flèches blanches dans la figure 3Ac). Cette fusion est également observable en microscopie électronique à balayage de coacervats d'Elastiv' formés à partir de différentes structures d'Elactiv' (3Ba), monomères (3Bb-c) et sphères coalescentes (3Bd-f) formées avec Elactiv' dans du PBS. Les bords des sphères ne sont pas réguliers (figure 3Bc). En plus de s'organiser dans les sphères, à une échelle supérieure, les sphères fusionnent ensemble et se connectent comme des perles sur une chaîne (figure 3Bd), formant ainsi des formes géométriques variées (figure 3Be-Bf).

**Réticulation des structures d'Elactiv'**

Lors de l'observation par microscopie électronique à balayage, en présence de génipine 50mM, les agrégats d'Elactiv' (à 2 mg/ml dans du PBS) forment un réseau très dense ces structures forment des fibres (figure 4A), avec des diamètres similaires à ceux des fibres élastiques *in vivo*. L'interconnexion de ces ensembles fibrilaires fait également apparaître de nouvelles structures vides assimilables à des pores. Lorsque la concentration d'Elactiv' est augmentée à 50 mg / ml avec la génipine 50 mM, la plupart des sphères d'Elactiv' ont déjà coalescé pour former une structure poreuse, mais une petite quantité de sphères isolées peuvent encore être observées sur la surface du réseau, ainsi qu'à l'intérieur des pores (figure 4B).

**Propriétés mécaniques d'Elactiv'**

Pour étudier les propriétés mécaniques, des tests d'indentation ont été réalisé à partir des structures 3D réticulées précédemment. Les courbes d'indentation ont été enregistrées en immersion totale dans du PBS avec une charge normale appliquée de 2 mN (figure 5Ai) ou 4 mN (figure 5Aii), pour une vitesse de pénétration constante V = 20 $\mu m\ s^{-1}$.

Les tests d'indentations permettent de déterminer un module d'Young réduit $E^*$ du réseau d'Elactiv', à partir de la mesure de la raideur de contact normale, $k_z$, correspondant à la pente de la partie initiale de la courbe de décharge. Pour un matériau en volume en contact avec un pénétrateur rigide à symétrie axiale, la raideur normale est liée au module d'Young réduit par [35]:

$$E^* = \frac{\sqrt{\pi}}{2}\frac{K_z}{\sqrt{A}}$$

où A est la surface de contact projetée. E * est définie comme suit : $\frac{1}{E^*} = \frac{1-\nu_1^2}{E_1} + \frac{1-\nu_2^2}{E_2}$, avec $E_1$, $\nu_1$ et $E_2$, $\nu_2$, le module d'Young et le coefficient de Poisson du pénétrateur et de la structure d'Elactiv' respectivement. Dans cette étude, étant donné que E1 (acier) << E2 (Elactiv'), il peut être considéré que E * est la réduction de module apparent d'Young de la structure d'Elactiv', $\frac{1}{E^*} = \frac{1-\nu_1^2}{E_1}$.

L'analyse énergétique de la courbe d'indentation permet d'estimer le rapport d'énergie restituée par la structure d'Elactiv' pendant la phase de déchargement. Le rapport de l'énergie restituée a été noté $\psi$ et défini comme suit :

$$\psi = \frac{\int_{z\max}^{0} F_z\big|_{\text{unloading}}\, dz}{\int_{0}^{z\max} F_z\big|_{\text{loading}}\, dz} \times 100$$

où $F_z|_{loading}$ correspond à la variation de charge normale pendant le chargement, et $F_z|_{unloading}$ qui est la variation de charge normale pendant le déchargement. $Z_{max}$ est la profondeur de pénétration maximale.

Les résultats indiquent que dans la plage de charge normale utilisée, la structure d'Elactiv' présente le même comportement mécanique : Le module d'Young réduit de la structure est compris entre 1,4 MPa et 1,5 MPa (figure 5C, module d'Young (MPa) de l'hydrogel d'Elactiv' défini selon la charge normale appliquée à l'échantillon pendant le test) quelle que soit la charge normale utilisée. Le rapport de l'énergie restaurée est d'environ 80% (figure 5B, énergie restaurée par l'hydrogel Elactiv' après le test d'indentation avec une charge normale de 2 mN et de 4 mN sur un hydrogel).

**EXEMPLE 2** : **Caractérisation des réponses cellulaires au polypeptide**

**Matériels et méthodes**

Pour la culture cellulaire, des fibroblastes dermiques normaux humains (NHDF) isolés à partir de prépuce juvénile (Promocell, Heidelberg, Allemagne) ont été cultivés à 37°C, 5% de $CO_2$ dans un milieu complet: DMEM/F-12 ratio 1:1 (Invitrogen, Cergy Pontoise, France) complété avec 10% de sérum de veau foetal (SVF) (Sigma-Aldrich), 100 ug / ml de pénicilline et 0,1 mg / ml de streptomycine (Sigma-Aldrich). Les cellules ont été ensemencées dans des boîtes Optilux 100 x 20 mm (BD Biosciences Labware, Brumath, France) à une densité minimale de 4 000 $cellules.cm^{-2}$ pour la sous-culture. Les cellules ont été utilisées après un nombre limité de sous-cultures (passage 5-6).

Le kit de dosage de prolifération cellulaire CyQuant (Molecular Probes, Invitrogen, Saint-Aubin, France) a été utilisé conjointement avec un lecteur de microplaque (Infinite® M1000 lecteur de microplaques PRO Tecan, Männedorf, Suisse) pour évaluer l'effet d'Elactiv' sur la viabilité (en phase homogène) et la prolifération (en phase hétérogène) des NHDF.

Avant le test de prolifération, les microplaques ont été adsorbées à 10 υg / ml dans du PBS pendant 2 h à 4°C avec Elactiv', hTE (Sigma-Aldrich), collagène I (Gibco, Invitrogen) ou de l'albumine bovine (BSA fraction V, Euromedex, Souffelweyersheim, France). Les plaques ont ensuite été lavées trois fois avec du PBS. Pour les essais, les cellules ont été ensemencées à une densité de 6000 cellules.$cm^{-2}$ et mises en incubation pendant une nuit dans du milieu complet pour permettre l'adhésion cellulaire. Pour les tests de viabilité, diverses concentrations d'Elactiv'ou du peptide d'élastine VGVAPG (GenScript) dilués dans un milieu complet, ont été ajoutées 24 heures après l'ensemencement des cellules. La viabilité et la prolifération ont été suivies pendant 10 jours. Les milieux de culture ont été rafraîchis tous les 2 jours et pour chaque temps d'arrêt, le milieu a été éliminé et les plaques congelées à -80°C avant analyse selon les recommandations du fabriquant.

L'adhérence cellulaire a été mesurée ainsi : Elactiv' a été adsorbé à la surface de lames de verre à 8 puits Millicell EZ (Millipore Merck) à 10 μg / ml dans du PBS à température ambiante pendant 2 h, puis les lames ont été lavées trois fois dans du PBS. Des NHDF à confluence ont été récoltés par incubation avec de l'EDTA 1 mM à 37 C pendant 5 min. La suspension cellulaire a été centrifugée à 300 g pendant 5 min et le culot cellulaire a été remis en suspension dans 1 ml de milieu sans sérum. La densité cellulaire a été ajustée à 4 x $10^4$ cellules / ml dans du milieu de croissance des fibroblastes (PromoCell). Avant l'ensemencement, les cellules ont été pré-incubées 45 min avec un anticorps bloquant l'intégrine αVβ3 (clone LM609, Merck Millipore, Saint-Quentin en Yvelines, France) à 20 μg / ml et/ou en présence de L-lactose 10 mM (Euromedex). Les cellules ont ensuite été ensemencées pendant 90 minutes sur les lames préalablement fonctionnalisées. Le milieu a été aspiré et les puits ont été doucement rincé une fois avec du PBS. Les cellules ont été fixées avec du paraformaldehyde (Sigma-Aldrich) 4% (v / v) pendant 20 minutes et rincées 3 fois avec du PBS. Le Hoechst (Sigma-Aldrich) et la phalloïdine-TRITC (Sigma-Aldrich) ont été ajoutés aux cellules à 1 μg / ml dans du PBS pendant 15 min à température ambiante. Les cellules ont été rincées 3 fois avec du PBS et les lames ont été montées avec une solution de Permafluor (LabVision, Thermo-scientifique, Illkirch, France). Les cellules ont été visualisées par microscopie à fluorescence avec un microscope Axioplan Imaging (Carl Zeiss, Le Pecq, France) et des photos ont été prises sur un appareil photo fx Coolsnap (Photometrics, Tucson, AZ, USA) pour la quantification des cellules.

Pour la mesure de la cinétique de la dégradation par la protéine MMP-12, la MMP-12 recombinante humaine a été diluée à 50 μg / ml dans du tampon d'essai (Tris 50 mM, $CaCl_2$ 10 mM, NaCl 150 mM, Brij-35 0,05% (p / v) de, pH 7,5) et activée par addition d'acétate de p-aminophénylmercurique (Calbiochem, Molsheim, France) à une concentration finale de 1 mM à 37°C pendant 4 heures. 1 μg d'Elactiv' ou de hTE a été incubé à 30°C en présence de 5 μg / ml de MMP-12 activée seule ou en combinaison avec de l'EDTA 10 mM (contrôle négatif inhibiteur de l'activité MMP). Après 0, 15, 30, 60 et 120 minutes, les réactions ont été arrêtées en ajoutant 10 mM d'EDTA. La capacité de la MMP-12 à dégrader Elactiv' et l'hTE a été évaluée par une analyse Western-Blot en utilisant un anticorps anti-alpha élastine (ab21607, Abcam, Paris, France).

**Résultats**

La biocompatibilité d'Elactiv' a été évaluée en présence de fibroblastes dermiques humains normaux (NHDFs) par des analyses de prolifération cellulaire (quantification de l'ADN) soit en phase homogène (en solution), soit en phase hétérogène (fonctionnalisation de surface).

En solution, le polypeptide est dispersé directement dans le milieu de culture cellulaire dans une gamme de concentration de 0,1 à 10 μg/ml. Pour la fonctionnalisation de surface, il s'agit de l'absorption du polypeptide à la surface des plaques de culture cellulaire. L'adsorption est réalisée à 10μg/ml dans du PBS pendant 2h à 4°C. La plaque est ensuite rincée 3 fois avec du PBS avant l'ensemencement des cellules.

Dans une première expérimentation, les NHDFs ont été incubés de 2 à 10 jours en phase homogène avec du milieu seul ou supplémenté en Elastiv' ou en peptide de l'élastine VGVAPG à 1 μg/ml et 10 μg/ml. Les résultats sont présentés sous la forme : moyenne ± EMS de triplicats. Un test ANOVA suivi d'un post-test Bonferroni ont été réalisés pour la détermination de valeurs p (* $p<0.05$). En ce qui concerne la prolifération en phase homogène, une augmentation significative de la prolifération cellulaire a été observée 10 jours après l'ensemencement avec 10 μg / ml Elactiv ', de manière similaire au peptide d'élastine VGVAPG.

La prolifération cellulaire en phase hétérogène a également été déterminée pendant 10 jours sur des surfaces revêtues d'Elactiv' ou de protéines matricielles contrôles. Les NHDFs ont été incubés de 2 à 10 jours en phase hétérogène sur différents substrats (non traité, BSA, collagène, peptide VGVAPG ou Elactiv'). Le résultat est exprimé par la moyenne de 4 réplicats (moyenne ± ESM). Aucune modification de la prolifération des fibroblastes n'a été observée dans ces conditions de culture.

Pour savoir si le motif C-terminal GRKRK et le motif VGVAPG au sein d'Elactiv', respectivement connus pour se lier à l'intégrine αvβ3 et l'EBP dans la tropoélastine humaine, sont capables d'induire l'attachement cellulaire, l'adhérence des NHDFs sur un revêtement d'Elactiv' a été étudiée avec un anticorps bloquant anti-intégrine αvβ3 et / ou en présence de L-lactose, inhibiteur de l'EBP (figure 6). L'inhibition de NHDFs sur du gel à 10 μg/ml d'Elactiv' en présence ou en absence d'un anticorps anti-intégrine αVβ3 et/ou de L-lactose est montrée. L'anticorps LM609 (anti-αVβ3) et le L-lactose sont utilisés aux concentrations respectives de 20 µg/ml et 3.6 mg/ml. Le résultat est la moyenne de duplicats (± ESM), la signification statistique a été évaluée au regard du contrôle négatif (sans anticorps anti-intégrine αVβ3 ni L-lactose), par un test ANOVA one way suivi d'un post-test Bonferroni. Les résultats statistiques sont présentés ainsi : ** P<0.01, *** P<0.001.

En présence d'anticorps anti-intégrine αvβ3 seul, l'adhérence des cellules a été inhibée de 30% tandis que la supplémentation du milieu de culture avec en L-lactose a donné lieu à une inhibition de 50%. Enfin, lorsque les deux inhibiteurs ont été ajoutés simultanément au milieu de culture, l'adhérence des NHDF a été inhibée de 78%.

Pour déterminer la sensibilité d'Elactiv' à être dégradée par les métalloprotéinases matricielles (MMPs), la cinétique de dégradation par rhMMP-12, MMP reconnue pour cliver au moins 86 sites de la tropoélastine *in vitro,* dont le motif VGVAPG, a été effectuée et analysée western-blot en utilisant un anticorps anti-alpha élastine. Les résultats montrent qu'Elactiv' est dégradé progressivement par la MMP-12, mais dans une moindre mesure que la tropoélastine humaine.

**EXEMPLE 3 : CARACTERISATION D'HYDROGELS ELASTINO-MIMETIQUES**

**Matériels et méthodes**

Les dendrigrafts de lysine (DGL) de troisième génération ont été préparés selon le procédé décrit dans la demande internationale WO2006/114528 par la société Colcom (Montpellier, France). Le O,O'-Bis[2-(N-Succinimidyl-succinylamino)ethyl]polyethylene glycol (PEG-NHS, $M_n$ 2000), l'alcool poly-vinylique (PVA, Mw 13000-23000), le kit Live/Dead Cell Double Staining Kit, la pénicilline et streptomycine, la phalloïdine, le 2-(4-Amidinophenyl)-6-indolecarbamidine dihydrochloride, 4',6-Diamidino-2-phenylindole dihydrochloride (DAPI), le para-formaldéhyde, le dimethyl formamide (DMF) et l'éthanol absolu ont été achetés auprès de Sigma Aldrich (Saint-Louis, MO, USA). La paraffine a été acquise chez Histolab products AB (Västra Frölunda, Suède). Le tampon phosphate (PBS) est commercialisé par Thermo fisher scientific (Waltham, MA, USA). Les tamis de différentes tailles ont été achetés auprès de la verrerie villeurbanaise (Villeurbanne, France). Le film optiskin est fabriqué par Urgo.

Pour former l'hydrogel, une solution de DGL à différentes concentrations en PBS, contenant ou non la PDE à différentes concentrations, est mélangée à une solution d'agent réticulant (PEG-NHS) à différentes concentrations en PBS ou DMF. Pour former 100 µl d'hydrogel, 37,5 µl de PBS contenant ou non la PDE sont ajoutés à 50 µl de solution de DGL et mélangés par vortex. Cette solution est placée sur glace puis 12.5µl de solution de PEG-NHS est ajoutée et mélangée par vortex. La réticulation résultante du gel se produit en conditions statiques à température ambiante.

Les billes de paraffines de différentes granulométries sont préparées par émulsion. 150 ml d'une solution de PVA à 0,5% (masse/volume) dans l'eau sont chauffés à 70°C dans un bécher de 200 ml. En parallèle, 5g de paraffine en paillettes sont mis à fondre dans un bécher en veillant à ce qu'il n'y ait pas d'ébullition. En maintenant la température à 70°C, une forte agitation magnétique est mise en place dans la solution de PVA et la paraffine liquide y est versée. Après 15 minutes, l'émulsion chaude de paraffine dans la solution de PVA est versée dans un bain d'eau froide (sur glace) afin de solidifier instantanément la paraffine. Le tout est ensuite filtré successivement avec des tamis de 250 µm, 180 µm, 100 µm et 50 µm. Les billes de paraffine sont récupérées et séchées au lyophilisateur puis conservées à 4°C.

Les gels poreux sont formés par approches porogènes en réticulant l'hydrogel autour et au sein d'un empilement de billes de paraffine de granulométrie contrôlée. Pour former 100 µl de gel poreux, 100 mg de billes de paraffine sont déposés dans un microtube de 2mL. Les billes sont tassées au fond du tube par action mécanique et par centrifugation (centrifugeuse de paillasse, 2000g, 15 secondes). S'il s'agit d'un mélange de billes de différentes granulométries, il faut préalablement les mélanger par retournement et vortex. Avant ajout de l'hydrogel, le microtube est placé sur glace. 100 µl d'hydrogel avant réticulation est ensuite dépose à la surface de l'empilement de billes de paraffine dans le microtube et immédiatement centrifugé à 2000g durant 2 secondes afin de faire pénétrer le gel entre les billes de paraffine. Le composite contenant le gel et les billes de paraffine est alors laissé pour réticulation en conditions statiques à température ambiante.

Après 2 heures, la paraffine est extraite de l'hydrogel par trois bains successifs d'1 heure dans 300 ml d'éthanol absolu bouillant. Les gels sont ensuite immergés dans de l'eau pure pendant 10 minute puis dans du PBS et stockés à 4 °C.

Les propriétés mécaniques de l'hydrogel ont été mesurées par analyse mécanique dynamique (DMA 3100, Bose ElectroForce, USA). Des disques d'hydrogels de diamètre 12 mm et épaisseur 2 mm sont compressés sinusoïdalement d'une amplitude de 10 %, après pré-compression de 10%, à une fréquence variant de 1 à 100 hz, à température ambiante. Les courbes de force/déplacement obtenues permettent de définir le module complexe d'élasticité (E*), ainsi que le module complexe de stockage (E') et de perte (E") et leur ratio (tan delta) pour chaque fréquence. Chaque composition d'hydrogel est mesurée en triplicata.

**Résultats**

**Caractérisation des hydrogels.**

Les hydrogels élasto-mimétiques sont formés par la combinaison de trois composants. L'action d'un agent réticulant (le PEG-NHS) sur les dendrigrafts de lysine (DGL) et la protéine dérivée de l'élastine (PDE) permet une réticulation efficace et la formation d'un hydrogel. La réticulation s'effectue grâce à la formation de liaisons covalentes amides entre les fonctions N-hydroxysuccinimine (NHS) de l'agent réticulant et les fonctions amines libres des DGL et de la PDE. La réticulation s'opère rapidement (entre 15 secondes et une dizaine de minutes) et dans de nombreuses conditions de concentrations. Les concentrations finales du PEG-NHS et des DGL contrôlent la formation de l'hydrogel ainsi que sa vitesse de réticulation (Tableau 2). Les hydrogels se forment à partir d'une concentration finale (dans l'hydrogel) de PEG-NHS à 50 mg/ml et de DGL à 25 mg/ml. La présence de la PDE dans le mélange ne semble pas induire de différences dans la capacité ou les temps de réticulation.

| | | DGL (mg/ml) | | |
|---|---|---|---|---|
| | | 25 | 50 | 100 |
| PEG-NHS (mg/ml) | 50 | 10 minutes | 30 secondes | |
| | 100 | | | 15 secondes |

Tableau 2 : Vitesse de formation des hydrogels en fonction des concentrations finales de PEG-NHS et de DGL

Les propriétés mécaniques des hydrogels sont également impactées par les concentrations finales des différents constituants. Comme illustré par la figure 7A, le module complexe E* des gels sans PDE peut être varié entre 7 et 121 kPa en augmentant les concentrations de DGL et de PEG-NHS entre respectivement 25 - 100 mg/ml et 50 - 100 mg/ml. La présence de la PDE dans l'hydrogel (3,75 mg/ml) résulte en l'augmentation de E* pour chaque combinaison des autres constituants (E* si situant entre 36 et 397 kPa), indiquant une meilleure résistance à la compression du matériau. Le comportement mécanique des hydrogels est caractérisé par un module de stockage E' plus élevé que le module de perte E", ce qui démontre un comportement élastique (Figure 7B). Ce comportement est amélioré par la présence de la PDE dans l'hydrogel, comme l'indique la diminution de tan delta (et donc la dissipation d'énergie) en présence de PDE. Cette augmentation du module de stockage en présence de la protéine tend à démontrer que la DPE conserve ses propriétés élastiques intrinsèques au sein de l'hydrogel et les transmet à l'hydrogel.

**Hydrogels Poreux.**

Par une approche de porogène, des pores de tailles variables peuvent être incorporés au sein des hydrogels. Des billes de paraffine fractionnées par tamisages en populations de diamètres différents permettent de fournir un échafaudage autour duquel l'hydrogel est réticulé. La granulométrie contrôlée des billes de paraffine, notamment de 250 à 180 microns, de 180 à 100 microns ou de 100 à 50 microns a permis ainsi de moduler la taille des pores formés après extraction de la paraffine (figure 8). Les pores interconnectés, de taille contrôlée entre 50 et 180 microns, permettent l'infiltration cellulaire. En faisant varier le rapport des diamètres de pores, les cellules peuvent atteindre une profondeur de 1250 microns, avec 80% des cellules envahissant 350 microns en moyenne, dans les 14 jours.

**EXEMPLE 4 : CYTOCOMPATIBILITE IN VITRO DES HYDROGELS**

**Matériels et méthodes**

La cytotoxicité des hydrogels a été évaluée par coloration fluorescente live/dead en cultivant des fibroblastes de prépuces humains sur des disques d'hydrogels denses de 8 mm de diamètre et d'environ 0,5 mm d'épaisseur. La morphologie et la prolifération cellulaire ont été observées par un marquage utilisant la phalloïdine fluorescente. Avant culture cellulaire, les hydrogels sont aseptisés en bain d'éthanol 70% durant 12 heures avant d'être rincés trois fois 10 minutes dans du PBS. Les cellules sont ensemencées à une densité de 5000 cellules par hydrogel et cultivées en DMEM-F12 (10% sérum de veau fœtal ; pénicilline 100U/ml ; streptomycine 100µg/ml) durant 8 jours à 37 °C dans une atmosphère contenant 5% de $CO_2$. Après 1, 5 et 8 jours, un marquage par la phalloïdine est réalisé afin de visualiser le cytosquelette d'actine, tandis qu'un marquage live/dead est réalisé après 5 jours afin de mettre en évidence les cellules mortes (en rouge) et les cellules vivantes (en vert).

La capacité des cellules à envahir et coloniser les hydrogels poreux a été évaluée après culture de fibroblastes de prépuces humains déposés à la surface de disques d'hydrogels poreux (6 mm de diamètre, 2-3 mm d'épaisseur) préalablement aseptisés. Une quantité de 100000 cellules, dans un volume de 10 μl, est ensemencée à la surface des disques d'hydrogels. Après 2 heures d'adhésion cellulaire à 37 °C, les hydrogels sont recouverts de milieu de culture (DMEM-F12 ; 10% sérum de veau fœtal ; pénicilline 100U/ml ; streptomycine 100μg/ml) et cultivés durant 14 jours en duplicats.

Après 1, 7 et 14 jours, les disques sont fixés en para-formaldéhyde (PFA, 4%) et des coupes fines de 8 μm sont réalisées par cryotomie. Les noyaux cellulaires sont alors marqués par DAPI et observés en microscopie de fluorescence. La pénétration cellulaire est quantifiée par analyse d'image en utilisant image J et exprimée en distance par rapport à la surface d'ensemencement cellulaire. 9 images de coupes sont analysées pour chaque hydrogel en duplicat, soit 18 images par composition d'hydrogel. Les résultats des analyses d'images sont comparés statistiquement par tests non paramétriques (kruskal-Wallis suivi du test *post-hoc* de Nemenyu).

**Résultats**

**Cyto-compatibilité des hydrogels**

Afin de déterminer la capacité des hydrogels à permettre l'adhésion et la prolifération cellulaire, des fibroblastes humains ont été ensemencés sur des gels denses de composition DGL/PEG-NHS 50/50 mg/ml, contenant ou non de la PDE (5,625 mg/ml). L'observation de la morphologie et de la prolifération de fibroblastes humains à la surface d'hydrogels contenant ou non de la PDE, à 1, 5 8 ou 12 jours montrent qu'après une journée, les cellules adhèrent à la surface des gels et environ la moitié acquiert un phénotype fusiforme caractéristique des cellules fibroblastiques. Les cellules prolifèrent à la surface des hydrogels et atteignent la confluence après 8 jours de culture. La présence de la PDE n'induit pas de différence notable sur le comportement cellulaire. L'adéquation et l'innocuité des hydrogels pour la culture cellulaire sont également indiquées par la faible mortalité observée après 5 jours de culture sur les hydrogels par test live/dead, dans lesquels la cytotoxicité des hydrogels est évaluée, les cellules vivantes étant indiquées en vert et les cellules mortes en rouge.

**Hydrogels poreux comme substrat cellulaire**

La formation de porosité au sein des hydrogels permet de laisser pénétrer les cellules afin d'assurer une colonisation du matériau et une formation tissulaire efficace. Pour valider cette hypothèse, des fibroblastes humains ont été ensemencés à la surface d'hydrogels poreux de même composition (DGL/PEG-NHS 50/50 mg/ml) mais de porosités différentes, et cultivés durant 14 jours. La variation de porosité peut être obtenue en mélangeant des billes de paraffine de granulométrie 180-100μm et 100-50μm à différents ratios massiques. Au cours de la culture, les cellules pénètrent dans les hydrogels poreux, comme visualisé par coupes et marquages DAPI des noyaux cellulaires, confirmant l'interconnexion des pores. Cette interconnexion peut être reliée à l'empilement préalable des particules de paraffines par centrifugation avant association avec l'hydrogel, qui permet d'assurer le contact entre les billes et leur compaction. Après 14 jours de culture les différentes porosités montrent toutes une invasion cellulaire importante pouvant atteindre 1250 μm de profondeur, comme indiqué sur des histogrammes de pénétration cellulaire obtenus par analyse de l'image et analyse statistique des pénétrations maximales atteintes par les cellules dans les hydrogels de différentes porosités. Une analyse statistique des histogrammes de pénétration cellulaire indique que si après 1 jour les cellules ont pénétré plus profondément dans les hydrogels de porosité 180-100μm et de mélange 25/75% 100-50/180-100μm, elles atteignent une profondeur maximale statistiquement plus importante pour les ratios massiques 50/50% 100-50/180-100μm et 75/25% 100-50/180-100μm ($p<0.05$).

**EXEMPLE 5 : UTILISATION DES HYDROGELS POREUX POUR LA CICATRISATION CUTANEE**

**Matériels et méthodes**

La possibilité pour les hydrogels poreux d'intégrer une plaie cutanée et de modifier le processus de cicatrisation a été évalué dans des modèles de plaies chez la souris. Deux défauts cutanés circulaires de 8 mm sont réalisés sur le dos de souris C57BL6 à l'aide d'un punch à biopsie pour ne prélever que la partie cutanée sans causer de dommage musculaire. Un anneau de silicone de diamètre interne 8 mm et externe 16 mm est collé et suturé aux abords de la plaie avant de la combler par des disques d'hydrogels (8 mm de diamètre) de composition DGL/PEG-NHS 50/50 mg/ml contenant 0.875 mg/ml de PDE, de porosité 180-100μm et d'épaisseurs 1 et 4 mm. La plaie comblée/vide et l'anneau de silicone sont ensuite recouverts par un film optiskin durant 7 jours. Une observation quotidienne des plaies est effectuée et la fermeture de la plaie photographiée. Après 14 jours, les souris sont euthanasiées, les plaies cicatrisées sont explantées, fixées en PFA 4%, incluses en paraffine, sectionnées en coupes de 8 μm et colorées par hématoxyline-éosine-safran.

**Résultats**

**Biocompatibilité des hydrogels in vivo**

L'implantation sous-cutanée des hydrogels chez la souris durant trois semaines a permis de montrer la biocompatibilité des hydrogels denses et poreux, comme observé après coloration au trichome de Masson qui indique le collagène en bleu turquoise. Une capsule fibreuse, caractéristique de la réaction classique aux corps étrangers, est présente autour de tous les hydrogels implantés. Contrairement aux hydrogels denses, les hydrogels poreux présentent une importante invasion cellulaire centripète qui confirme l'interconnexion des pores, de diamètres 180-100 μm et 100-50 μm. La majorité des cellules envahissant les pores vides sont d'un morphotype macrophagique. Leur activité de phagocytose peut être observée sur l'hydrogel, que ce soit au niveau des membranes d'hydrogel entre les pores ou en périphérie des hydrogels denses. Au-delà des macrophages, des cellules fibroblastiques sont également présentes dans les hydrogels poreux et une déposition de matrice collagénique est constatée dans les pores, jusqu'à une profondeur importante (1 mm). Par ailleurs, de nombreux vaisseaux sanguins ont envahi les hydrogels poreux, permettant d'expliquer la présence importante de macrophages et la survie cellulaire à des profondeurs importantes dans les hydrogels. La présence de la PDE (3,75 mg/ml) dans les hydrogels denses ou poreux ne semble pas induire de différence cellulaire, vasculaire ou d'inflammation notable.

Dans toutes les conditions testées (hydrogel dense, poreux, avec ou sans présence de PDE), il est important de constater que la réponse inflammatoire induite par les hydrogels est absente ou très faible. En effet, aucun granulocyte ou lymphocyte n'ont été détectés.

Conclusion : L'implantation sous-cutanée d'un biomatériau selon l'invention pendant trois semaines met en évidence une invasion cellulaire importante et la biorésorption du matériau, l'inflammation et la réponse fibreuse péri-implantation apparaissent faibles.

**Utilisation des hydrogels poreux pour la reconstruction cutanée**

La forte colonisation cellulaire observée *in vitro* et *in vivo* laisse envisager l'adéquation des hydrogels poreux pour la culture de cellules en trois dimensions et pour leur utilisation en tant que support de formation de tissus complexes. Pour évaluer cette possibilité, des peaux reconstruites ont été réalisées en cultivant successivement des fibroblastes et des kératinocytes humains respectivement au sein et en surface d'hydrogels poreux de différentes porosités.

Comme indiqué par la figure 9 les fibroblastes ont colonisé des hydrogels, que ce soit pour une porosité de 75% 100-50 μm/25% 180-100 μm, ou une porosité de 180-100μm. Les kératinocytes déposés en surface des gels, après prolifération des fibroblastes, forment une structure pluristratifiée caractéristique des épithélia. La couche la plus profonde est formée par des cellules cohésives présentant un noyau en position basale. En se dirigeant vers la surface, le diamètre des cellules diminue et les noyaux tendent à s'aplatir et disparaître dans la couche la plus externe assimilable à une couche cornée. Dans son ensemble, cette structure est proche de la structure épidermique naturelle de la peau. Il est toutefois à noter que les hydrogels de porosité 180-100μm permettent à quelques kératinocytes de pénétrer dans les pores proches de la surface des hydrogels empêchant la formation d'une couche basale homogène. Notre hypothèse est que dans ces conditions, la lame basale sur laquelle sont en principe assis les kératinocytes prolifératifs, est discontinue. Une modification du protocole d'ensemencement des fibroblastes et des kératinocytes pourrait permettre d'obtenir une colonisation plus homogène *ab initio* et des structures épidermiques plus marquées à terme.

**Intégration des hydrogels à la plaie cutanée et utilité potentielle.**

La biocompatibilité, la capacité de vascularisation, de colonisation cellulaire et de support au dépôt de MEC, ainsi que la nature élastique versatile des hydrogels en font des candidats intéressants pour s'intégrer à différents types de plaies et pour guider la cicatrisation. Pour évaluer ce potentiel, des hydrogels (DGL/PEG-NHS 50/50 mg/ml, contenant 0,875 mg/ml de PDE) poreux (180-100 μm) ont été déposés au sein de plaies cutanées réalisées chez la souris. Après deux semaine de cicatrisation les hydrogels d'épaisseur 1 et 4 mm sont présents dans le tissu cicatrisé (figure 10). Ils sont fragmentés et intégralement cellularisés. En comparaison de la plaie laissée vide, les hydrogels semblent améliorer le tissu cicatriciel, notamment par la présence d'annexes cutanées et de poils dans le derme, dont certains sont en contact direct avec des fragments d'hydrogel. Il n'y a pas de différences notables au niveau de l'épiderme entre plaies vides ou comblées par les hydrogels, qui montrent toutes un épaississement.

**EXEMPLE 6 : COMPLEXATION DANS DES FIBRES PRE-ELASTIQUES DU POLYPEPTIDE SELON L'INVENTION ET DE POLYPEPTIDES MUTES.**

**Matériels et méthodes**

Pour la culture cellulaire, des fibroblastes dermiques normaux humains (NHDF) isolés à partir de prépuce juvénile (Promocell, Heidelberg, Allemagne) ont été cultivés à 37°C, 5% de CO2 dans un milieu complet: DMEM/F-12 ratio 1:1 (Invitrogen, Cergy Pontoise, France) complété avec 10% de sérum de veau foetal (SVF) (Sigma-Aldrich), 100 ug / ml de pénicilline et 0,1 mg / ml de streptomycine (Sigma-Aldrich). Les cellules ont été ensemencées à une densité de 8 000 cellules.$cm^{-2}$ à la surface de lamelles en verre rondes de 12 mm de diamètre dans des puits de plaques 24 puits. Le milieu de culture est remplacé tous les deux jours jusqu'à 8 jours post-confluence. Aux temps indiqués, le polypeptide sauvage (Elactiv') ou ses formes mutées ont été ajoutés dans le milieu de culture à la concentration de 1 μg / ml pendant 3 jours. L'inhibiteur de lysyls oxydases, le β-aminopropionitrile, a été ajouté simultanément au polypeptide à la concentration de 350 μM.

Les mutations du gène codant pour le polypeptide ont été réalisées à l'aide du kit de mutagenèse dirigée QuickChange II (Agilent Technologies, Les Ulis) selon les recommandations du fournisseur à partir du plasmide d'expression comportant le gène sauvage (SEQ ID N°12). La mutation sur la séquence codant le motif RKRK du domaine D, a été obtenue en utilisant les amorces suivantes :

- sens : 5'-aagcctgtggccgtaaacCTaGgtaagtcgacaagcttgc (SEQ ID N°15);
- antisens 5'- gcaagcttgtcgacttacCtAGgtttacggccacaggctt (SEQ ID N°16).

La mutation sur le codon correspondant à la première cystéine du domaine D, a été obtenue en utilisant les amorces suivantes :

- sens 5'- gcgtttacggccaccggctttacccaggc (SEQ ID N°17);
- antisens 5'- gcctgggtaaagccggtggccgtaaacgc (SEQ ID N°18).

Les productions et purifications des protéines mutantes ont été réalisées de façon similaire à la protéine sauvage.

Les marquages fluorescents de chacun des polypeptides ont été réalisés par conjugaison du tetraméthylrhodamine-5-maléimide (Sigma-Aldrich). Les polypeptides et l'agent fluorescent ont été mélangés dans un tampon phosphate salin (PBS) aux concentrations de 2 mg / ml et 10 mg / ml respectivement. Après une incubation d'une nuit à température ambiante à l'abri de la lumière, les polypeptides marqués ont été purifiés par ITC pour éliminer l'excédent d'agent fluorescent.

A la fin de la culture, un marquage immunofluorescent de la tropoélastine et des noyaux a été réalisé. Le milieu de culture a été aspiré et les puits ont été rincés trois fois avec du PBS avant d'être fixées au méthanol pur pendant 20 min à -20°C. Après trois nouveaux rinçages au PBS, un traitement à l'albumine sérique bovine (BSA) 5% en PBS (p/v) pendant 1h à température ambiante a permis de saturer les sites aspécifiques. L'anticorps primaire anti-a élastine (polyclonal, lapin, ab 21607, Abcam) dilué au 1/200ème dans du PBS/BSA à 0,5% (p/v) a été incubés 1h à température ambiante. Après trois rinçages de 5 min au PBS, l'anticorps secondaire anti-lapin couplé à l'Alexa fluor 488 (Invitrogen ® A11008) dilué au 1/600ème dans du PBS/BSA 0,5% (p/v) a été incubé 1h à température ambiante et à l'obscurité. Enfin, les noyaux ont été contremarqués avec du 4,6-Diamidino-2-phenylindole (DAPI) à 2 μg / ml dans du PBS pendant 10 min à température ambiante. Après cinq rinçages au PBS, les lamelles ont été montées en milieu aqueux avec une solution de Permafluor (LabVision, Thermo-scientifique). Les images ont été acquises à l'aide des microscopes à fluorescence Nikon TE300 ou Nikon TiE.

**Démonstration du rôle de la lysyl oxydase dans la complexation**

Le polypeptide élastique selon l'invention (polypeptide sauvage) couplé à un composé fluorescent (rhodamine) a été ajouté au milieu de culture des fibroblastes dermiques humains post-confluents (5 jours) à la concentration de 1 μg/ml pour évaluer son intégration potentielle dans les dépôts de fibres élastiques (fig. 11A). Après 3 jours, les cultures ont été arrêtées, les cellules fixées et une observation en microscopie à fluorescence a été réalisée. Dans ces conditions, le polypeptide est colocalisé avec des dépôts fibreux de tropoélastine (flèche blanche, fig. 11B). Ceci n'est pas observé lorsque les enzymes lysyl oxydases sont préalablement inhibées par le β-aminopropionitrile (350 μM) (fig. 11C), démontrant que l'activité de la lysyl oxydase est nécessaire pour que le polypeptide soit complexé avec les fibres élastiques préexistantes. Ces résultats démontrent que le polypeptide est reconnu comme un substrat par les lysyls oxydases Ce résultat n'avait jamais été démontré pour aucun autre polypeptide élastique.

**Complexation de polypeptides mutés dans le domaine C-terminal**

L'hypothèse est que la capacité unique du polypeptide selon l'invention d'être intégré aux fibres élastiques préexistantes est rendue possible par la présence du domaine D (GGACLGKACGRKRK) en position C-terminale, correspondant au domaine 36 de la tropoélastine humaine. Pour la tropoélastine humaine, il a été démontré que deux motifs particuliers de ce domaine favorisent l'incorporation et l'assemblage au sein des fibres élastiques : le motif RKRK et la formation d'un pont disulfure entre les deux cystéines séparées par quatre acides aminés (Nonaka R. *et al,* 2014).

Afin de vérifier la fonctionnalité de ces motifs dans le polypeptide selon l'invention, deux mutants du polypeptide sauvage ont été réalisés. Le premier mutant, dont la séquence est la séquence SEQ ID N°13, porte sur les deux derniers acides aminés du polypeptide, résultant en la séquence RKPR. Le deuxième mutant, dont la séquence est la séquence SEQ ID N°14, cible la première cystéine du domaine D, résultant en une glycine et empêchant la formation du pont disulfure. Les deux polypeptides mutants ont été produits selon le protocole décrit dans l'exemple 1. Le polypeptide sauvage et les deux mutants couplés à la rhodamine ont été ajoutés dans le milieu de culture de fibroblastes dermiques humains (fig. 12A), selon le protocole décrit précédemment dans l'exemple 6.

Le polypeptide sauvage est intégré dans de larges zones des fibres élastiques préexistantes ou en formation (fig. 12A). Cette intégration est très perturbée lorsque le motif RKRK est muté en RKPR (fig. 12B). Cette version mutée du polypeptide ne permet d'obtenir que de petits agrégats épars le long de quelques fibres élastiques (flèche blanche). En revanche, la mutation d'une cystéine empêchant la formation du pont disulfure ne semble pas affecter l'intégration du polypeptide dans les fibres élastiques (flèche blanche, fig. 12C).

Ces résultats démontrent la nécessité de la présence du domaine D dans le polypeptide selon l'invention pour qu'il soit reconnu et intégré dans un réseau de fibres élastiques formé par des fibroblastes dermiques humains. Plus particulièrement, le motif « RKRK » doit être intègre pour que cet assemblage soit effectif. Le pont disulfure n'apparaît pas requis dans ce processus particulier.

**EXEMPLE 7 : CREATION DE COMPOSITES HYDROGEL/NANOFIBRES POUR SUBSTITUTS VASCULAIRES.**

**Matériels et méthodes**

La facilité de mise en forme des hydrogels permet d'aisément les associer avec d'autres matériaux, notamment pour la formation de tubes composites hydrogels/nanofibres destinés à créer des substituts vasculaires.

Les nanofibres sont produites par la technique de jet-spraying (Sohier *et al,* 2014). Brièvement, cette méthode consiste à diffracter des solutions de polymères par un flux d'air pour former des nanofibres. Une solution de polycaprolactone (PCL, masse moléculaire 80 000 g/mol) dans du chloroforme est placée dans un réservoir connecté à un appareil de pulvérisation par un tube. L'appareil de pulvérisation consiste en un cône contenant une aiguille de position ajustable afin de contrôler l'ouverture du cône. Le bout du cône est positionné en face d'un tube distribuant un flux d'air comprimé à différentes pressions. En créant une dépression, le flux d'air conduit la solution de polymère du réservoir à la pointe du cône par l'aiguille, où il est diffracté et projeté sur une cible qui collecte des fibres solides dues à l'évaporation du chloroforme pendant le transit.

Afin de former des structures tubulaires en nanofibres de différents diamètres et épaisseurs, le polymère est pulvérisé sur un tube en verre préalablement trempé dans une solution de dichlorométhyle silane, de diamètre défini et variable, en rotation à une vitesse définie comprise entre 100 et 1000 rpm. L'épaisseur est contrôlée par la durée de pulvérisation. Après création d'une couche de nanofibres, les tubes de verre recouverts sont placés dans une solution de 70% d'éthanol puis rincés avec du tampon phosphate salin (PBS, 137 mM NaCl, 2.7 mM KCl, 10 mM $Na_2HPO_4$, 2 mM $KH2PO_4$, pH 7.4.), afin de rendre le polymère hydrophile.

L'hydrogel est préparé comme défini dans l'exemple 3, avec la composition suivante : DGL 50 mg/ml, PEG-NHS 50 mg/ml, PDE 3,75 mg/ml.

Afin de former les composites, les tubes en verre sur lesquels sont déposées les nanofibres sont placés horizontalement à l'intérieur d'un tube en verre de diamètre défini et variable, préalablement trempé dans une solution de dichlorométhyle silane. Le tube de verre interne est centré et la composition d'hydrogel est injectée à l'aide d'une pipette entre les parois des deux tubes de verre. L'hydrogel remplit l'espace disponible et pénètre la structure nanofibrillaire avant de réticuler et ainsi former un composite de diamètre interne contrôlé par le tube de verre ayant collecté les fibres; et de diamètre externe contrôlé par le second tube de verre (fig.13A et 13B).

Les tubes composites sont retirés en incubant les tubes de verres dans de l'éthanol pur, permettant de retirer le tube de verre interne, qui est ensuite placé dans de l'eau pure afin de retirer le composite tubulaire hydrogel/nanofibres.

**Résultats**

Différents composites tubulaires hydrogel/nanofibres ont pu être créés par la création de tubes en nanofibres polymère, suivie de l'enrobage et la pénétration de l'hydrogel au sein de la matrice nanofibrillaire avant sa réticulation. L'application de l'hydrogel entre deux tubes de verre a permis de former des tubes composites de lumière interne 1 et 2 mm et de diamètre externe de 2 et 3 mm (fig. 14).

Les tubes nanofibrillaires apparaissent enchâssés dans l'hydrogel (fig. 14). Leur diamètre peut être varié lors de leur production initiale par jet-spraying et ces tubes peuvent être intégrés dans des tubes hydrogel de lumière interne inférieure, résultant en un positionnement de la couche de nanofibres entre deux couches d'hydrogel.

Les mesures réalisées à postériori des diamètres des tubes composites sont en adéquation avec celles attendues des diamètres des tubes de verre utilisés pour la fabrication (fig. 15).

Macroscopiquement, les tubes obtenus ne se délaminent pas et montrent une plus grande résistance à l'élongation et à la rupture que des tubes similaires composés uniquement de l'hydrogel.

**REFERENCES BIBLIOGRAPHIQUES**

- Annabi N. et al (2013) Elastomeric recombinant protein-based biomaterials. Biochem. Eng. J. 77: p.110-118.
- Denkewalter R. G., et al (1981) Macromolecular highly branched homogeneous compound based on lysine units, in USP. 1981, Allied Corp.: US.
- Denkewalter R.G. et al (1983) Macromolecular highly branched homogeneous compound, USP. 1983, Allied Corp. : US.
- Jeon W.B. et al (2011) Stimulation of fibroblasts and neuroblasts on a biomimetic extracellular matrix consisting of tandem repeats of the elastic VGVPG domain and RGD motif. J. Biomed. Mater. Res. A. 97(2): p.152-157.
- Klok H.A. et al (2002) Dendritic graft polypeptides. Macromolecules, 35, p.8718-8723.
- Nonaka R. et al (2014) Domain 36 of tropoelastin in elastic fiber formation. Biol Pharm Bull 37 (2014) 698-702).
- Rodriguez-Hernandez J., et al (2003) Highly Branched Poly (L-lysine), Biomacromolecules, 4: p. 249-258.
- Pailler-Mattei et al (2013) In vivo skin biophysical behaviour and surface topography as a function of ageing, J. Mech. Behav. Biomed. Mater., Dec., 28, 474-83.
- Rossi J.C. et al (2012) Functionalisation of free amino groups of lysine dendrigraft (DGL) polymers, Tetrahedron Letters, 53, p: 2976-2979.
- Sohier J. et al (2014) novel and simple alternative to create nanofibrillar matrices of interest for tissue engineering. Tissue Engineering Part C-Methods, 2014. 20(4): p. 285-29
- Wise S. G. et al (2014) Tropoelastin: a versatile, bioactive assembly module. Acta Biomater, 10, (4), 1532-41.
- Yeo G.C. et al (2011) Coacervation of tropoelastin. Adv Colloid Interface Sci. 167(1-2): p. 94-103.
- Yeo G.C. et al (2015) Fabricated Elastin. Advanced healthcare materials, 4(16): p. 2530-2556.


## Revendications

1. Polypeptide isolé dérivé de la tropoélastine, dont la séquence en acides aminés comprend successivement, de l'extrémité N-terminale à l'extrémité C-terminale dudit polypeptide :

   • Un domaine consistant en au moins 2 et au plus 40 répétitions d'une unité consistant :

      • Une région hydrophobe (A) consistant en successivement au moins 6 et au plus 20 séquences
      $X_1GX_2X_3PG$ (SEQ ID N°1)
      dans lesquelles $X_1$, $X_2$ et $X_3$ représentent un acide aminé hydrophobe indépendamment choisi dans le groupe consistant en : Alanine, Valine, Leucine et Isoleucine ; lesdites SEQ ID N°1 étant identiques ou différentes, et
      • Une région (B) comprenant au moins la séquence
      AAAKAAK (SEQ ID N°2),

   • Un domaine hydrophobe (C) consistant en successivement au moins 6 et au plus 20 séquences
   $X_4GX_5X_6PG$ (SEQ ID N°4)
   dans laquelle $X_4$, $X_5$ et $X_6$ représentent un acide aminé hydrophobe indépendamment choisi dans le groupe consistant en : Alanine, Valine, Leucine et Isoleucine ; lesdites SEQ ID N°4 étant identiques ou différentes, et
   • Un domaine (D) comprenant au moins deux acides aminés cystéine non consécutifs et la séquence
   RKRK (SEQ ID N°5).

2. Polypeptide selon la revendication 1, **caractérisé en ce que** la région (B) comprend la séquence AAAKAAAKAAK (SEQ ID N°3) et **en ce que** le domaine (D) comprend la séquence GGACLGKACGRKRK (SEQ ID N°6).

3. Polypeptide selon l'une des revendications 1 à 2, comprenant la séquence en acides aminés SEQ ID N°8.

4. Matériau biocompatible comprenant un polypeptide selon l'une quelconque des revendications 1 à 3.

5. Matériau biocompatible selon la revendication 4, **caractérisé en ce qu'**il comprend en outre un polymère hydrophile.

6. Matériau biocompatible selon la revendication 5 **caractérisé en ce que** :

   - ledit polypeptide comprend la séquence en acides aminés SEQ ID N°8,
   - ledit polymère est un dendrimère greffé de polylysine de génération 3, et
   - ledit matériau biocompatible comprend un agent réticulant bifonctionnel comprenant une chaîne de polyéthylène glycol (PEG).

7. Biomatériau composite comprenant un matériau biocompatible selon l'une des revendications 5 ou 6, et un composé constitué par un matériau synthétique, par un matériau d'origine naturelle, ou par un mélange d'un matériau synthétique et d'un matériau d'origine naturelle.

8. Procédé de préparation d'un matériau biocompatible selon l'une des revendications 4 à 6, comprenant une étape de mélange d'un polypeptide selon l'une des revendications 1 à 3 et d'un agent réticulant.

9. Procédé selon la revendication 8, dans lequel ledit agent réticulant est un agent bifonctionnel et dans lequel ladite étape de mélange du polypeptide et de l'agent réticulant est réalisée en présence d'un polymère hydrophile.

10. Procédé selon l'une des revendications 8 ou 9 comprenant une étape de coulage dudit matériau biocompatible dans un moule de forme définie, et une étape de génération de pores au sein dudit matériau, les deux étapes pouvant être successives, dans n'importe quel ordre, ou simultanées.

11. Procédé selon l'une des revendications 8 à 10 comprenant en outre une étape de de mise en contact du mélange d'un polypeptide selon l'une des revendications 1 à 2 et d'un agent réticulant, avec un composé constitué par un matériau synthétique, par un matériau d'origine naturelle, ou par un mélange de matériaux synthétiques et de matériaux d'origine naturelle.

**12.** Utilisation d'un matériau biocompatible selon l'une des revendications 4 à 6 pour la préparation d'un biomatériau composite comprenant ledit matériau biocompatible et un autre composé, ledit composé étant constitué par un matériau synthétique, par un matériau d'origine naturelle, ou par un mélange de d'un matériau synthétique et d'un matériau d'origine naturelle.

**13.** Support de culture cellulaire in vitro comprenant un matériau biocompatible comprenant un polypeptide selon l'une quelconque des revendications 1 à 3 et obtenu par un procédé selon la revendication 10.

**14.** Matériau biocompatible selon l'une des revendications 4 à 6, ou tel qu'obtenu par un procédé selon l'une des revendications 8 à 10, biomatériau composite selon la revendication 7 ou tel qu'obtenu selon par un procédé selon la revendication 11, pour son utilisation en thérapie chez l'homme ou chez l'animal.

**Patentansprüche**

**1.** Isoliertes Polypeptid, das von Tropoelastin abgeleitet ist, dessen Aminosäuresequenz aufeinanderfolgend vom N-terminalen Ende zum C-terminalen Ende des besagten Peptids aufweist:

• eine Domäne, die aus wenigstens 2 und höchstens 40 Wiederholungen einer Einheit besteht, bestehend aus:

◦ einen hydrophoben Bereich (A), welcher aufeinanderfolgend aus wenigstens 6 und höchstens 20 Sequenzen
$X_1GX_2X_3PG$ (SEQ ID N°1)
besteht, wobei $X_1$, $X_2$ und $X_3$ eine hydrophobe Aminosäure darstellen, die unabhängig ausgewählt ist aus der Gruppe bestehend aus: Alanin, Valin, Leucin und Isoleucin, wobei besagte SEQ ID N°1 gleich oder unterschiedlich sind, und
◦ einen Bereich (B), welcher wenigstens aufweist die Sequenz
AAAKAAK (SEQ ID N°2),

• eine hydrophobe Domäne (C), welche aufeinanderfolgend aus wenigstens 6 und höchstens 20 Sequenzen
$X_4GX_5X_6PG$ (SEQ ID N°4)
besteht, wobei $X_4$, $X_5$ und $X_6$ eine hydrophobe Aminosäure darstellen, die unabhängig ausgewählt ist aus der Gruppe bestehend aus: Alanin, Valin, Leucin und Isoleucin, wobei besagte SEQ ID N°4 gleich oder verschieden sind, und
• eine Domäne (D), welche wenigstens aufweist zwei nicht aufeinanderfolgende Cystein-Aminosäuren und die Sequenz
RKRK (SEQ ID N°5).

**2.** Polypeptid gemäß Anspruch 1, **dadurch gekennzeichnet, dass** der Bereich (B) die Sequenz AAAKAAAKAAK (SEQ ID N°3) aufweist, und **dadurch gekennzeichnet, dass** der Bereich (D) die Sequenz GGACLGKACGRKRK (SEQ ID N°6) aufweist.

**3.** Polypeptid gemäß einem der Ansprüche 1 bis 2, welches die Aminosäuresequenz SEQ ID N°8 aufweist.

**4.** Biokompatibles Material, welches ein Polypeptid gemäß irgendeinem der Ansprüche 1 bis 3 aufweist.

**5.** Biokompatibles Material gemäß Anspruch 4, **dadurch gekennzeichnet, dass** es weiterhin ein hydrophiles Polymer aufweist.

**6.** Biokompatibles Material gemäß Anspruch 5, **dadurch gekennzeichnet, dass**:

- besagtes Polypeptid die Aminosäuresequenz SEQ ID N°8 aufweist,
- besagtes Polymer ein gepfropftes Polylysin-Dendrimer der Generation 3 ist, und
- besagtes biokompatibles Material ein bifunktionelles Vernetzungsmittel aufweist, welches eine Polyethylenglykol (PEG)-Kette aufweist.

**7.** Biomaterialzusammensetzung, welche aufweist ein biokompatibles Material gemäß einem der Ansprüche 5 oder 6 und eine Verbindung, welche aus einem synthetischen Material, aus einem Material natürlichen Ursprungs oder aus einer Mischung eines synthetischen Materials und eines Materials natürlichen Ursprungs ausgebildet ist.

**8.** Herstellungsverfahren eines biokompatiblen Materials gemäß einem der Ansprüche 4 bis 6, welches einen Schritt des Mischens eines Polypeptids gemäß einem der Ansprüche 1 bis 3 und eines Vernetzungsmittels aufweist.

**9.** Verfahren gemäß Anspruch 8, wobei besagtes Vernetzungsmittel ein bifunktionelles Mittel ist und wobei besagter Schritt des Mischens des Polypeptids und des Vernetzungsmittels in Anwesenheit eines hydrophilen Polymers durchgeführt wird.

**10.** Verfahren gemäß einem der Ansprüche 8 oder 9, welches einen Schritt des Gießens des besagten biokompatiblen Materials in eine Gießform mit bestimmter Form und einen Schritt des Generierens von Poren innerhalb des besagten Materials aufweist, wobei die zwei Schritte nacheinander, in beliebiger Reihenfolge, oder zugleich sein können.

**11.** Verfahren gemäß einem der Ansprüche 8 bis 10, welches weiterhin aufweist einen Schritt des in-Kontakt-Bringens der Mischung eines Polypeptids gemäß einem der Ansprüche 1 bis 2 und eines Vernetzungsmittels mit einer Verbindung, welche aus einem synthetischen Material, aus einem Material natürlichen Ursprungs oder aus einer Mischung von synthetischen Materialien und Materialien natürlichen Ursprungs ausgebildet ist.

**12.** Verwendung eines biokompatiblen Materials gemäß einem der Ansprüche 4 bis 6 zur Herstellung einer Biomaterialzusammensetzung, welche besagtes biokompatibles Material und eine andere Verbindung aufweist, wobei besagte Verbindung aus einem synthetischen Material, aus einem Material natürlichen Ursprungs oder aus einer Mischung eines synthetischen Materials und eines Materials natürlichen Ursprungs ausgebildet ist.

**13.** In-vitro-Zellkulturträger, welcher ein biokompatibles Material aufweist, das ein Polypeptid gemäß irgendeinem der Ansprüche 1 bis 3 aufweist, und durch ein Verfahren gemäß Anspruch 10 erlangt wurde.

**14.** Biokompatibles Material gemäß einem der Ansprüche 4 bis 6, oder so wie erlangt durch ein Verfahren gemäß einem der Ansprüche 8 bis 10, Biomaterialzusammensetzung gemäß Anspruch 7 oder so wie erlangt durch ein Verfahren gemäß Anspruch 11 zur Verwendung bei der Behandlung von Menschen oder Tieren.

## Claims

**1.** Isolated polypeptide derived from tropoelastine, wherein the amino acid sequence comprises, successively, from the N-terminus to the C-terminus of the polypeptide:

- A domain consisting of at least 2 and at most 40 repeats of a unit consisting of:
  - A hydrophobic region (A) consisting of successively at least 6 and at most 20 sequences
    $X_1GX_2X_3PG$ (SEQ ID N°1)
    wherein $X_1$, $X_2$ and $X_3$ represent a hydrophobic amino acid independently selected from the group consisting of: alanine, valine, leucine and isoleucine; wherein the SEQ ID No. 1 is identical or different, and
  - A region (B) comprising at least the sequence
    AAAKAAK (SEQ ID No. 2),
- An hydrophobic domain (C) consisting of successively at least 6 and at most 20 sequences
  $X_4GX_5X_6PG$ (SEQ ID N°4)
  wherein $X_4$, $X_5$ and $X_6$ are any hydrophobic amino acid independently selected from the group consisting of: alanine, valine, leucine and isoleucine; and wherein the SEQ ID No. 4 is identical or different, and
- A domain (D) comprising at least two non-consecutive cysteine amino acids and the sequence
  RKRK (SEQ ID No. 5).

**2.** Isolated polypeptide according to claim 1, wherein the region (B) comprises at least the sequence AAAKAAAKAAK (SEQ ID No. 3), and the domain (D) comprises the sequence GGACLGKACGRKRK (SEQ ID No. 6).

**3.** Polypeptide according to any one of claims 1 to 2, comprising the amino acid sequence SEQ ID No. 8.

4. Biocompatible material comprising a polypeptide according to any one of claims 1 to 3.

5. Biocompatible material according to claim 4, **characterized in that** it further comprises a hydrophilic polymer.

6. Biocompatible material according to claim 5 **characterized in that**:

- the polypeptide comprises the amino acid sequence SEQ ID No. 8,
- the polymer is a grafted dendrimer of polylysine of generation 3, and
- the biocompatible material comprises a bifunctional crosslinking agent comprising a polyethylene glycol (PEG) chain.

7. Composite biomaterial comprising a biocompatible material according to one of the claims 5 or 6, and a compound consisting of a synthetic material, a material of natural origin, or a mixture of a synthetic material and a material of natural origin.

8. Method for preparing a biocompatible material according to one of claims 4 to 6, comprising a step of mixing a polypeptide according to one of the claims 1 to 3 and a crosslinking agent.

9. Method according to claim 8, wherein the crosslinking agent is a bifunctional agent and wherein the step of mixing the polypeptide and the crosslinking agent is carried out in the presence of a hydrophilic polymer.

10. Method according to one of the claims 8 or 9 comprising a step of casting the biocompatible material in a mold of defined shape, and a step of generating pores within the material, wherein the two steps may be successive, in any what order, or simultaneous.

11. Method according to one of the claims 8 to 10 further comprising a step of contacting the mixture of a polypeptide according to one of claims 1 to 2 and a crosslinking agent with a compound consisting of a synthetic material, of a material of natural origin, or a mixture of synthetic materials and materials of natural origin.

12. Use of a biocompatible material according to one of the claims 4 to 6 for the preparation of a composite biomaterial comprising the biocompatible material and another compound, wherein the compound is constituted by a synthetic material, by a material of natural origin, or by a mixture of a synthetic material and a material of natural origin.

13. *In vitro* cell culture support comprising a biocompatible material comprising a polypeptide according to one of claims 1 to 3 and obtained by a method according to claim 10.

14. Biocompatible material according to one of the claims 4 to 6, or as obtained by a method according to one of the claims 8 to 10, composite biomaterial according to claim 7 or as obtained according to a method according to claim 11, for use in therapy in human or in animal.

**FIG 1**

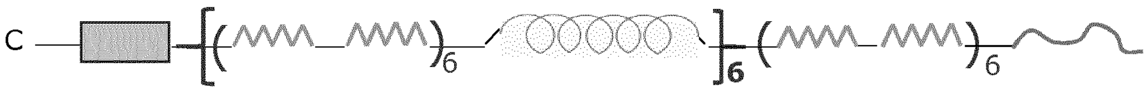


C-DYKDDDDK-{(VGVAPG-VGVLPG)$_6$-AAAKAAAKAAK}$_6$-(VGVAPG-VGVLPG)$_6$-
GGACLGKACGRKRK

**FIG 2**

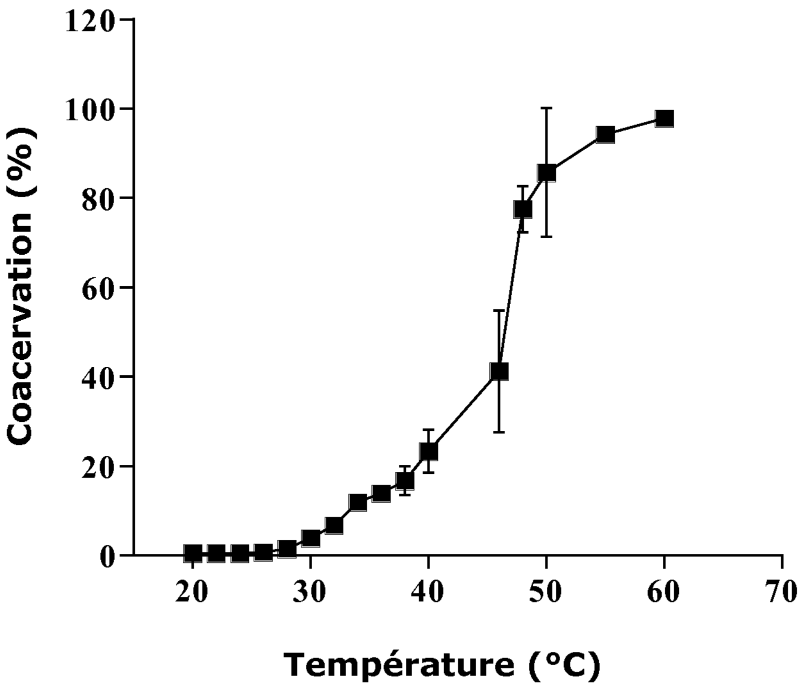

FIG 3 A, 3 B

A

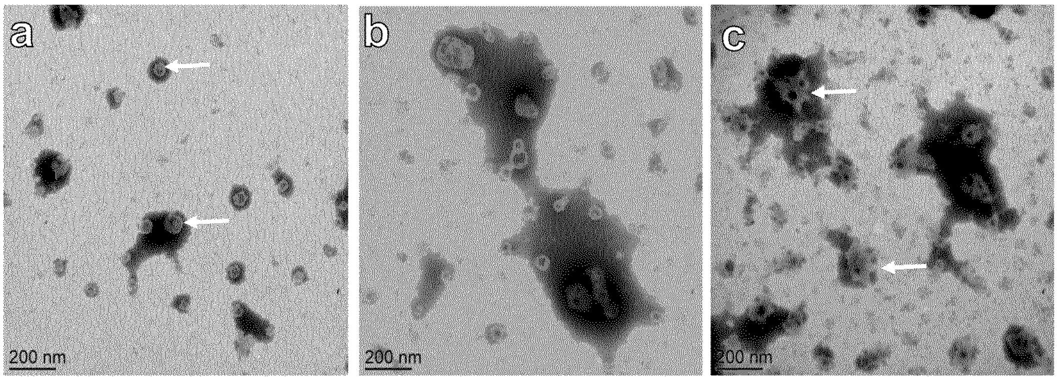


B

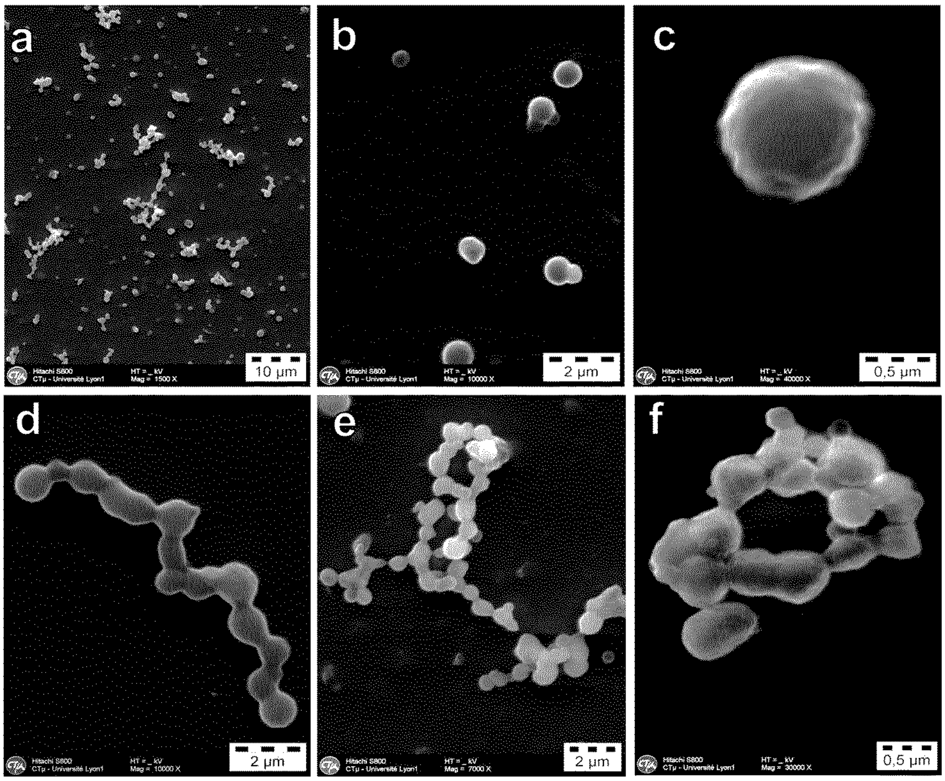

FIG 4 A, 4 B

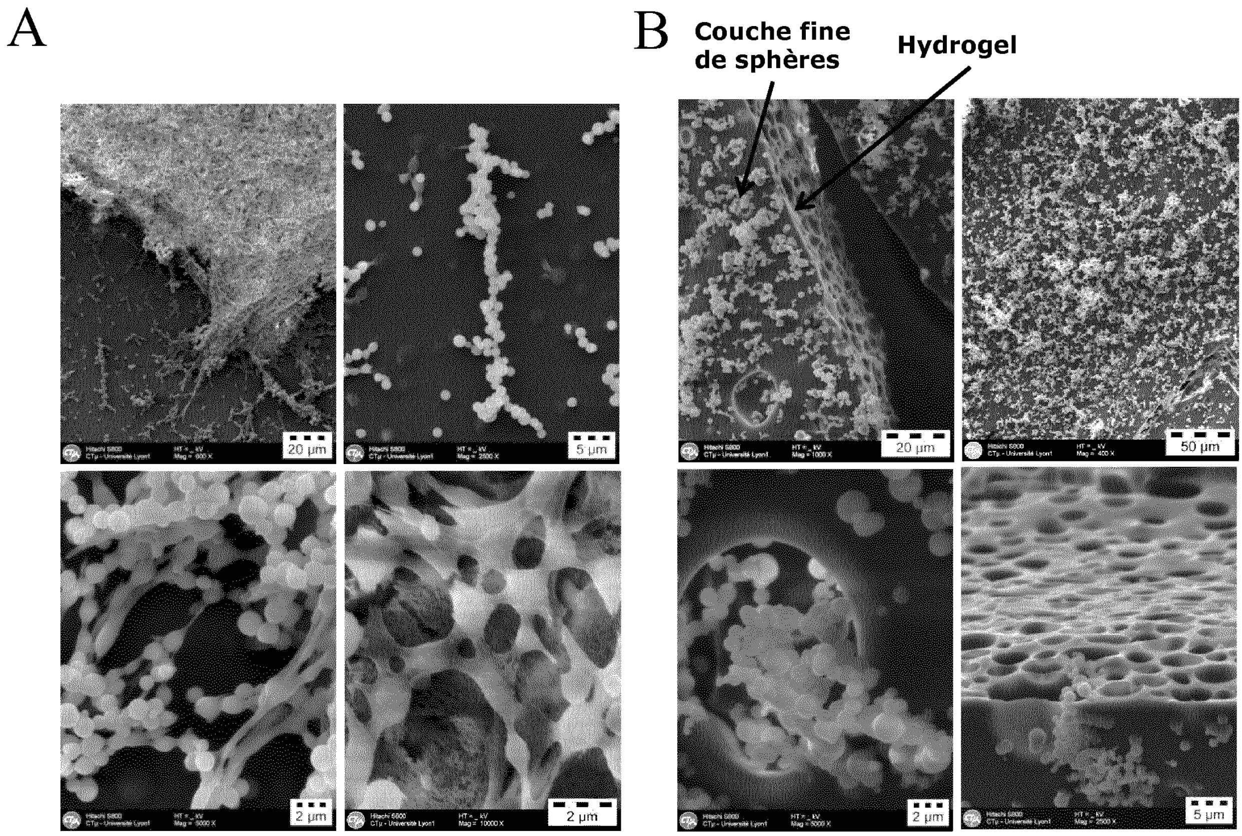
A
B
Couche fine
de sphères
Hydrogel
20 µm
5 µm
2 µm
2 µm
20 µm
50 µm
2 µm
5 µm

**FIG 5 A, 5 B, 5 C**

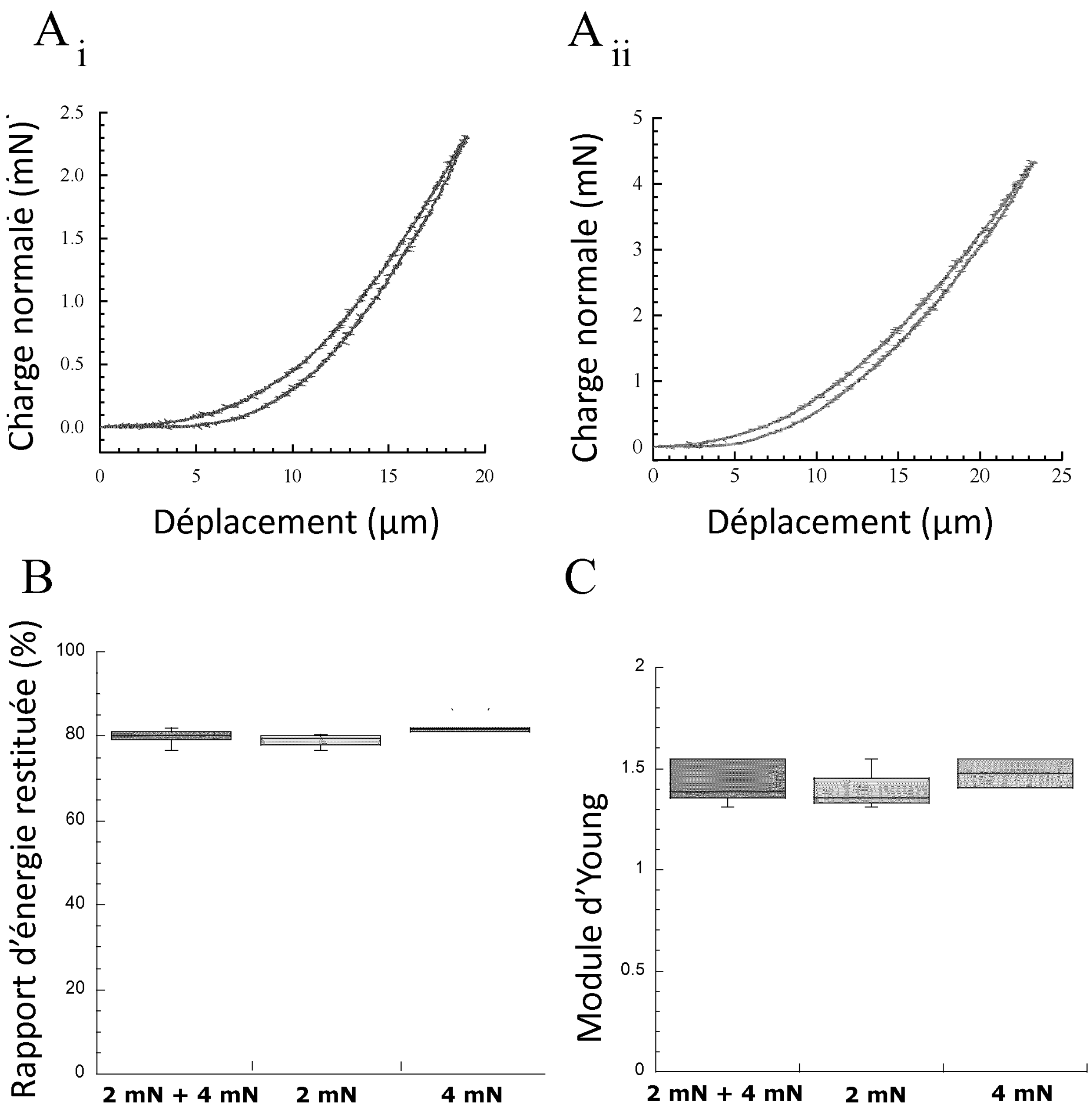
Ai
Charge normale (mN)
2.5
2.0
1.5
1.0
0.5
0.0
0
5
10
15
20
Déplacement (µm)
Aii
Charge normale (mN)
5
4
3
2
1
0
0
5
10
15
20
25
Déplacement (µm)
B
Rapport d'énergie restituée (%)
100
80
60
40
20
0
2 mN + 4 mN
2 mN
4 mN
C
Module d'Young
2
1.5
1
0.5
0
2 mN + 4 mN
2 mN
4 mN

FIG 6

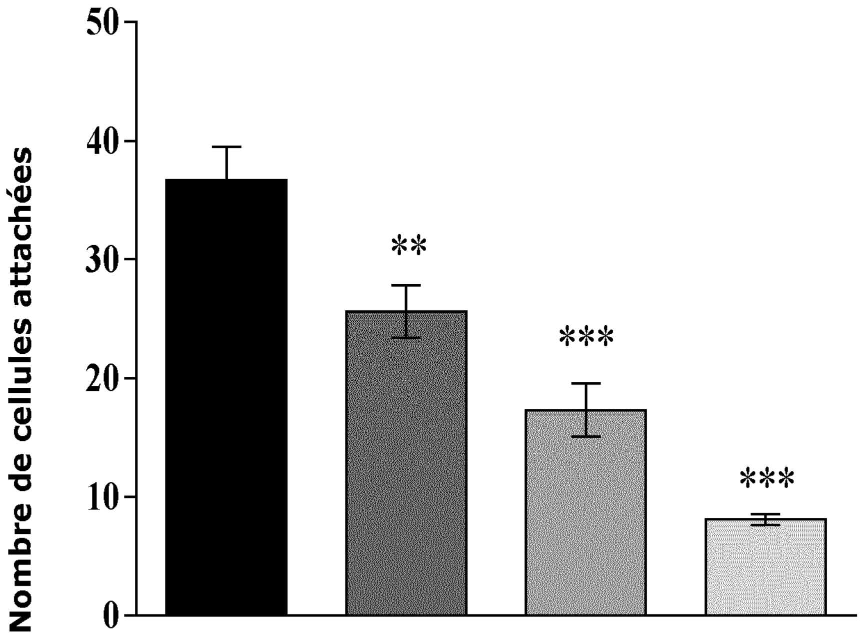


| | | | | |
|---|---|---|---|---|
| **Anti-intégrine αVβ3** *(20µg/ml)* | - | + | - | + |
| **L-lactose** *(3,6mg/ml)* | - | - | + | + |

FIG 7 A, 7 B

A

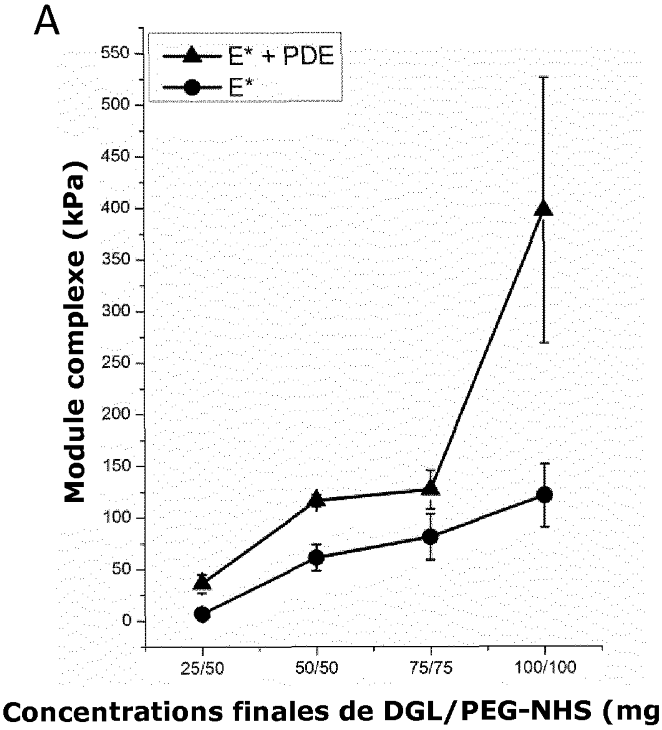
E* + PDE
E*
Module complexe (kPa)
550
500
450
400
350
300
250
200
150
100
50
0
25/50
50/50
75/75
100/100
Concentrations finales de DGL/PEG-NHS (mg/ml)

B

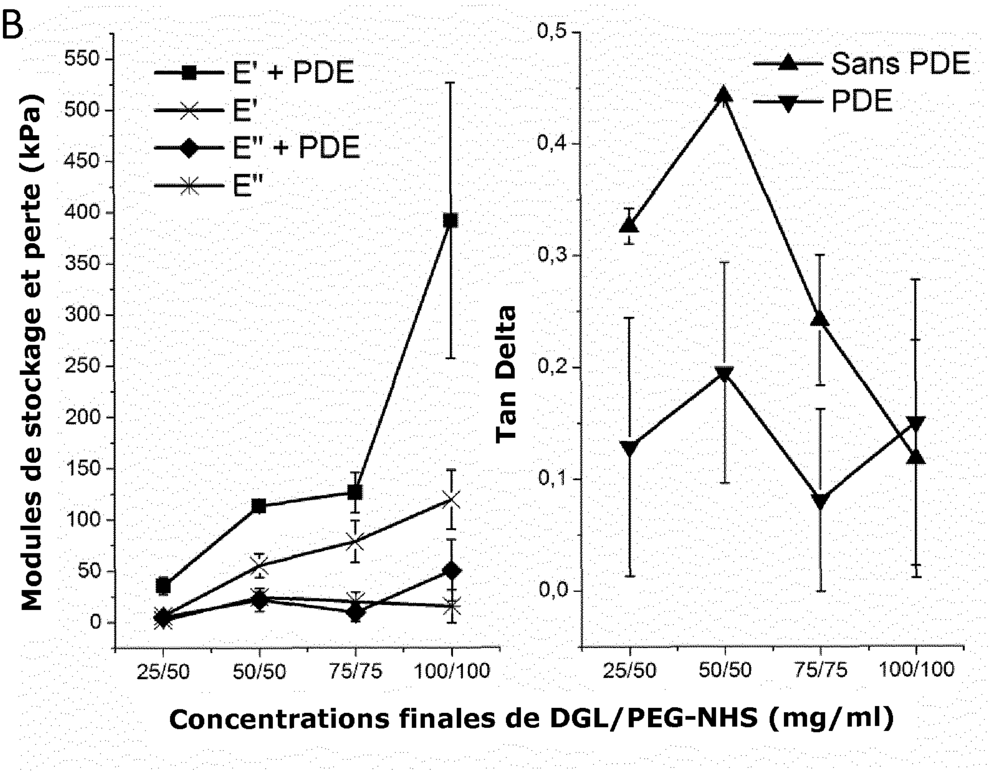
E' + PDE
E'
E" + PDE
E"
Modules de stockage et perte (kPa)
550
500
450
400
350
300
250
200
150
100
50
0
25/50
50/50
75/75
100/100
Sans PDE
PDE
Tan Delta
0,5
0,4
0,3
0,2
0,1
0,0
25/50
50/50
75/75
100/100
Concentrations finales de DGL/PEG-NHS (mg/ml)

FIG 8

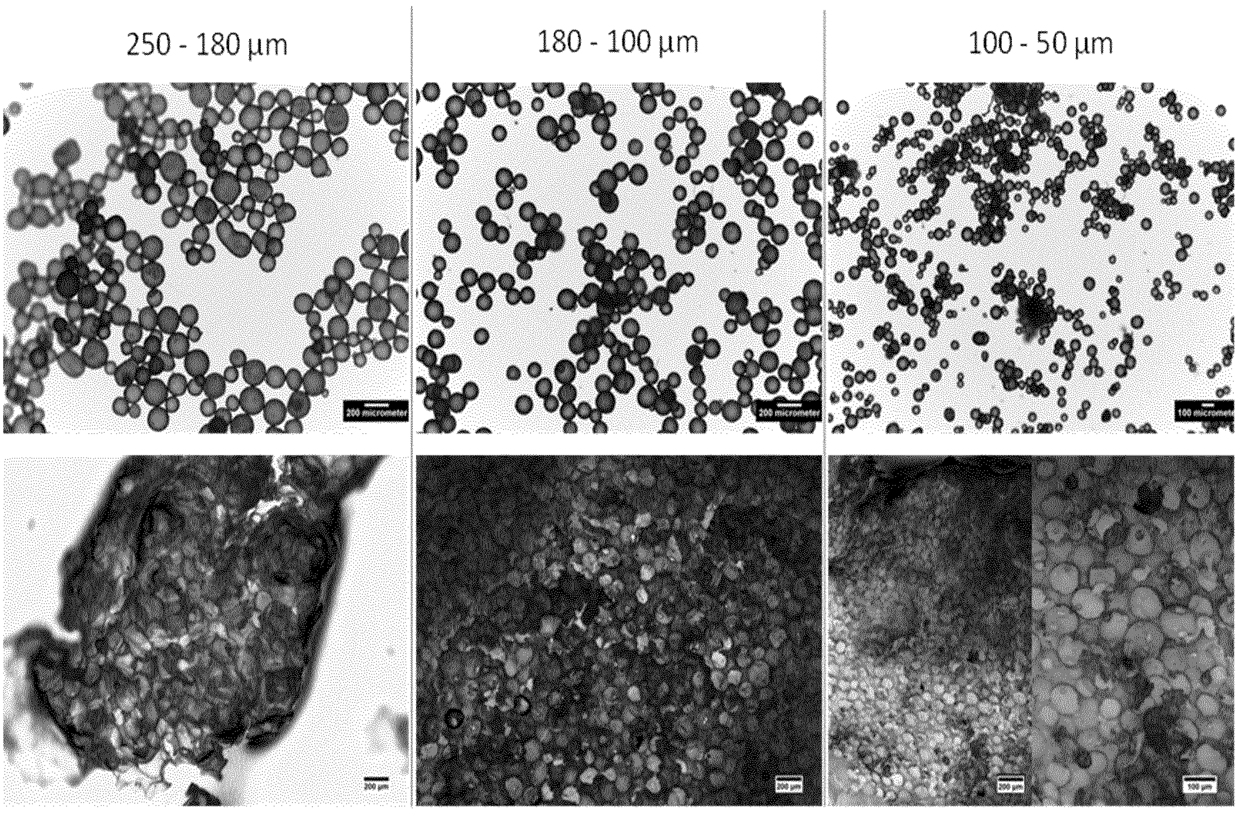
250 - 180 μm
180 - 100 μm
100 - 50 μm
200 micrometer
200 micrometer
100 micrometer
200 μm
200 μm
200 μm
100 μm

**FIG 9**

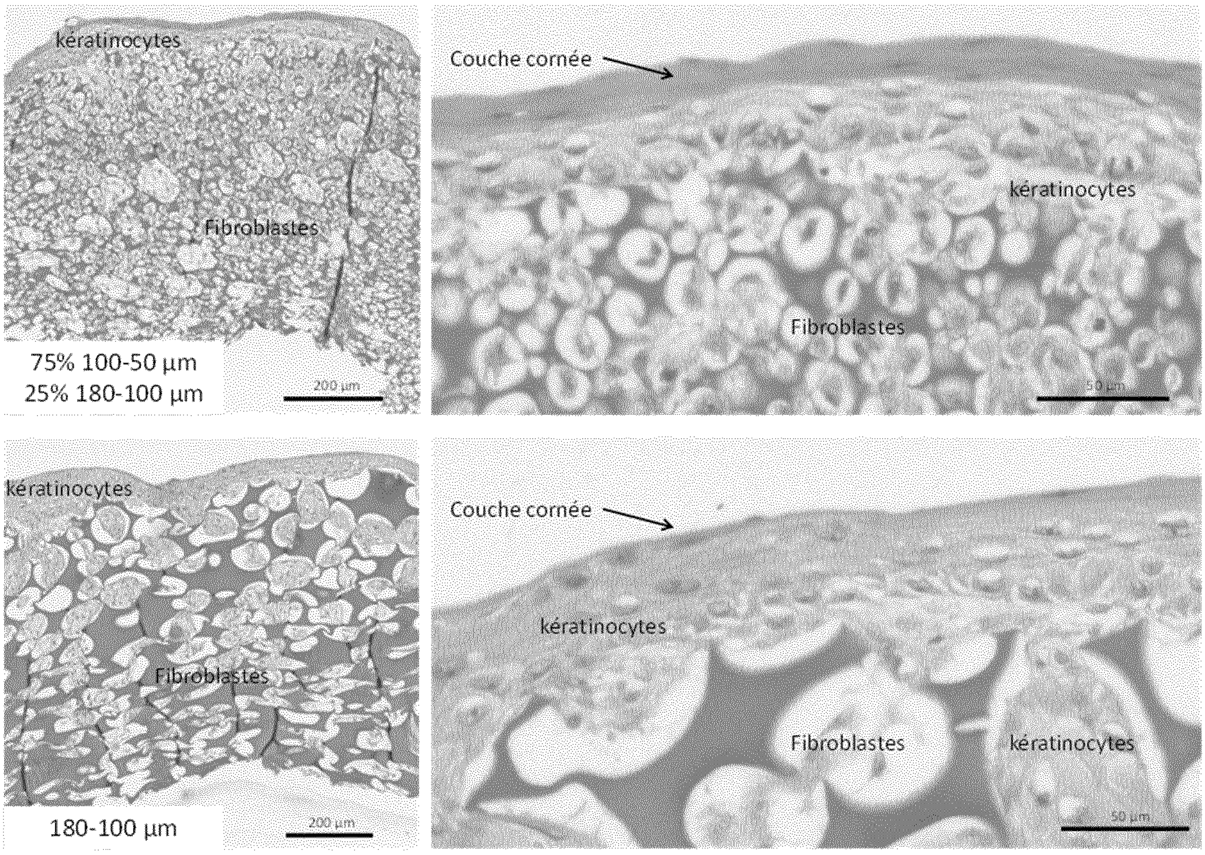
kératinocytes
Fibroblastes
75% 100-50 µm
25% 180-100 µm
200 µm
Couche cornée
kératinocytes
Fibroblastes
50 µm
kératinocytes
Fibroblastes
180-100 µm
200 µm
Couche cornée
kératinocytes
Fibroblastes
kératinocytes
50 µm

FIG 10

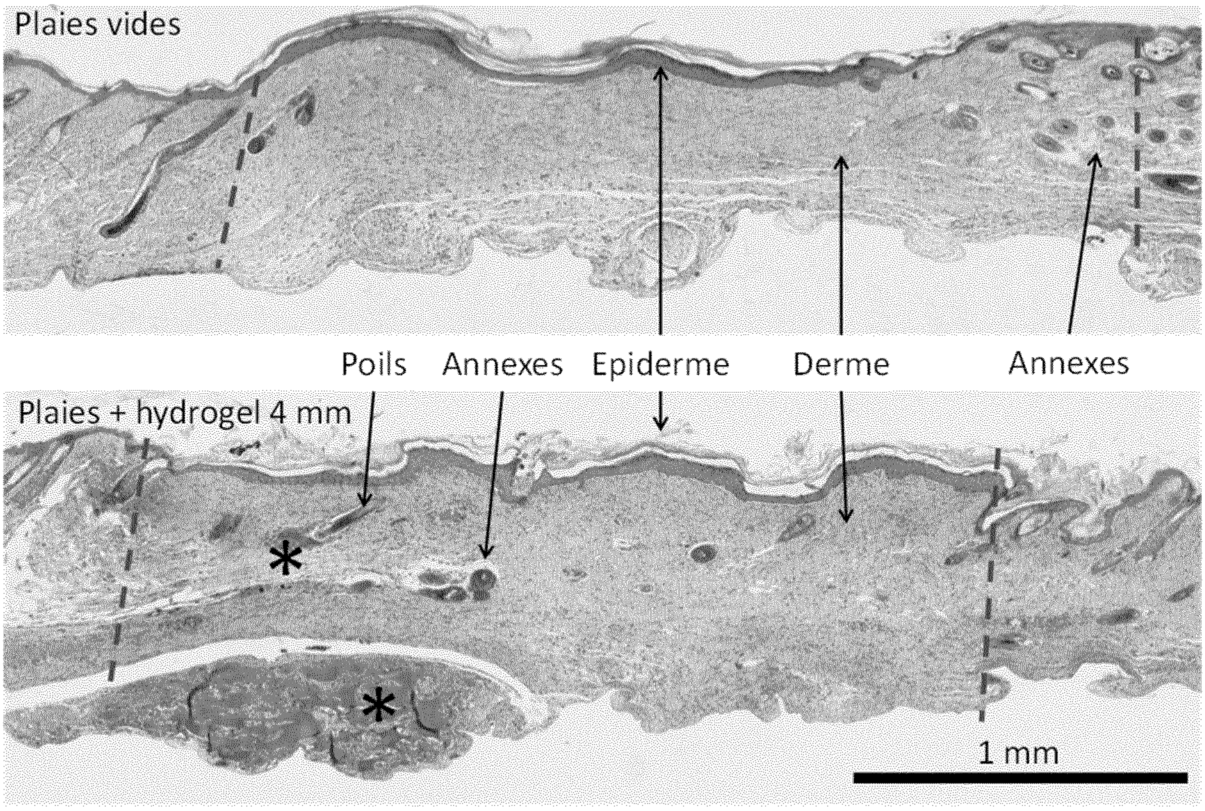
Plaies vides
Poils
Annexes
Epiderme
Derme
Annexes
Plaies + hydrogel 4 mm
*
*
1 mm

FIG 11A, 11B, 11C

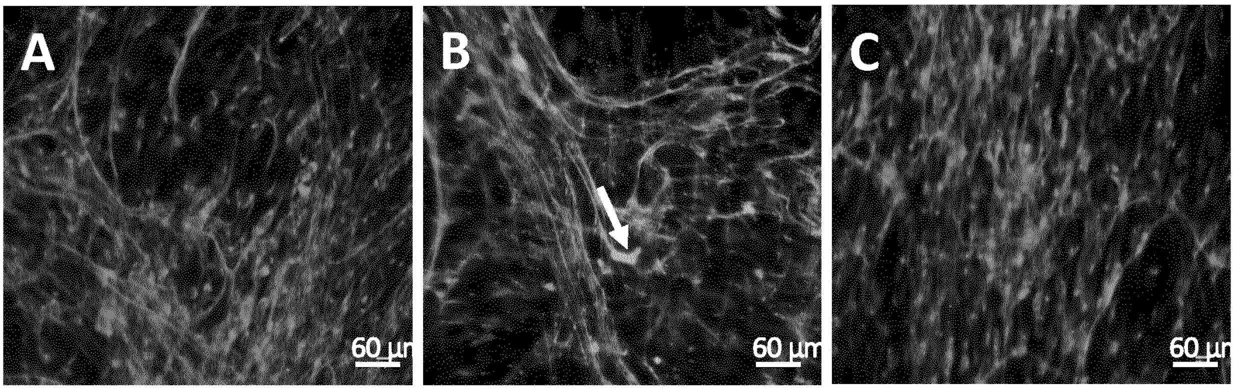


FIG 12A, 12B, 12C

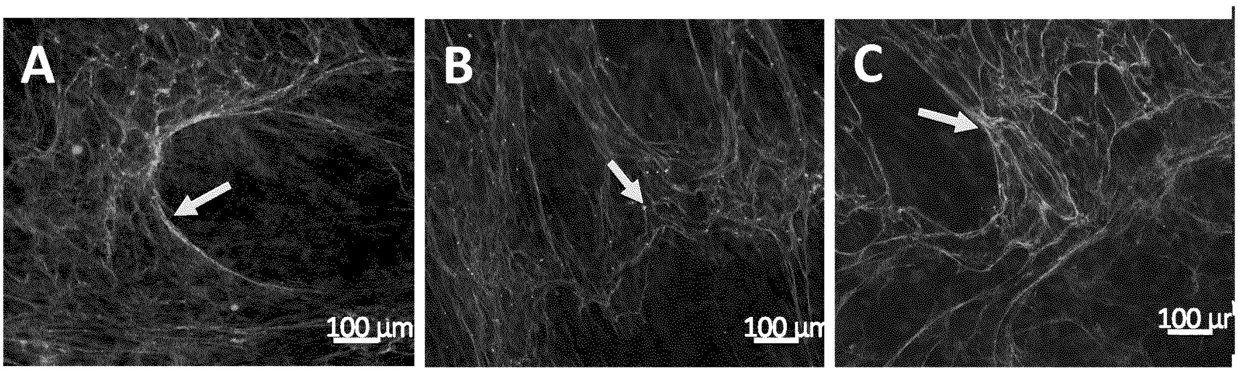

FIG. 13

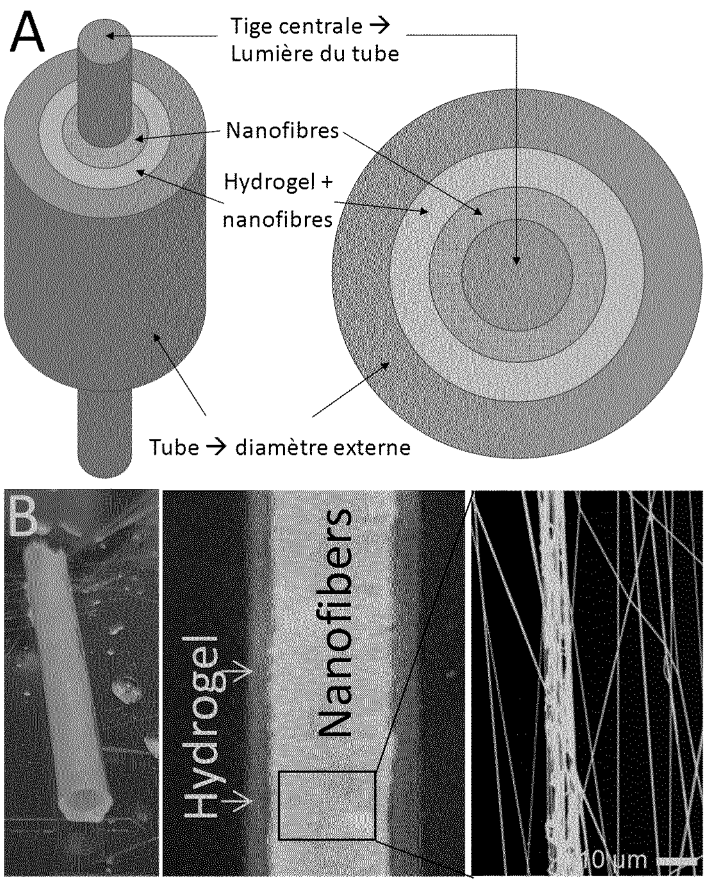
A
Tige centrale →
Lumière du tube
Nanofibres
Hydrogel +
nanofibres
Tube → diamètre externe
B
Hydrogel
Nanofibers
10 µm

**FIG 14A, 14B, 14C, 14D**

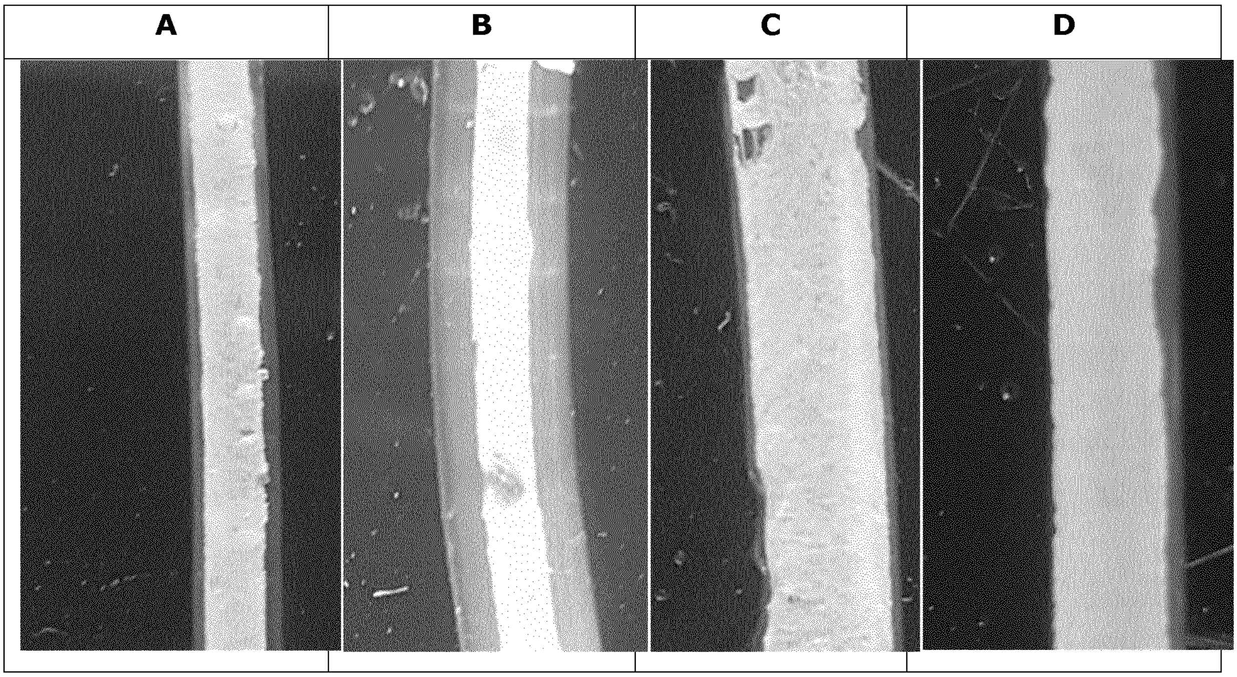


**FIG 15A, 15B**

**FIG 15A**

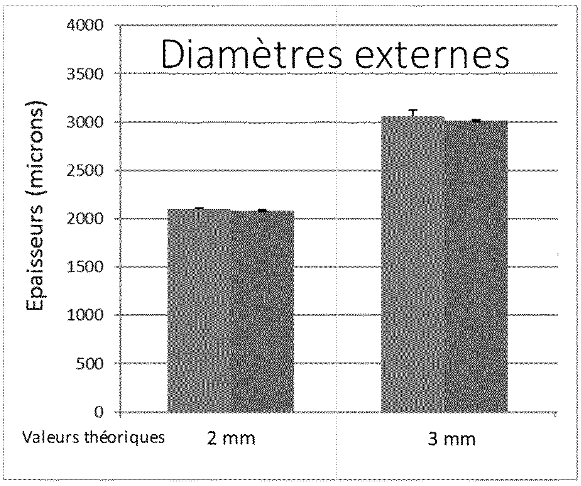


**FIG 15B**

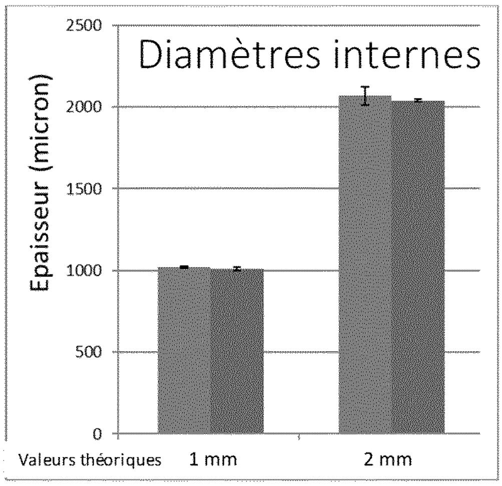

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1007555 A
- WO 2010119420 A
- WO 9903886 A
- WO 2006114528 A
- FR 1654306


### Littérature non-brevet citée dans la description


- **BANDIERA et al.** *Biotechnol Appl Biochem,* 2005, vol. 42, 247-256
- **ANNABI N. et al.** Elastomeric recombinant protein-based biomaterials. *Biochem. Eng. J.,* 2013, vol. 77, 110-118
- Macromolecular highly branched homogeneous compound based on lysine units. **DENKEWALTER R. G. et al.** USP. 1981. Allied Corp, 1981
- Macromolecular highly branched homogeneous compound. **DENKEWALTER R.G. et al.** USP. 1983. Allied Corp, 1983
- **JEON W.B. et al.** Stimulation of fibroblasts and neuroblasts on a biomimetic extracellular matrix consisting of tandem repeats of the elastic VGVPG domain and RGD motif. *J. Biomed. Mater. Res. A.,* 2011, vol. 97 (2), 152-157
- **KLOK H.A. et al.** Dendritic graft polypeptides. *Macromolecules,* 2002, vol. 35, 8718-8723
- **NONAKA R. et al.** Domain 36 of tropoelastin in elastic fiber formation. *Biol Pharm Bull,* 2014, vol. 37, 698-702
- **RODRIGUEZ-HERNANDEZ J. et al.** Highly Branched Poly (L-lysine). *Biomacromolecules,* 2003, vol. 4, 249-258
- **PAILLER-MATTEI et al.** In vivo skin biophysical behaviour and surface topography as a function of ageing. *J. Mech. Behav. Biomed. Mater.,* Décembre 2013, vol. 28, 474-83
- **ROSSI J.C. et al.** Functionalisation of free amino groups of lysine dendrigraft (DGL) polymers. *Tetrahedron Letters,* 2012, vol. 53, 2976-2979
- **SOHIER J. et al.** novel and simple alternative to create nanofibrillar matrices of interest for tissue engineering. *Tissue Engineering Part C-Methods, 2014,* 2014, vol. 20 (4), 285-29
- **WISE S. G. et al.** Tropoelastin: a versatile, bioactive assembly module. *Acta Biomater,* 2014, vol. 10 (4), 1532-41
- **YEO G.C. et al.** Coacervation of tropoelastin. *Adv Colloid Interface Sci,* 2011, vol. 167 (1-2), 94-103
- **YEO G.C. et al.** *Fabricated Elastin. Advanced healthcare materials,* 2015, vol. 4 (16), 2530-2556